# EUROPEAN PATENT SPECIFICATION

(11) **EP 2 492 276 B1**
(45) Date of publication and mention of the grant of the patent: **08.10.2014**
(21) Application number: 10825075.4
(22) Date of filing: 18.10.2010
(51) Int. Cl.: C07F 15/00, C07D 217/02, C07D 401/04, C08G 61/00, C08L 65/00, C09K 11/06, H01L 51/50

(54) **METAL COMPLEX, POLYMER, AND ELEMENT OBTAINED USING SAME**
METALLKOMPLEX, POLYMER UND DARAUS GEWONNENES ELEMENT
COMPLEXE MÉTALLIQUE, POLYMÈRE, ET ÉLÉMENT OBTENU AU MOYEN DE CEUX-CI

(30) Priority: 19.10.2009 JP 2009240163
(43) Date of publication of application: 29.08.2012
(73) Proprietor: Sumitomo Chemical Company, Limited, Tokyo 104-8260 (JP)
(72) Inventor: OKAMURA, Rei, Tsukuba-shi Ibaraki 305-0821 (JP); ASADA, Kohei, Tsukuba-shi Ibaraki 305-0821 (JP)
(74) Representative: Duckworth, Timothy John
(86) International application number: PCT/JP2010/068782
(87) International publication number: WO 2011/049226

(56) References cited:
- WO-A1-2006/059758
- WO-A1-2008/078800
- JP-A- 2007 269 733
- JP-A- 2008 179 617
- JP-A- 2008 505 239
- US-A1- 2008 023 672

## Description

### Technical Field

The present invention relates to a metal complex, a polymer compound and a device using the same.

### Background Art

An iridium complex having coordination of three molecules of 1-phenylisoquinoline is known as a red light emitting material used in a light emitting layer of an organic EL device (International Publication WO 2002-44189 pamphlet).

Document WO2008/078800 discloses irdium complexes, as well as polymers comprising said complexes and their application in devices.

### Disclosure of the Invention

This iridium complex, however, shows insufficient solution stability.

The present invention has an object of providing a red light emitting material excellent in solution stability.

The present invention provides a metal complex, a metal-containing polymer compound, a composition, a film, a device, a surface light source, an illuminating system, and a method for producing a metal complex, described below.
[1] A metal complex represented by the following formula (1) : 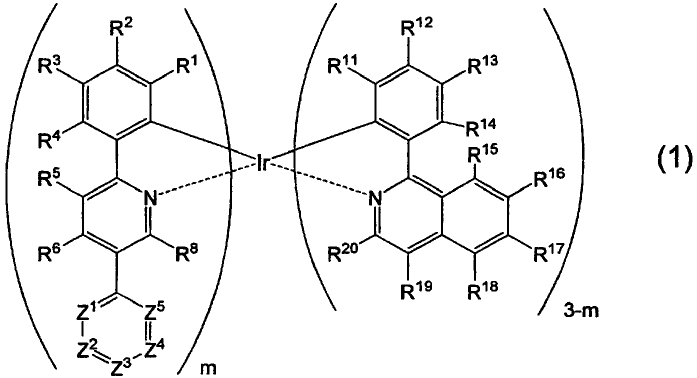
   wherein R¹, R², R³, R⁴, R⁵, R⁶, R⁸, R¹¹ R¹², R¹³, R¹⁴, R¹⁵, R¹⁶, R¹⁷, R¹⁸, R¹⁹ and R²⁰ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent;
   Z¹, Z², Z³, Z⁴ and Z⁵ each independently represent -C(R*)= or a nitrogen atom, where R* represents a hydrogen atom or a substituent;
   when there are two or more moieties of each of R¹, R² R⁴, R⁵, R⁶, R⁸, R¹¹, R¹², R¹³, R¹⁴, R¹⁵, R¹⁷, R¹⁸, R¹⁹, R²⁰, Z¹, Z², Z³, Z⁴ and Z⁵, the two or more moieties may be the same or different, provided that at least two of Z¹, Z², Z³, Z⁴ and Z⁵ are nitrogen atoms;
   m represents 1 or 2.
[2] The metal complex according to [1], represented by the following formula (1c): 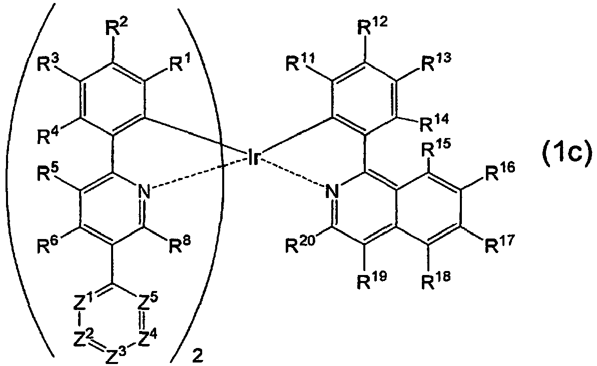 wherein R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above.
[3] The metal complex according to [2], represented by the following formula (1a) or the following formula (1b): 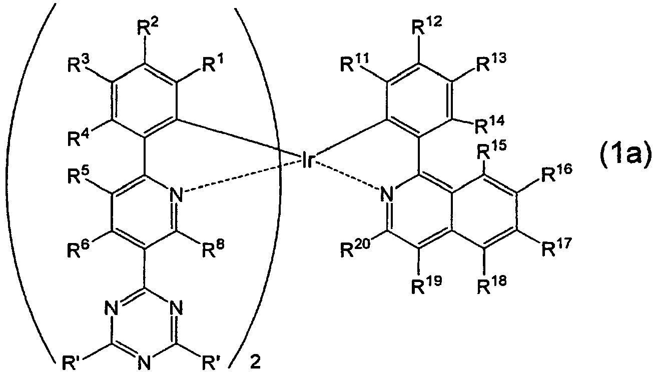 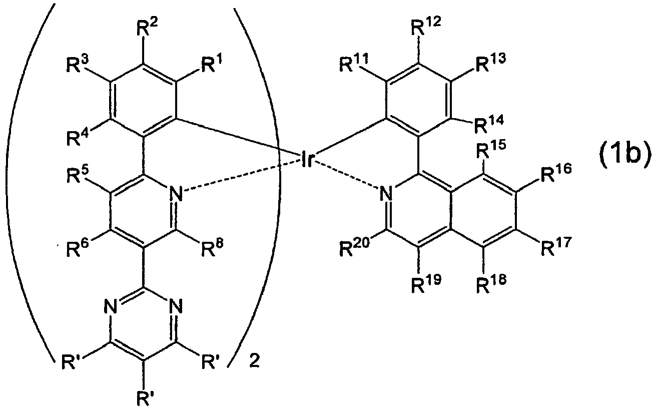
   wherein R¹, R² R³, R⁴, R⁵ , R⁶, R⁸, R¹¹, R¹², R¹³, R¹⁴, R¹⁵, R¹⁶, R¹⁷, R¹⁸, R¹⁹ and R²⁰ have the same meaning as described above;
   R' represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent, wherein the plurality of R' moieties may be the same or different.
[4] A metal-containing polymer compound having a residue of the metal complex according to any one of [1] to [3].
[5] A composition comprising the metal complex according to any one of [1] to [3] or the metal-containing polymer compound according to [4].
[6] The composition according to [5], comprising two or more metal complexes according to any one of [1] to [3] or comprising two or more metal-containing polymer compounds according to [4].
[7] The composition according to [5] or [6], comprising the metal complex according to any one of [1] to [3], and a metal complex represented by the following formula (2), a metal complex represented by the following formula (3) or a combination thereof: 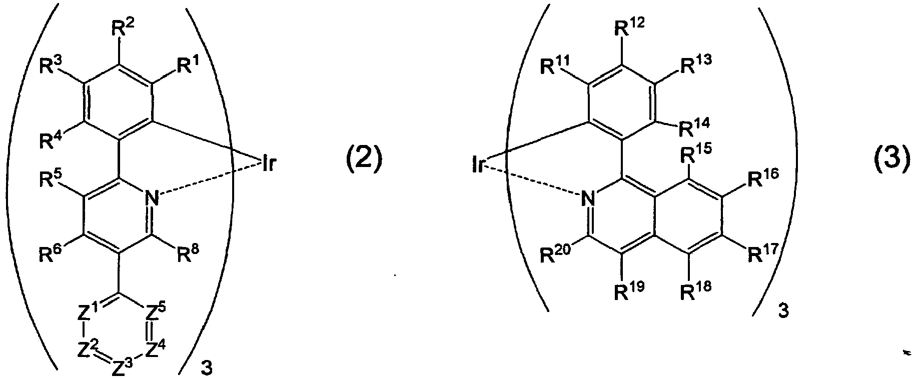 wherein R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above.
[8] The composition according to any one of [5] to [7], further comprising a non-metal-containing polymer compound.
[9] The composition according to [8], wherein the above-described non-metal-containing polymer compound has at least one member selected from the group consisting of a divalent group represented by the following formula (7-1), a divalent group represented by the following formula (7-2), a divalent group represented by the following formula (7-3), a divalent group represented by the following formula (7-4), a divalent group represented by the following formula (7-5) and a divalent group represented by the following formula (8), as a repeating unit: 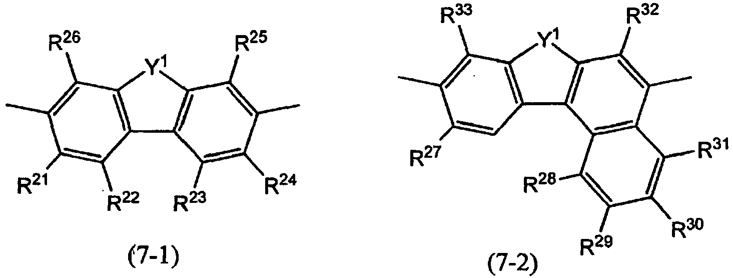 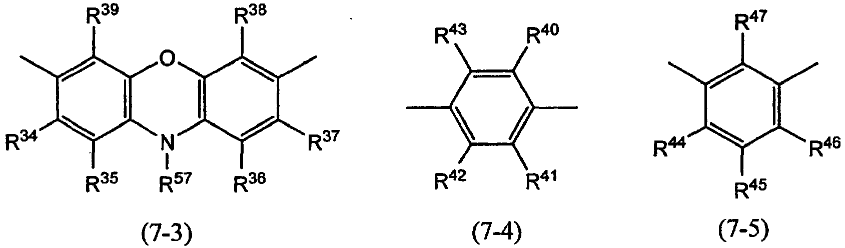
   wherein Y¹ represents -C(R⁴⁸)(R⁴⁹)-, -O-C(R⁵⁰)(R⁵¹)-, -O-, -S-, -B(R⁵²)-, -Si(R⁵³)(R⁵⁴)-, -P(R⁵⁵)-, -P(R⁵⁶) ( = O) - or N(R⁵⁷)-;
   R⁴⁸, R⁴⁹, R⁵⁰, R⁵¹, R⁵², R⁵³, R⁵⁴, R⁵⁵, R⁵⁶ and R⁵⁷ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group or a halogen atom, each of which may have a substituent;
   R²¹, R²², R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶ and R⁴⁷ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group or a cyano group, each of which may have a substituent: 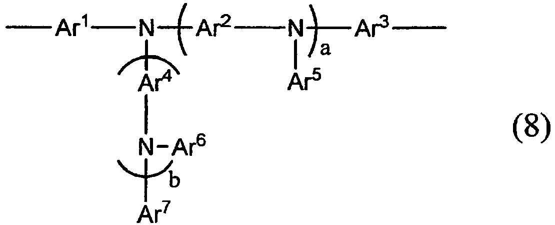
   wherein Ar¹, Ar², Ar³ and Ar⁴ each independently represent an arylene group or a divalent heterocyclic group, and Ar⁵, Ar⁶ and Ar⁷ each independently represent an aryl group or a monovalent heterocyclic group, wherein Ar¹, Ar², Ar³, Ar⁴, Ar⁵, Ar⁶ and Ar⁷ may have a substituent; a and b each independently represent 0 or 1, and 0 ≤ a+b ≤ 1.
[10] The composition according to [9], wherein the above-described non-metal-containing polymer compound further has a repeating unit represented by the following formula (10): 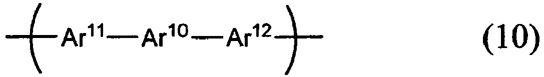 wherein Ar¹⁰ represents a divalent nitrogen-containing aromatic heterocyclic group, and Ar¹¹ and Ar¹² each independently represent an arylene group or a divalent heterocyclic group.
[11] The composition according to [10], wherein the repeating unit represented by the formula (10) is a repeating unit represented by the following formula (11): 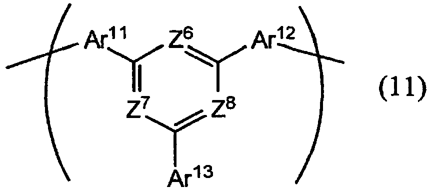
   wherein Ar¹¹ and Ar¹² have the same meaning as described above and Ar¹³ represents an aryl group or a monovalent heterocyclic group, wherein Ar¹¹, Ar¹² and Ar¹³ may have a substituent;
   Z⁶, Z⁷ and Z⁸ each independently represent -C(R^{k}) = or a nitrogen atom and R^{k} represents a hydrogen atom or a substituent, provided that at least two of Z⁶, Z⁷ and Z⁸ are nitrogen atoms.].
[12] The composition according to any one of [5] to [11], further comprising a charge transporting material, a light emitting material or a combination thereof.
[13] The composition according to any one of [5] to [12], further comprising a solvent or a dispersing medium.
[14] A film comprising the metal complex according to any one of [1] to [3], the metal-containing polymer compound according to [4] or the composition according to any one of [5] to [12].
[15] A device comprising the metal complex according to any one of [1] to [3], the metal-containing polymer compound according to [4] or the composition according to any one of [5] to [12].
[16] The device according to [15], wherein the above-described device is a light emitting device.
[17] A surface light source comprising the device according to [16].
[18] An illuminating system comprising the device according to [16].
[19] A method for producing a metal complex represented by the following formula (1c): 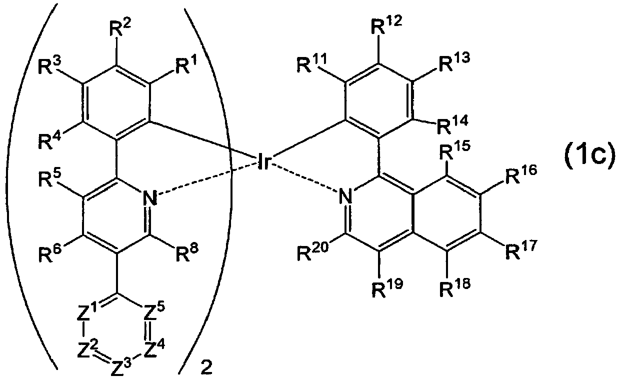
   wherein R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described below,
   comprising reacting a metal complex represented by the following formula (4): 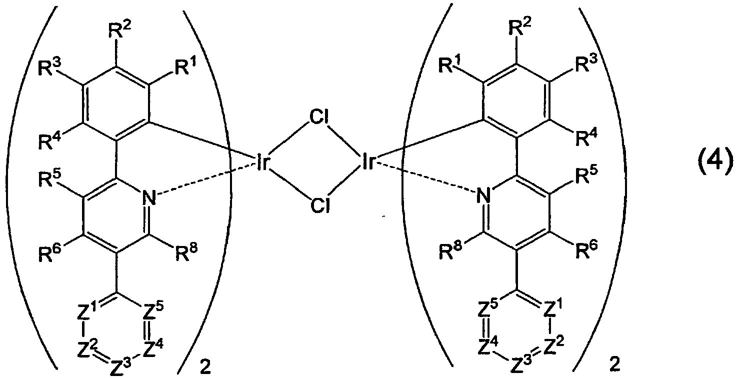
   wherein R¹, R², R³, R⁴, R⁵, R⁶ and R⁸ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent;
   Z¹, Z², Z³, Z⁴ and Z⁵ each independently represent, -C(R*)= or a nitrogen atom, wherein R* represents a hydrogen atom or a substituent;
   the plurality of moieties of each of R¹, R², R³, R⁴, R⁵, R⁶, R⁸, Z¹, Z², Z³, Z⁴ and Z⁵ may be the same or different, provided that at least two of Z¹, Z², Z³, Z⁴ and Z⁵ are nitrogen atoms,
      with a compound represented by the following formula (5): 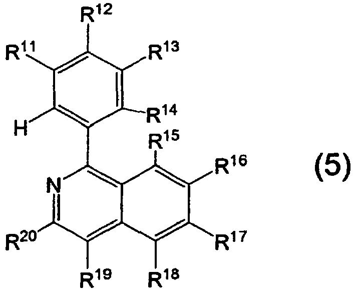 wherein R¹¹, R¹², R¹³, R¹⁴, R¹⁵, R¹⁶, R¹⁷, R¹⁸, R¹⁹ and R²⁰ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent.
[20] A method for producing a metal complex represented by the following formula (1c): 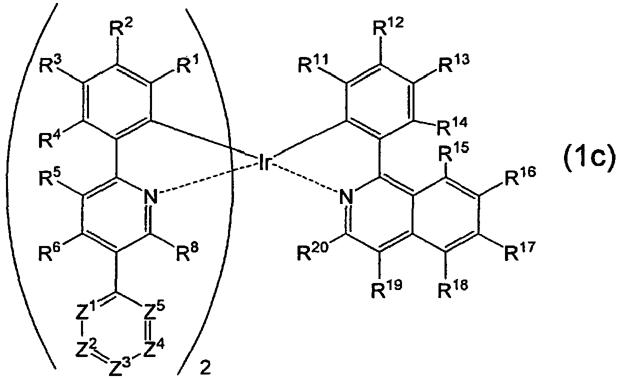
   wherein R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described below,
   comprising reacting a metal complex represented by the following formula (6): 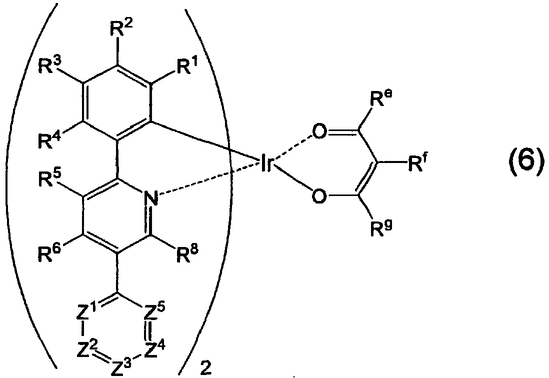
   wherein R¹, R², R³, R⁴, R⁵, R⁶ and R⁸ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent;
   Z¹, Z², Z³, Z⁴ and Z⁵ each independently represent, -C(R*)= or a nitrogen atom, wherein R* represents a hydrogen atom or a substituent;
   the two moieties of each of R¹, R², R³, R⁴, R⁵, R⁶, R⁸, Z¹, Z², Z³, Z⁴ and Z⁵ may be the same or different, provided that at least two of Z¹, Z², Z³, Z⁴ and Z⁵ are nitrogen atoms;
   R^{e}, R^{f} and R^{g} each independently represent a hydrogen atom, an alkyl group, an alkoxy group, an aryl group or a monovalent heterocyclic group,
   with a compound represented by the following formula (5): 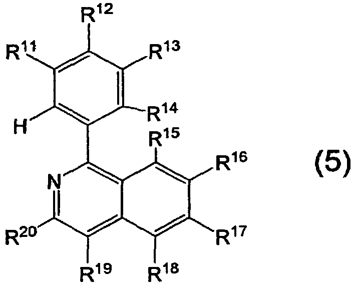 wherein R¹¹, R¹², R¹³, R¹⁴, R¹⁵, R¹⁶, R¹⁷, R¹⁸, R¹⁹ and R²⁰ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent.

### Modes for Carrying Out the Invention

The present invention will be illustrated in detail below. In the structural formulae in the present specification, a bond represented by a broken line denotes a coordination bond. "Metal-containing polymer compound" means a polymer compound containing a metal atom and/or a metal ion in the molecule, and "non-metal-containing polymer compound" means a polymer compound not containing a metal atom and a metal ion in the molecule.

### <Metal complex>

The metal complex of the present invention is represented by the formula (1).

The metal complex represented by the formula (1) is constituted of an iridium atom and a bidentate ligand.

In the formula (1), m is preferably 2. In this case, the formula (1) is the formula (1c).

In the formula (1), the halogen atom represented by R¹ to R⁶, R⁸ and R¹¹ to R²⁰ includes a fluorine atom, a chlorine atom, a bromine atom, an iodine atom and the like.

In the formula (1), the alkyl group represented by R¹ to R⁶, R⁸ and R¹¹ to R²⁰ may be any of linear, branched and cyclic. This alkyl group has a carbon atom number of usually 1 to 12. The alkyl group includes a methyl group, an ethyl group, a propyl group, an isopropyl group, a butyl group, a sec-butyl group, an isobutyl group, a tert-butyl group, a pentyl group, a hexyl group, a cyclohexyl group, a heptyl group, an octyl group, a 2-ethylhexyl group, a nonyl group, a decyl group, a 3,7-dimethyloctyl group, a lauryl group, a trifluoromethyl group, a pentafluoroethyl group, a perfluorobutyl group, a perfluorohexyl group, a perfluorooctyl group and the like, and preferable are a tert-butyl group, a pentyl group, a hexyl group, an octyl group, a 2-ethylhexyl group, a decyl group and a 3,7-dimethyloctyl group.

In the formula (1), the alkoxy group represented by R¹ to R⁶, R⁸ and R¹¹ to R²⁰ may be any of linear, branched and cyclic. This alkoxy group has a carbon atom number of usually 1 to 12. The alkoxy group includes a methoxy group, an ethoxy group, a propyloxy group, an isopropyloxy group, a butoxy group, an isobutoxy group, a tert-butoxy group, a pentyloxy group, a hexyloxy group, a cyclohexyloxy group, a heptyloxy group, an octyloxy group, a 2-ethylhexyloxy group, a nonyloxy group, a decyloxy group, a 3,7-dimethyloctyloxy group, a lauryloxy group, a trifluoromethoxy group, a pentafluoroethoxy group, a perfluorobutoxy group, a perfluorohexyloxy group, a perfluorooctyloxy group, a methoxymethyloxy group, a 2-methoxyethyloxy group and the like, and preferable are a pentyloxy group, a hexyloxy group, an octyloxy group, a 2-ethylhexyloxy group, a decyloxy group and a 3,7-dimethyloctyloxy group.

In the formula (1), the alkylthio group represented by R¹ to R⁶, R⁸ and R¹¹ to R²⁰ may be any of linear, branched and cyclic. This alkylthio group has a carbon atom number of usually 1 to 12. The alkylthio group includes a methylthio group, an ethylthio group, a propylthio group, an isopropylthio group, a butylthio group, a sec-butylthio group, an isobutylthio group, a tert-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group, a laurylthio group, a trifluoromethylthio group and the like, and preferable are a pentylthio group, a hexylthio group, an octylthio group, a 2-ethylhexylthio group, a decylthio group and a 3,7-dimethyloctylthio group.

In the formula (1), the aryl group represented by R¹ to R⁶, R⁸ and R¹¹ to R²⁰ has a carbon atom number of usually 6 to 60, preferably 7 to 48. The aryl group includes a phenyl group, C₁ to C₁₂ alkoxyphenyl groups ("C₁ to C₁₂ alkoxy" means that the alkoxy portion has a carbon atom number of 1 to 12. The same shall apply hereinafter.), C₁ to C₁₂ alkylphenyl groups ("C₁ to C₁₂ alkyl" means that the alkyl portion has a carbon atom number of 1 to 12. The same shall apply hereinafter.), a 1-naphthyl group, a 2-naphthyl group, a 1-anthracenyl group, a 2-anthracenyl group, a 9-anthracenyl group, a pentafluorophenyl group and the like, and preferable are C₁ to C₁₂ alkoxyphenyl groups and C₁ to C₁₂ alkylphenyl groups. The aryl group is an atomic group obtained by removing one hydrogen atom from an aromatic hydrocarbon. This aromatic hydrocarbon includes also compounds having a condensed ring and compounds having two or more independent benzene rings or condensed rings linked directly or via a vinylene group or the like. Further, the aryl group may have a substituent, and the substituent includes C₁ to C₁₂ alkoxyphenyl groups, C₁ to C₁₂ alkylphenyl groups and the like.

The C₁ to C₁₂ alkoxyphenyl group includes a methoxyphenyl group, an ethoxyphenyl group, a propyloxyphenyl group, an isopropyloxyphenyl group, a butoxyphenyl group, an isobutoxyphenyl group, a tert-butoxyphenyl group, a pentyloxyphenyl group, a hexyloxyphenyl group, a cyclohexyloxyphenyl group, a heptyloxyphenyl group, an octyloxyphenyl group, a 2-ethylhexyloxyphenyl group, a nonyloxyphenyl group, a decyloxyphenyl group, a 3,7-dimethyloctyloxyphenyl group, a lauryloxyphenyl group and the like.

The C₁ to C₁₂ alkylphenyl group includes a methylphenyl group, an ethylphenyl group, a dimethylphenyl group, a propylphenyl group, a mesityl group, a methylethylphenyl group, an isopropylphenyl group, a butylphenyl group, a sec-butylphenyl group, an isobutylphenyl group, a tert-butylphenyl group, a pentylphenyl group, an isoamylphenyl group, a hexylphenyl group, a heptylphenyl group, an octylphenyl group, a nonylphenyl group, a decylphenyl group, a dodecylphenyl group and the like.

The aryl group having two or more independent benzene rings linked directly or via a vinylene group or the like includes groups represented by the following formulae: 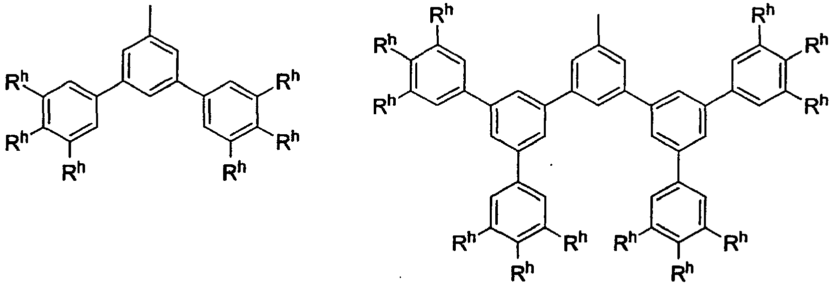 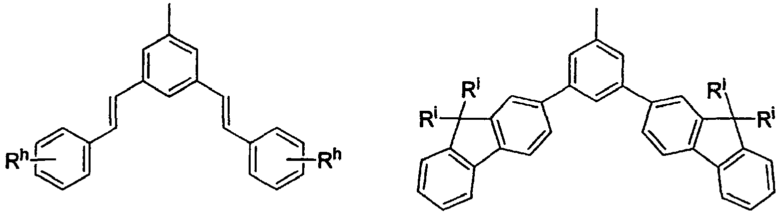 [wherein R^{h} represents a hydrogen atom, an alkyl group having 1 to 12 carbon atoms or an alkoxy group having 1 to 12 carbon atoms, and some hydrogen atoms of these groups may be substituted by a halogen atom. The plurality of R^{h} moieties may be the same or different, with the proviso that at least one of R^{h} s is an alkyl group having 1 to 12 carbon atoms or an alkoxy group having 1 to 12 carbon atoms. Rⁱ represents a linear or branched alkyl group having 1 to 12 carbon atoms. The plurality of Rⁱ moieties may be the same or different.].

In the formula (1), the aryloxy group represented by R¹ to R⁶, R⁸ and R¹¹ to R²⁰ has a carbon atom number of usually 6 to 60, preferably 7 to 48. The aryloxy group includes a phenoxy group, C₁ to C₁₂ alkoxyphenoxy groups, C₁ to C₁₂ alkylphenoxy groups, a 1-naphthyloxy group, a 2-naphthyloxy group, a pentafluorophenyloxy group and the like, and preferable are C₁ to C₁₂ alkoxyphenoxy groups and C₁ to C₁₂ alkylphenoxy groups.

The C₁ to C₁₂ alkoxyphenoxy group includes a methoxyphenoxy group, an ethoxyphenoxy group, a propyloxyphenoxy group, an isopropyloxyphenoxy group, a butoxyphenoxy group, an isobutoxyphenoxy group, a tert-butoxyphenoxy group, a pentyloxyphenoxy group, a hexyloxyphenoxy group, a cyclohexyloxyphenoxy group, a heptyloxyphenoxy group, an octyloxyphenoxy group, a 2-ethylhexyloxyphenoxy group, a nonyloxyphenoxy group, a decyloxyphenoxy group, a 3,7-dimethyloctyloxyphenoxy group, a lauryloxyphenoxy group and the like.

The C₁ to C₁₂ alkylphenoxy group includes a methylphenoxy group, an ethylphenoxy group, a dimethylphenoxy group, a propylphenoxy group, a 1,3,5-trimethylphenoxy group, a methylethylphenoxy group, an isopropylphenoxy group, a butylphenoxy group, a sec-butylphenoxy group, an isobutylphenoxy group, a tert-butylphenoxy group, a pentylphenoxy group, an isoamylphenoxy group, a hexylphenoxy group, a heptylphenoxy group, an octylphenoxy group, a nonylphenoxy group, a decylphenoxy group, a dodecylphenoxy group and the like.

In the formula (1), the arylthio group represented by R¹ to R⁶, R⁸ and R¹¹ to R²⁰ has a carbon atom number of usually 6 to 60, preferably 7 to 48. The arylthio group includes a phenylthio group, C₁ to C₁₂ alkoxyphenylthio groups, C₁ to C₁₂ alkylphenylthio groups, a 1-naphthylthio group, a 2-naphthylthio group, a pentafluorophenylthio group and the like, and preferable are C₁ to C₁₂ alkoxyphenylthio groups and C₁ to C₁₂ alkylphenylthio groups.

In the formula (1), the arylalkyl group represented by R¹ to R⁶, R⁸ and R¹¹ to R²⁰ has a carbon atom number of usually 7 to 60, preferably 7 to 48. The arylalkyl group includes phenyl C₁ to C₁₂ alkyl groups, C₁ to C₁₂ alkoxyphenyl C₁ to C₁₂ alkyl groups, C₁ to C₁₂ alkylphenyl C₁ to C₁₂ alkyl groups, 1-naphthyl C₁ to C₁₂ alkyl groups, 2-naphthyl C₁ to C₁₂ alkyl groups and the like, and preferable are C₁ to C₁₂ alkoxyphenyl C₁ to C₁₂ alkyl groups and C₁ to C₁₂ alkylphenyl C₁ to C₁₂ alkyl groups.

In the formula (1), the arylalkoxy group represented by R¹ to R⁶, R⁸ and R¹¹ to R²⁰ has a carbon atom number of usually 7 to 60, preferably 7 to 48. The arylalkoxy group includes phenyl C₁ to C₁₂ alkoxy groups such as a phenylmethoxy group, a phenylethoxy group, a phenylbutoxy group, a phenylpentyloxy group, a phenylhexyloxy group, a phenylheptyloxy group, a phenyloctyloxy group and the like; C₁ to C₁₂ alkoxyphenyl C₁ to C₁₂ alkoxy groups, C₁ to C₁₂ alkylphenyl C₁ to C₁₂ alkoxy groups, 1-naphthyl C₁ to C₁₂ alkoxy groups, 2-naphthyl C₁ to C₁₂ alkoxy groups and the like, and preferable are C₁ to C₁₂ alkoxyphenyl C₁ to C₁₂ alkoxy groups and C₁ to C₁₂ alkylphenyl C₁ to C₁₂ alkoxy groups.

In the formula (1), the arylalkylthio group represented by R¹ to R⁶, R⁸ and R¹¹ to R²⁰ has a carbon atom number of usually 7 to 60, preferably 7 to 48. The arylalkylthio group includes phenyl C₁ to C₁₂ alkylthio groups, C₁ to C₁₂ alkoxyphenyl Ci to C₁₂ alkylthio groups, C₁ to C₁₂ alkylphenyl C₁ to C₁₂ alkylthio groups, 1-naphthyl C₁ to C₁₂ alkylthio groups, 2-naphthyl-C₁ to C₁₂ alkylthio groups and the like, and preferable are C₁ to C₁₂ alkoxyphenyl C₁ to C₁₂ alkylthio groups and C₁ to C₁₂ alkylphenyl C₁ to C₁₂ alkylthio groups.

In the formula (1), the acyl group represented by R¹ to R⁶, R⁸ and R¹¹ to R²⁰ has a carbon atom number of usually 2 to 20, preferably 2 to 18. The acyl group includes an acetyl group, a propionyl group, a butyryl group, an isobutyryl group, a pivaloyl group, a benzoyl group, a trifluoroacetyl group, a pentafluorobenzoyl group and the like.

In the formula (1), the acyloxy group represented by R¹ to R⁶, R⁸ and R¹¹ to R²⁰ has a carbon atom number of usually 2 to 20, preferably 2 to 18. The acyloxy group includes an acetoxy group, a propionyloxy group, a butyryloxy group, an isobutyryloxy group, a pivaloyloxy group, a benzoyloxy group, a trifluoroacetyloxy group, a pentafluorobenzoyloxy group and the like.

In the formula (1), the amide group represented by R¹ to R⁶, R⁸ and R¹¹ to R²⁰ has a carbon atom number of usually 1 to 20, preferably 1 to 18. The amide group includes a formamide group, an acetamide group, a propioamide group, a butyroamide group, a benzamide group, a trifluoroacetamide group, a pentafluorobenzamide group, a diformamide group, a diacetamide group, a dipropioamide group, a dibutyroamide group, a dibenzamide group, a ditrifluoroacetamide group, a dipentafluorobenzamide group and the like.

In the formula (1), the acid imide group represented by R¹ to R⁶, R⁸ and R¹¹ to R²⁰ means a monovalent residue obtained by removing from an acid imide one hydrogen atom linked to its nitrogen atom. This acid imide group has a carbon atom number of usually 2 to 60, preferably 2 to 48. The acid imide group includes groups represented by the following structural formulae and the like. 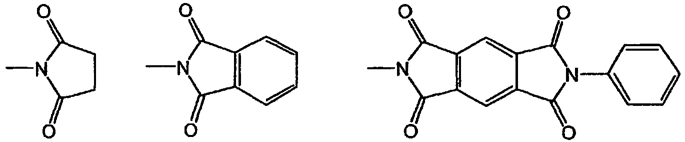 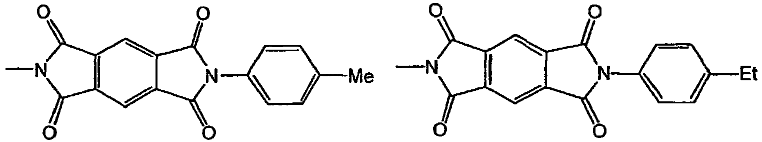 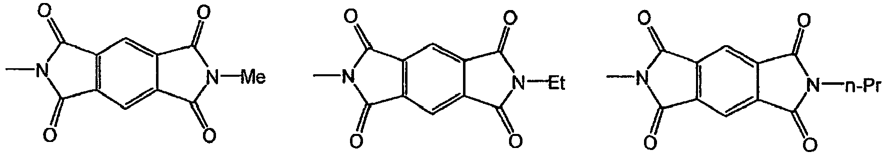 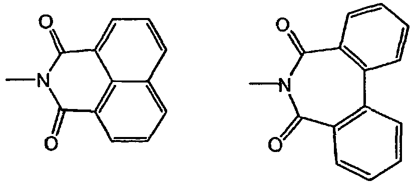 [wherein a line extending from a nitrogen atom represents a connecting bond, Me represents a methyl group, Et represents an ethyl group and n-Pr represents a n-propyl group. The same shall apply hereinafter.]

In the formula (1), the imine residue represented by R¹ to R⁶, R⁸ and R¹¹ to R²⁰ means a monovalent residue obtained by removing one hydrogen atom from an imine compound (that is, an organic compound having -N=C- in the molecule. Examples thereof include aldimines, ketimines, compounds obtained by substituting a hydrogen atom linked to a nitrogen atom in these molecules by an alkyl group or the like; etc.). This imine residue has a carbon atom number of usually 2 to 20, preferably 2 to 18.

The imine residue includes groups represented by the following structural formulae and the like. 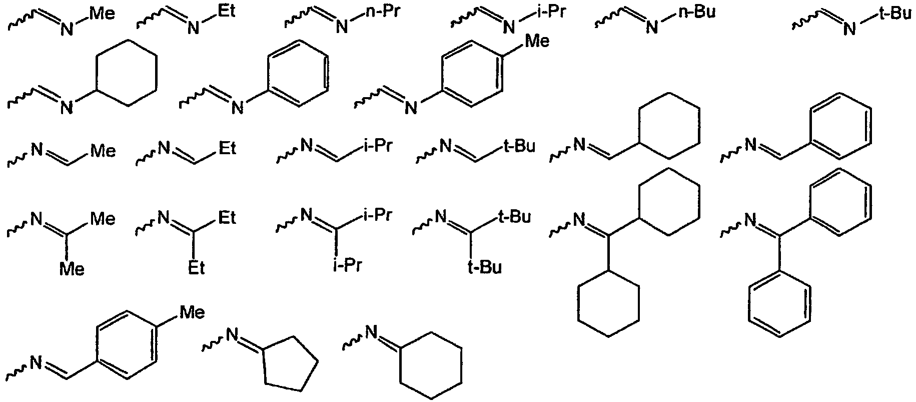 [wherein i-Pr represents an isopropyl group, n-Bu represents a n-butyl group and t-Bu represents a tert-butyl group. The bond represented by a wavy line means "bond represented by wedge shape" and/or "bond represented by dashed line". Here, "bond represented by wedge shape" means a bond protruding toward the near side from the plane of paper and "bond represented by dashed line" means a bond protruding toward the far side of the plane of paper.]

In the formula (1), the substituted amino group represented by R¹ to R⁶, R⁸ and R¹¹ to R²⁰ means an amino group substituted by one or two groups selected from the group consisting of alkyl groups, aryl groups, arylalkyl groups and monovalent heterocyclic groups. The alkyl group, aryl group, arylalkyl group or monovalent heterocyclic group may have a substituent. The substituted amino group has a carbon atom number, not including the carbon atom number of the substituent, of usually 1 to 60, preferably 2 to 48.

The substituted amino group includes a methylamino group, a dimethylamino group, an ethylamino group, a diethylamino group, a propylamino group, a dipropylamino group, an isopropylamino group, a diisopropylamino group, a butylamino group, a sec-butylamino group, an isobutylamino group, a tert-butylamino group, a pentylamino group, a hexylamino group, a cyclohexylamino group, a heptylamino group, an octylamino group, a 2-ethylhexylamino group, a nonylamino group, a decylamino group, a 3,7-dimethyloctylamino group, a laurylamino group, a cyclopentylamino group, a dicyclopentylamino group, a cyclohexylamino group, a dicyclohexylamino group, a pyrrolidyl group, a piperidyl group, a ditrifluoromethylamino group, a phenylamino group, a diphenylamino group, C₁ to C₁₂ alkoxyphenylamino groups, di(C₁ to C₁₂ alkoxyphenyl)amino groups, di (C₁ to C₁₂ alkylphenyl)amino groups, a 1-naphthylamino group, a 2-naphthylamino group, a pentafluorophenylamino group, a pyridylamino group, a pyridazinylamino group, a pyrimidylamino group, a pyrazylamino group, a triazylamino group, phenyl C₁ to C₁₂ alkylamino groups, C₁ to C₁₂ alkoxyphenyl C₁ to C₁₂ alkylamino groups, C₁ to C₁₂ alkylphenyl C₁ to C₁₂ alkylamino groups, di(C₁ to C₁₂ alkoxyphenyl C₁ to C₁₂ alkyl)amino groups, di (C₁ to C₁₂ alkylphenyl C₁ to C₁₂ alkyl)amino groups, 1-naphthyl C₁ to C₁₂ alkylamino groups, 2-naphthyl C₁ to C₁₂ alkylamino groups and the like.

In the formula (1), the substituted silyl group represented by R¹ to R⁶, R⁸ and R¹¹ to R²⁰ means a silyl group substituted by 1, 2 or 3 groups selected from the group consisting of alkyl groups, aryl groups, arylalkyl groups and monovalent heterocyclic groups. The substituted silyl group has a carbon atom number of usually 1 to 60, preferably 3 to 48. The alkyl group, aryl group, arylalkyl group or monovalent heterocyclic group may have a substituent.

The substituted silyl group includes a trimethylsilyl group, a triethylsilyl group, a tripropylsilyl group, a triisopropylsilyl group, an isopropyldimethylsilyl group, a diethylisopropylsilyl group, a tert-butyldimethylsilyl group, a dimethylpentylsilyl group, a hexyldimethylsilyl group, a heptyldimethylsilyl group, a dimethyloctylsilyl group, a 2-ethylhexyldimethylsilyl group, a dimethylnonylsilyl group, a decyldimethylsilyl group, a 3,7-dimethyloctyldimethylsilyl group, a lauryldimethylsilyl group, phenyl C₁ to C₁₂ alkylsilyl groups, C₁ to C₁₂ alkoxyphenyl C₁ to C₁₂ alkylsilyl groups, C₁ to C₁₂ alkylphenyl C₁ to C₁₂ alkylsilyl groups, 1-naphthyl C₁ to C₁₂ alkylsilyl groups, 2-naphthyl C₁ to C₁₂ alkylsilyl groups, phenyl C₁ to C₁₂ alkyldimethylsilyl groups, a triphenylsilyl group, a tri(p-tolyl)silyl group, a tribenzylsilyl group, a methyldiphenylsilyl group, a tert-butyldiphenylsilyl group, a dimethylphenylsilyl group and the like.

In the formula (1), the substituted silyloxy group represented by R¹ to R⁶, R⁸ and R¹¹ to R²⁰ means a silyloxy group substituted by 1, 2 or 3 groups selected from the group consisting of alkoxy groups, aryloxy groups, arylalkoxy groups and monovalent heterocyclicoxy groups. The substituted silyloxy group has a carbon atom number of usually 1 to 60, preferably 3 to 48. The alkoxy group, aryloxy group, arylalkoxy group or monovalent heterocyclicoxy group may have a substituent.

The substituted silyloxy group includes a trimethylsilyloxy group, a triethylsilyloxy group, a tripropylsilyloxy group, a triisopropylsilyloxy group, an isopropyldimethylsilyloxy group, a diethylisopropylsilyloxy group, a tert-butyldimethylsilyloxy group, a dimethylpentylsilyloxy group, a hexyldimethylsilyloxy group, a heptyldimethylsilyloxy group, a dimethyloctylsilyloxy group, a 2-ethylhexyldimethylsilyloxy group, a dimethylnonylsilyloxy group, a decyldimethylsilyloxy group, a 3,7-dimethyloctyldimethylsilyloxy group, a lauryldimethylsilyloxy group, phenyl C₁ to C₁₂ alkylsilyloxy groups, C₁ to C₁₂ alkoxyphenyl C₁ to C₁₂ alkylsilyloxy groups, C₁ to C₁₂ alkylphenyl C₁ to C₁₂ alkylsilyloxy groups, 1-naphthyl C₁ to C₁₂ alkylsilyloxy groups, 2-naphthyl C₁ to C₁₂ alkylsilyloxy groups, phenyl C₁ to C₁₂ alkyldimethylsilyloxy groups, a triphenylsilyloxy group, a tri(p-tolyl)silyloxy group, a tribenzylsilyloxy group, a methyldiphenylsilyloxy group, a tert-butyldiphenylsilyloxy group, a dimethylphenylsilyloxy group and the like.

In the formula (1), the substituted silylthio group represented by R¹ to R⁶, R⁸ and R¹¹ to R²⁰ means a silylthio group substituted by 1, 2 or 3 groups selected from the group consisting of alkylthio groups, arylthio groups, arylalkylthio groups and monovalent heterocyclicthio groups. The substituted silylthio group has a carbon atom number of usually 1 to 60, preferably 3 to 48. The alkoxy group, arylthio group, arylalkylthio group or monovalent heterocyclicthio group may have a substituent.

The substituted silylthio group includes a trimethylsilylthio group, a triethylsilylthio group, a tripropylsilylthio group, a triisopropylsilylthio group, an isopropyldimethylsilylthio group, a diethylisopropylsilylthio group, a tert-butyldimethylsilylthio group, a dimethylpentylsilylthio group, a hexyldimethylsilylthio group, a heptyldimethylsilylthio group, a dimethyloctylsilylthio group, a 2-ethylhexyldimethylsilylthio group, a dimethylnonylsilylthio group, a decyldimethylsilylthio group, a 3,7-dimethyloctyldimethylsilylthio group, a lauryldimethylsilylthio group, phenyl C₁ to C₁₂ alkylsilylthio groups, C₁ to C₁₂ alkoxyphenyl C₁ to C₁₂ alkylsilylthio groups, C₁ to C₁₂ alkylphenyl C₁ to C₁₂ alkylsilylthio groups, 1-naphthyl C₁ to C₁₂ alkylsilylthio groups, 2-naphthyl C₁ to C₁₂ alkylsilylthio groups, phenyl C₁ to C₁₂ alkyldimethylsilylthio groups, a triphenylsilylthio group, a tri(p-tolyl)silylthio group, a tribenzylsilylthio group, a methyldiphenylsilylthio group, a tert-butyldiphenylsilylthio group, a dimethylphenylsilylthio group and the like.

In the formula (1), the substituted silylamino group represented by R¹ to R⁶, R⁸ and R¹¹ to R²⁰ means a silylamino group substituted by 1, 2 or 3 groups selected from the group consisting of alkylamino groups, arylamino groups, arylalkylamino groups and monovalent heterocyclicamino groups. The substituted silylamino group has a carbon atom number of usually 1 to 60, preferably 3 to 48. The alkoxy group, arylamino group, arylalkylamino group ormonovalent heterocyclicamino group may have a substituent.

The substituted silylamino group includes a trimethylsilylamino group, a triethylsilylamino group, a tripropylsilylamino group, a triisopropylsilylamino group, a dimethyli-propylsilylamino group, a diethylisopropylsilylamino group, a tert-butyldimethylsilylamino group, a pentyldimethylsilylamino group, a hexyldimethylsilylamino group, a heptyldimethylsilylamino group, an octyldimethylsilylamino group, a 2-ethylhexyldimethylsilylamino group, a nonyldimethylsilylamino group, a decyldimethylsilylamino group, a 3,7-dimethyloctyldimethylsilylamino group, a lauryldimethylsilylamino group, phenyl C₁ to C₁₂ alkylsilyloxy groups, C₁ to C₁₂ alkoxyphenyl C₁ to C₁₂ alkylsilylamino groups, C₁ to C₁₂ alkylphenyl C₁ to C₁₂ alkylsilylamino groups, 1-naphthyl C₁ to C₁₂ alkylsilylamino groups, 2-naphthyl C₁ to C₁₂ alkylsilylamino groups, phenyl C₁ to C₁₂ alkyldimethylsilylamino groups, a triphenylsilylamino group, a tri(p-tolyl)silylamino group, a tribenzylsilylamino group, a diphenylmethylsilylamino group, a tert-butyldiphenylsilylamino group, a dimethylphenylsilylamino group and the like.

In the formula (1), the monovalent heterocyclic group represented by R¹ to R⁶, R⁸ and R¹¹ to R²⁰ means an atomic group remaining after removal of one hydrogen atom from a heterocyclic compound. The monovalent heterocyclic group has a carbon atom number of usually 3 to 60, preferably 3 to 20. The carbon atom number of the monovalent heterocyclic group does not include the carbon atom number of the substituent. The heterocyclic compound includes organic compounds having a cyclic structure in which elements constituting the ring include not only a carbon atom but also a hetero atom such as an oxygen atom, a sulfur atom, a nitrogen atom, a phosphorus atom, a boron atom and the like contained in the ring.

The monovalent heterocyclic group includes a thienyl group, C₁ to C₁₂ alkylthienyl groups, a pyrrolyl group, a furyl group, a pyridyl group, C₁ to C₁₂ alkylpyridyl groups, a pyrimidyl group, C₁ to C₁₂ alkylpyrimidyl groups, a triazyl group, C₁ to C₁₂ alkyltriazyl groups, a piperidyl group, a quinolyl group, an isoquinolyl group and the like, and preferable are a thienyl group, C₁ to C₁₂ alkylthienyl groups, a pyridyl group, C₁ to C₁₂ alkylpyridyl groups, a pyrimidyl group, C₁ to C₁₂ alkylpyrimidyl groups, a triazyl group and C₁ to C₁₂ alkyltriazyl groups. Further, the monovalent heterocyclic group may have a substituent. The monovalent heterocyclic group is preferably a monovalent aromatic heterocyclic group.

In the formula (1), the heteroaryloxy group represented by R¹ to R⁶, R⁸ and R¹¹ to R²⁰ has a carbon atom number of usually 6 to 60, preferably 7 to 48. The heteroaryloxy group includes a thienyloxy group, C₁ to C₁₂ alkoxythienyloxy groups, C₁ to C₁₂ alkylthienyloxy groups, C₁ to C₁₂ alkoxypyridyloxy groups, C₁ to C₁₂ alkylpyridyloxy groups, an isoquinolyloxy group and the like, and preferable are C₁ to C₁₂ alkoxypyridyloxy groups and C₁ to C₁₂ alkylpyridyloxy groups.

The C₁ to C₁₂ alkoxypyridyloxy group includes a methoxypyridyloxy group, an ethoxypyridyloxy group, a propyloxypyridyloxy group, an isopropyloxypyridyloxy group, a butoxypyridyloxy group, an isobutoxypyridyloxy group, a tert-butoxypyridyloxy group, a pentyloxypyridyloxy group, a hexyloxypyridyloxy group, a cyclohexyloxypyridyloxy group, a heptyloxypyridyloxy group, an octyloxypyridyloxy group, a 2-ethylhexyloxypyridyloxy group, a nonyloxypyridyloxy group, a decyloxypyridyloxy group, a 3,7-dimethyloctyloxypyridyloxy group, a lauryloxypyridyloxy group and the like.

The C₁ to C₁₂ alkylpyridyloxy group includes a methylpyridyloxy group, an ethylpyridyloxy group, a dimethylpyridyloxy group, a propylpyridyloxy group, a 1,3,5-trimethylpyridyloxy group, a methylethylpyridyloxy group, an isopropylpyridyloxy group, a butylpyridyloxy group, a sec-butylpyridyloxy group, an isobutylpyridyloxy group, a tert-butylpyridyloxy group, a pentylpyridyloxy group, an isoamylpyridyloxy group, a hexylpyridyloxy group, a heptylpyridyloxy group, an octylpyridyloxy group, a nonylpyridyloxy group, a decylpyridyloxy group, a dodecylpyridyloxy group and the like.

In the formula (1), the heteroarylthio group represented by R¹ to R⁶, R⁸ and R¹¹ to R²⁰ has a carbon atom number of usually 6 to 60, preferably 7 to 48. The heteroarylthio group includes a pyridylthio group, C₁ to C₁₂ alkoxypyridylthio groups, C₁ to C₁₂ alkylpyridylthio groups, an isoquinolylthio group and the like, and preferable are C₁ to C₁₂ alkoxypyridylthio groups and C₁ to C₁₂ alkylpyridylthio groups.

In the formula (1), the arylalkenyl group represented by R¹ to R⁶, R⁸ and R¹¹ to R²⁰ has a carbon atom number of usually 8 to 60, preferably 8 to 48. The arylalkenyl group includes phenyl C₂ to C₁₂ alkenyl groups ("C₂ to C₁₂ alkenyl" means that the alkenyl portion has a carbon atom number of 2 to 12. The same shall apply hereinafter.), C₁ to C₁₂ alkoxyphenyl C₂ to C₁₂ alkenyl groups, C₁ to C₁₂ alkylphenyl C₂ to C₁₂ alkenyl groups, 1-naphthyl C₂ to C₁₂ alkenyl groups, 2-naphthyl C₂ to C₁₂ alkenyl groups and the like, and preferable are C₁ to C₁₂ alkoxyphenyl C₂ to C₁₂ alkenyl groups and C₁ to C₁₂ alkylphenyl C₂ to C₁₂ alkenyl groups.

In the formula (1), the arylalkynyl group represented by R¹ to R⁶, R⁸ and R¹¹ to R²⁰ has a carbon atom number of usually 8 to 60, preferably 8 to 48. The arylalkynyl group includes phenyl C₂ to C₁₂ alkynyl groups ("C₂ to C₁₂ alkynyl" means that the alkynyl portion has a carbon atom number of 2 to 12. The same shall apply hereinafter.), C₁ to C₁₂ alkoxyphenyl C₂ to C₁₂ alkynyl groups, C₁ to C₁₂ alkylphenyl C₂ to C₁₂ alkynyl groups, 1-naphthyl C₂ to C₁₂ alkynyl groups, 2-naphthyl C₂ to C₁₂ alkynyl groups and the like, and preferable are C₁ to C₁₂ alkoxyphenyl C₂ to C₁₂ alkynyl groups and C₁ to C₁₂ alkylphenyl C₂ to C₁₂ alkynyl groups.

In the formula (1), the substituted carboxyl group represented by R¹ to R⁶, R⁸ and R¹¹ to R²⁰ has a carbon atom number of usually 2 to 60, preferably 2 to 48, and means a carboxyl group substituted by an alkyl group, an aryl group, an arylalkyl group or a monovalent heterocyclic group.

The substituted carboxyl group includes a methoxycarbonyl group, an ethoxycarbonyl group, a propyloxycarbonyl group, an isopropyloxycarbonyl group, a butoxycarbonyl group, an isobutoxycarbonyl group, a tert-butoxycarbonyl group, a pentyloxycarbonyl group, a hexyloxycarbonyl group, a cyclohexyloxycarbonyl group, a heptyloxycarbonyl group, an octyloxycarbonyl group, a 2-ethylhexyloxycarbonyl group, a nonyloxycarbonyl group, a decyloxycarbonyl group, a 3,7-dimethyloctyloxycarbonyl group, a dodecyloxycarbonyl group, a trifluoromethoxycarbonyl group, a pentafluoroethoxycarbonyl group, a perfluorobutoxycarbonyl group, a perfluorohexyloxycarbonyl group, a perfluorooctyloxycarbonyl group, a pyridyloxycarbonyl group, a naphthoxycarbonyl group and the like.

In the formula (1), at least one of atoms and groups represented by R¹ to R⁶, R⁸ and R¹¹ to R²⁰ is preferably an atom or group other than a hydrogen atom from the viewpoint of solubility, and R², R³, R¹², R¹³, R¹⁶, R¹⁷, R¹⁸ and R¹⁹ are preferably an atom or group other than a hydrogen atom and R³ and R¹³ are further preferably an atom or group other than a hydrogen atom from the viewpoint of easiness of synthesis of a metal complex.

In the formula (1), two or three of Z¹, Z², Z³, Z⁴ and Z⁵ are preferably a nitrogen atom.

As the metal complex represented by the formula (1), preferable are metal complexes in which several nitrogen atoms represented by Z¹, Z², Z³, Z⁴ and Z⁵ are not present at mutually adjacent positions and more preferable are metal complexes in which two or three of Z¹, Z², Z³, Z⁴ and Z⁵ are a nitrogen atom and these nitrogen atoms are not present at mutually adjacent positions, since the chemical stability of a ligand is enhanced in these structures.

In the formula (1), the substituent represented by R^{*} in -C(R*) = represented by Z¹, Z², Z³, Z⁴ and Z⁵ includes a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group, a cyano group and the like. These atoms and groups have the same meaning as described above.

As the metal complex represented by the formula (1), several geometric isomers are envisaged, and the metal complex represented by the formula (1) may be any geometric isomer.

As the metal complex represented by the formula (1), preferable is a metal complex represented by the above-described formula (1c), more preferable is a metal complex represented by the following formula (1a) or (1b) : 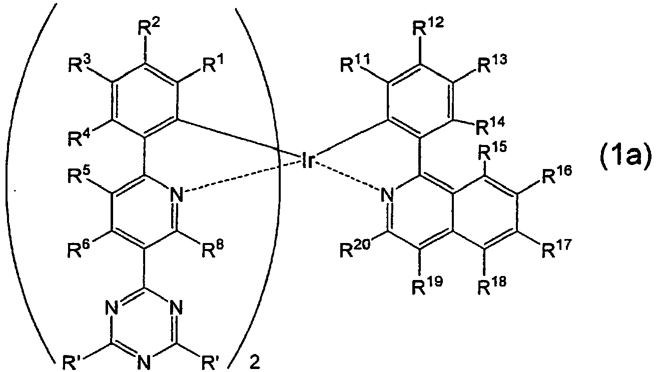 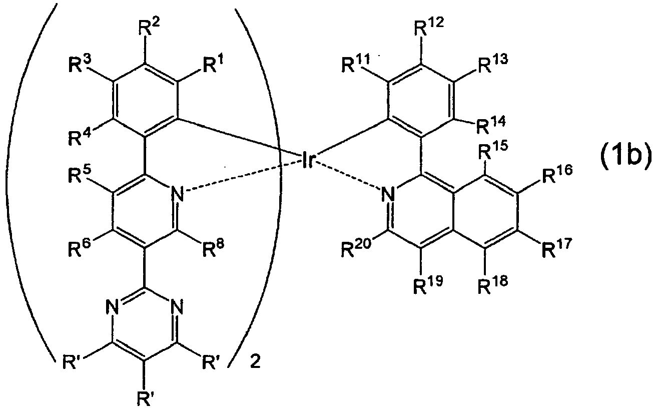
wherein R¹, R², R³, R⁴, R⁵, R⁶, R⁸, R¹¹, R¹², R¹³, R¹⁴, R¹⁵, R¹⁶, R¹⁷, R¹⁸, R¹⁹ and R²⁰ have the same meaning as described above;
R' represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent, wherein the plurality of R' moieties of may be the same or different, further preferable is a metal complex represented by the above-described formula (1a), from the viewpoint of easiness of synthesis of a compound as a ligand and light emission efficiency when used in a light emitting device.

The halogen atom, the alkyl group, the alkoxy group, the alkylthio group, the aryl group, the aryloxy group, the arylthio group, the arylalkyl group, the arylalkoxy group, the arylalkylthio group, the acyl group, the acyloxy group, the amide group, the acid imide group, the imine residue, the substituted amino group, the substituted silyl group, the substituted silyloxy group, the substituted silylthio group, the substituted silylamino group, the monovalent heterocyclic group, the heteroaryloxy group, the heteroarylthio group, the arylalkenyl group, the arylalkynyl group and the substituted carboxyl group represented by R' have the same meaning as described above.

A ligand constituting a metal complex exerts an influence on the light emission color, light emission intensity, light emission efficiency and the like of the metal complex. Preferable as the above-described ligand is a ligand which minimizes an energy deactivation process in the ligand.

The bidentate ligand of which number is defined by a subscript m in a metal complex represented by the formula (1) includes the following ligands. 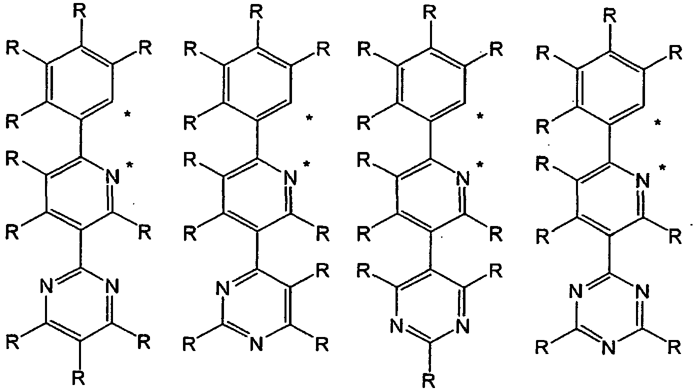 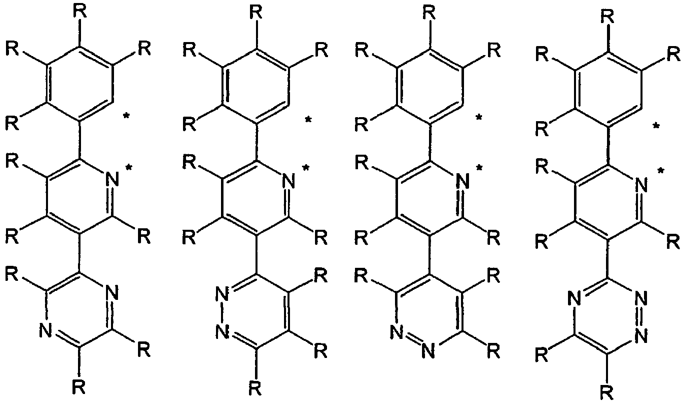 [wherein R represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group. * represents a binding site to an iridium atom. The plurality of R moieties may be the same or different.]

The halogen atom, the alkyl group, the alkoxy group, the alkylthio group, the aryl group, the aryloxy group, the arylthio group, the arylalkyl group, the arylalkoxy group, the arylalkylthio group, the acyl group, the acyloxy group, the amide group, the acid imide group, the imine residue, the substituted amino group, the substituted silyl group, the substituted silyloxy group, the substituted silylthio group, the substituted silylamino group, the monovalent heterocyclic group, the heteroaryloxy group, the heteroarylthio group, the arylalkenyl group, the arylalkynyl group and the substituted carboxyl group represented by R have the same meaning as explained and exemplified for the above-described R¹ to R⁶, R⁸ and R¹¹ to R²⁰.

The bidentate ligand compound of which number is defined by a subscript m includes preferably the following compounds.

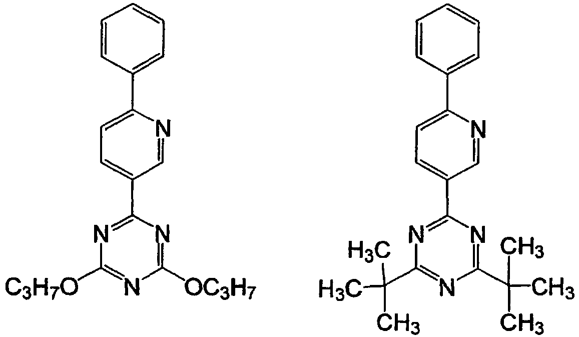

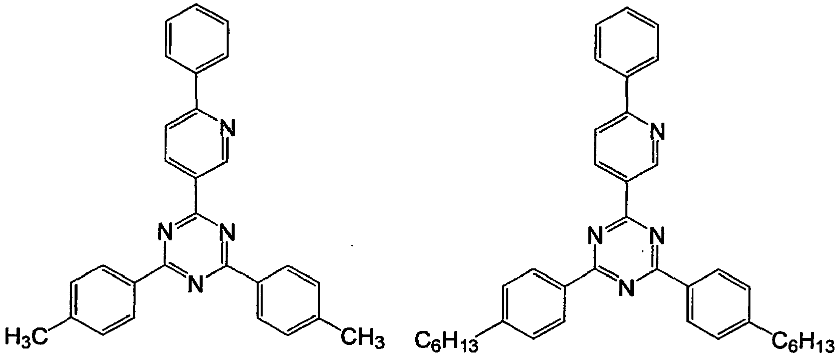

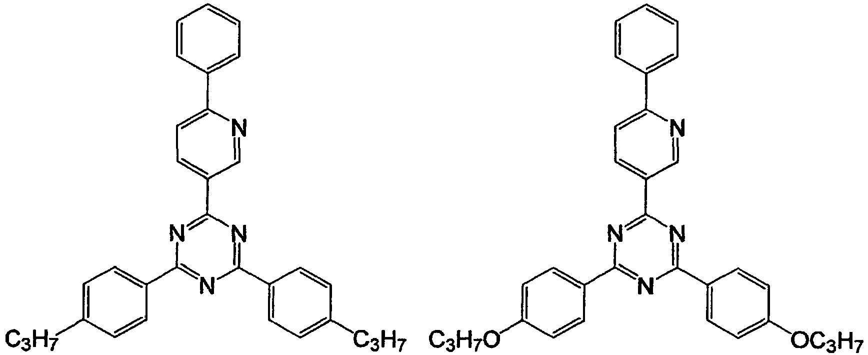

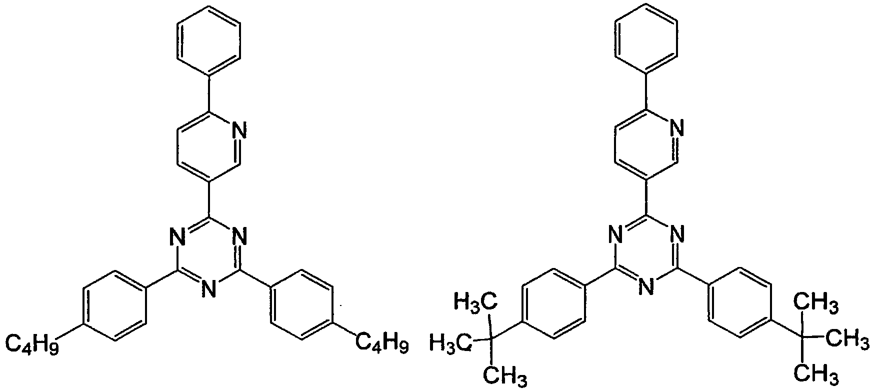

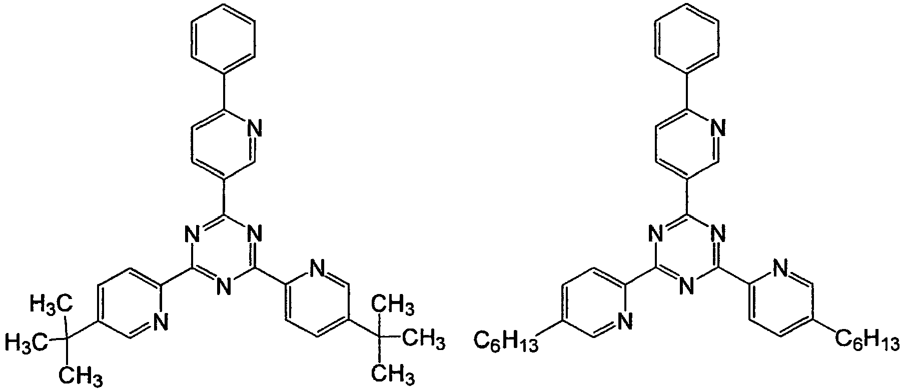

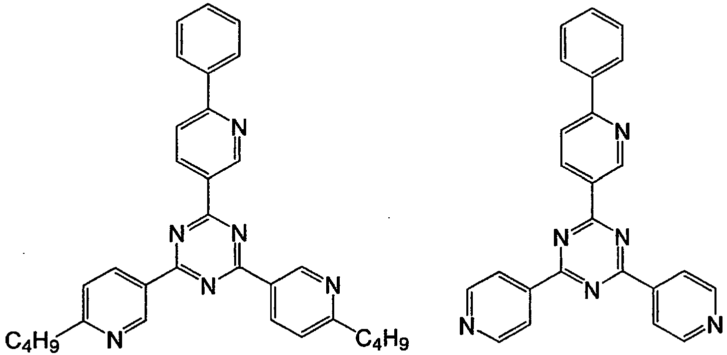

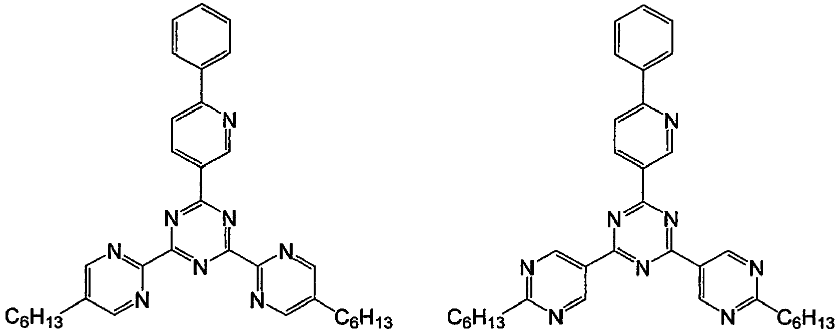

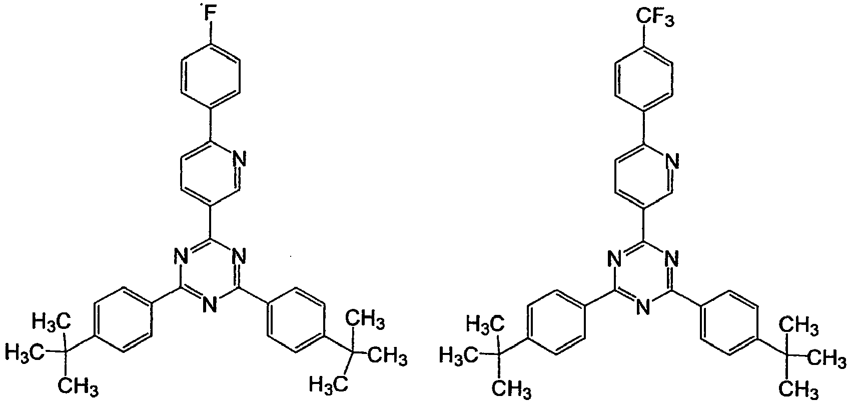

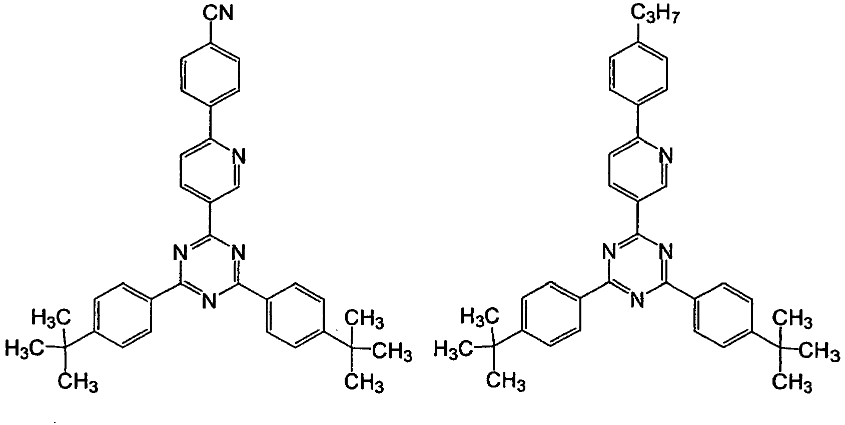

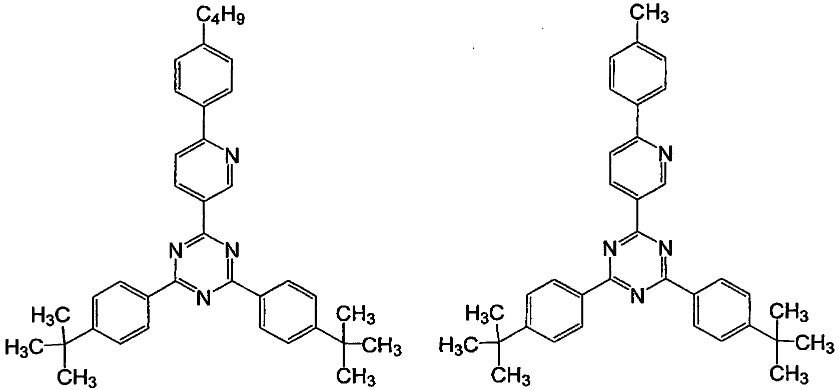

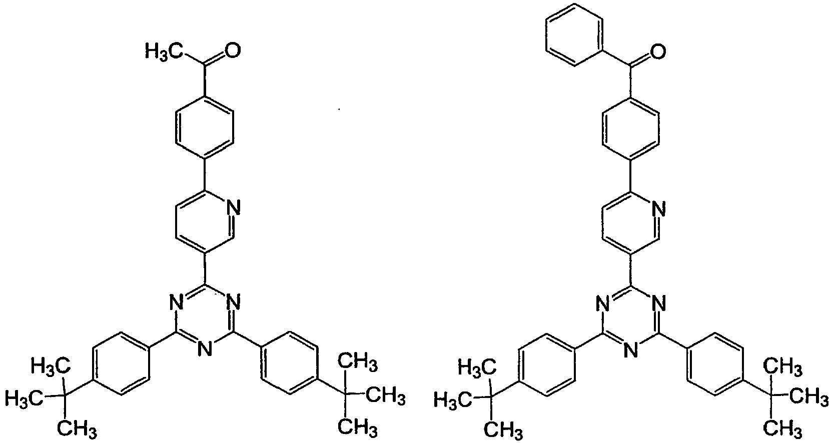

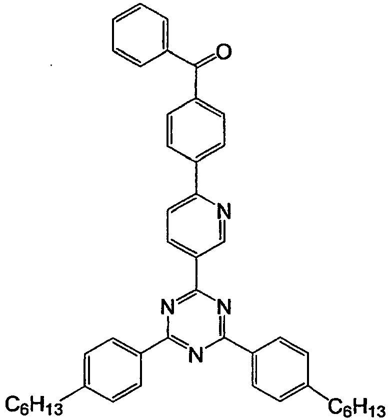

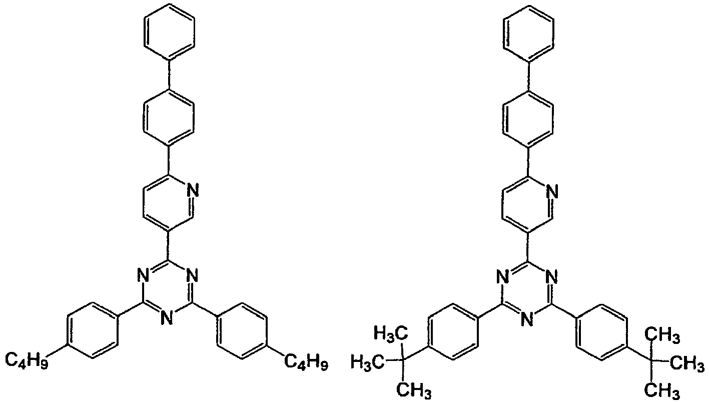

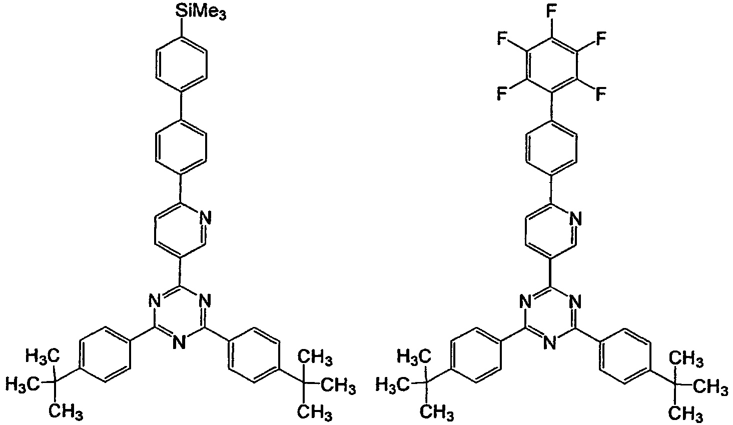

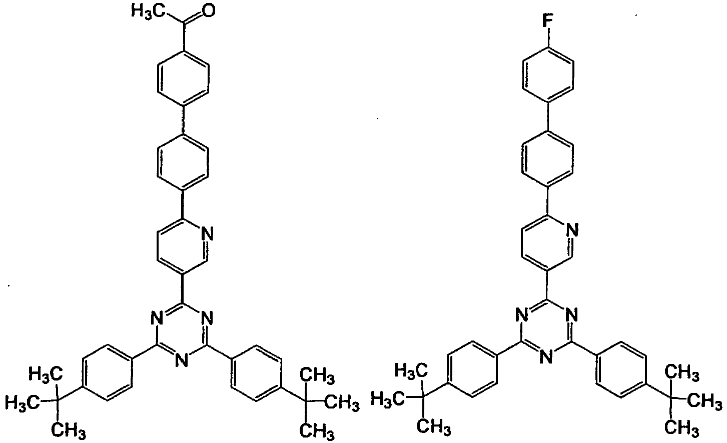

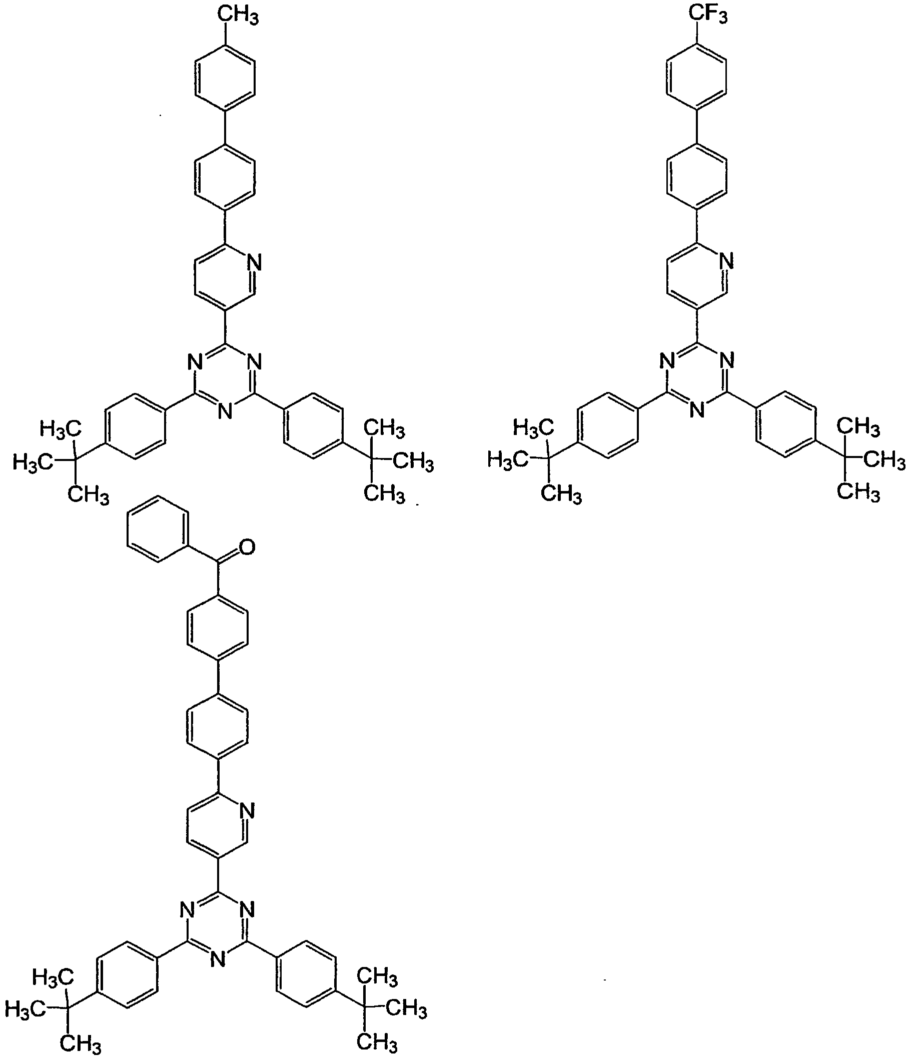

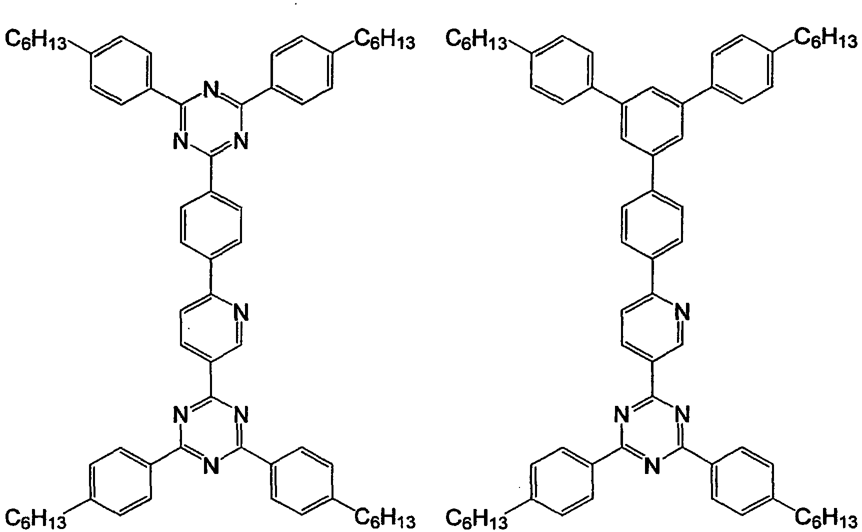

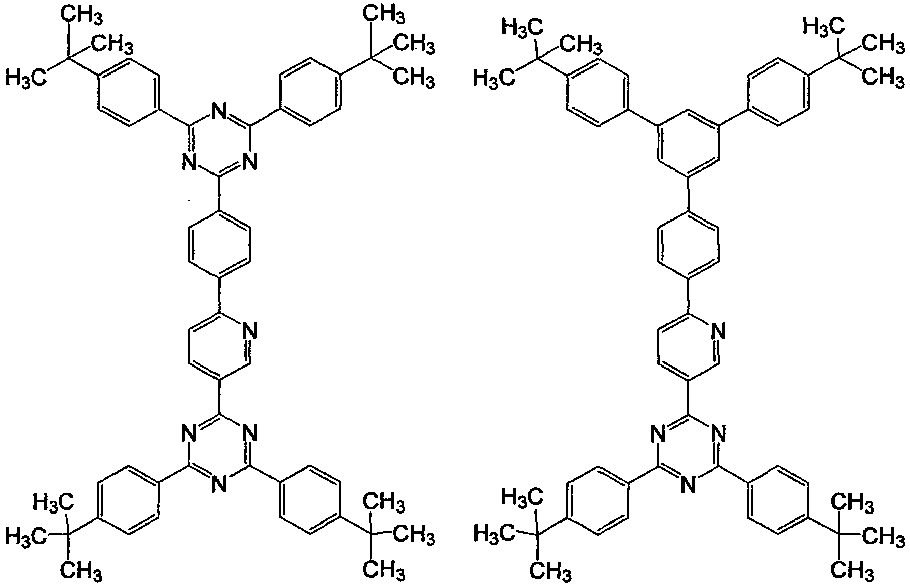

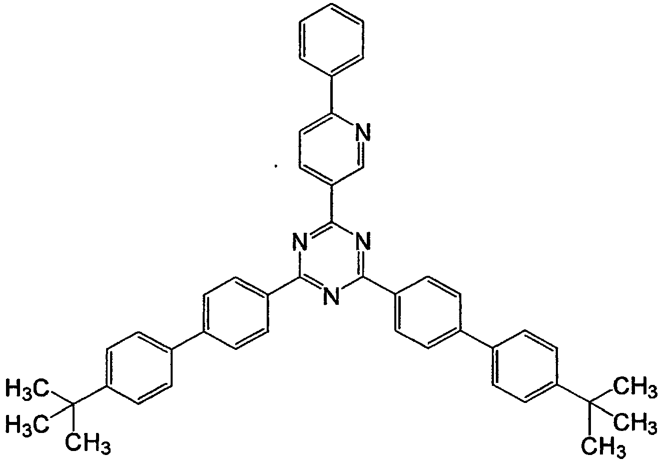

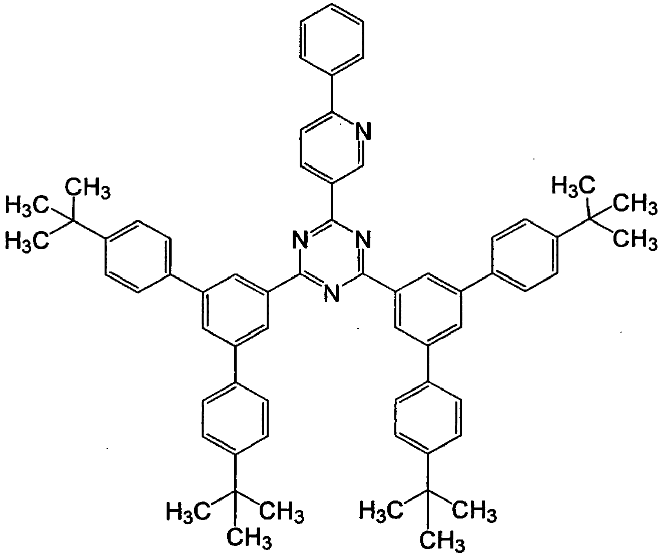

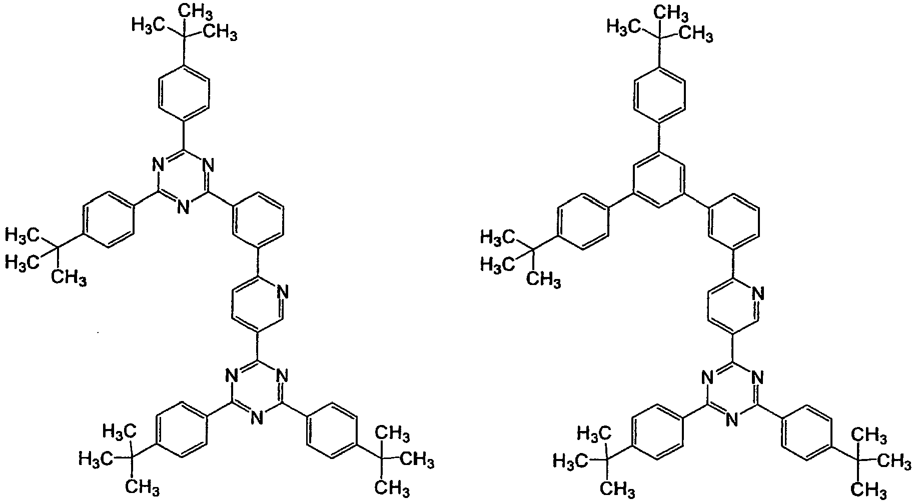

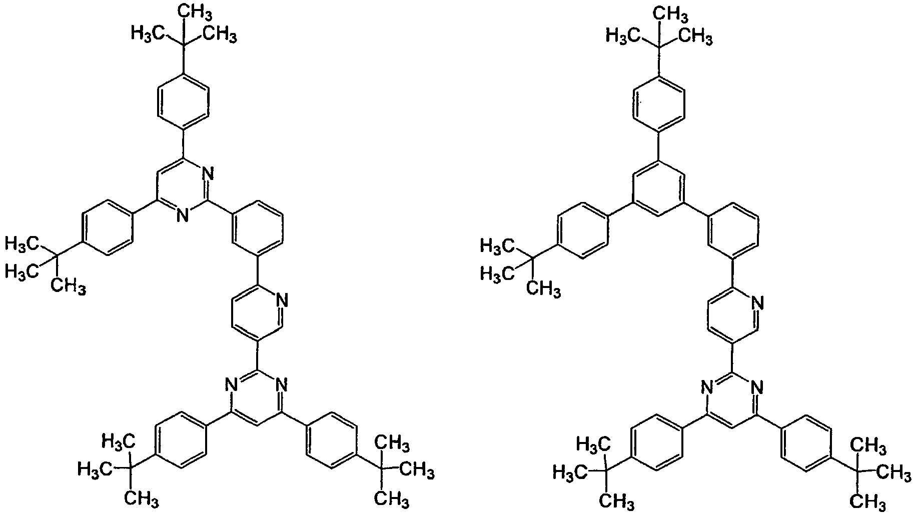

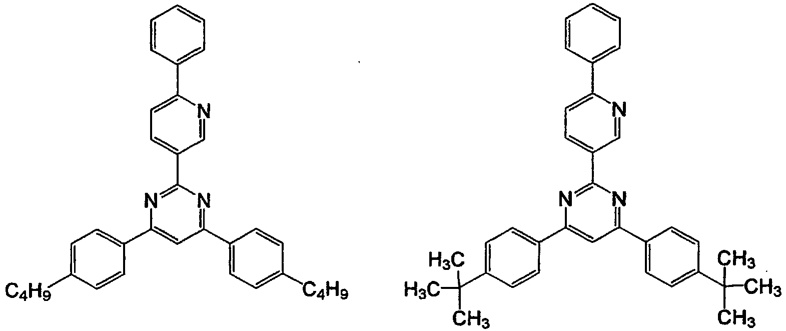

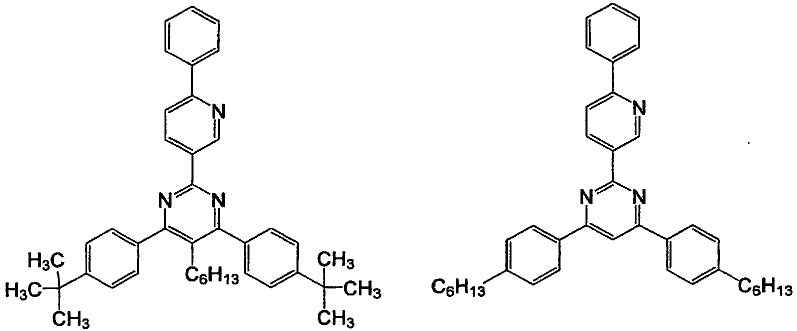

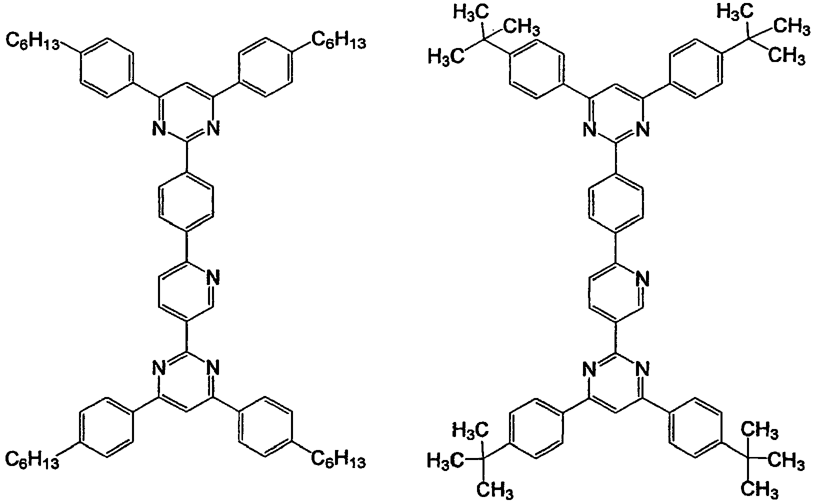

The bidentate ligand of which number is defined by a subscript 3-m in a metal complex represented by the formula (1) includes the following ligands. 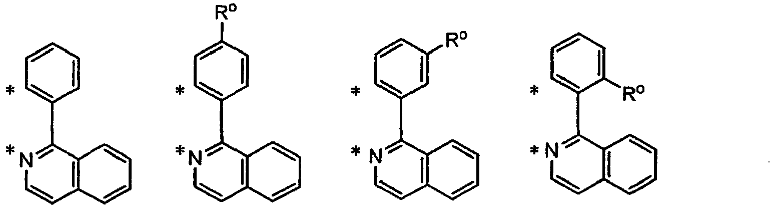 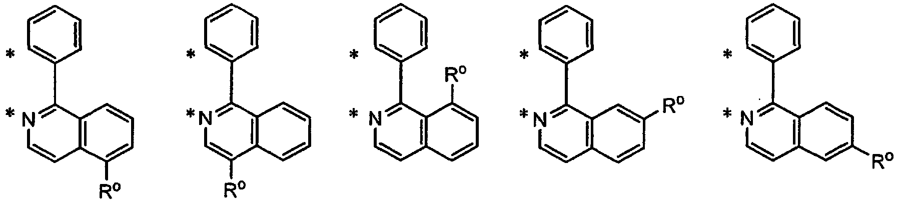 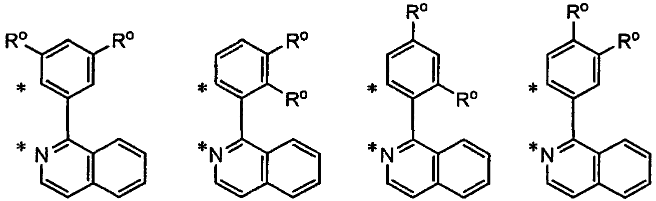 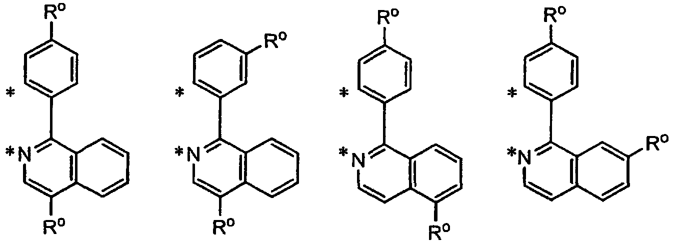 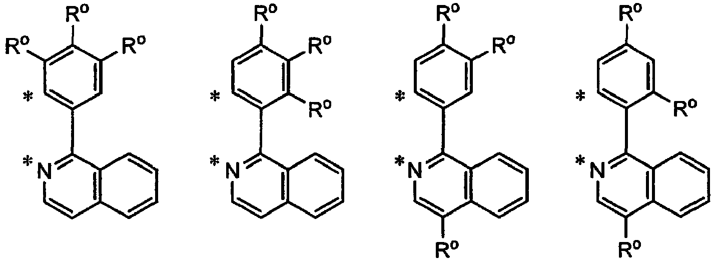 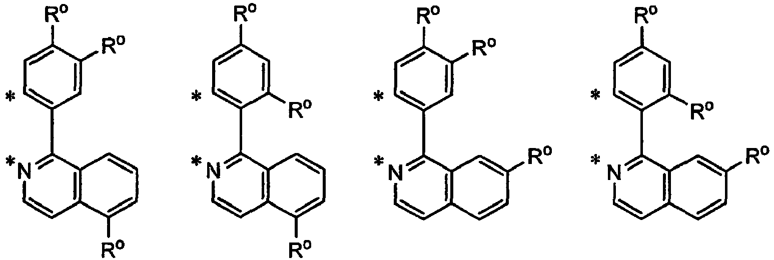 [wherein * has the same meaning as described above. R^{o} represents a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group. The plurality of R^{o} moieties may be the same or different.]

The above-described bidentate ligand compound of which number is defined by a subscript 3-m includes preferably the following compounds.

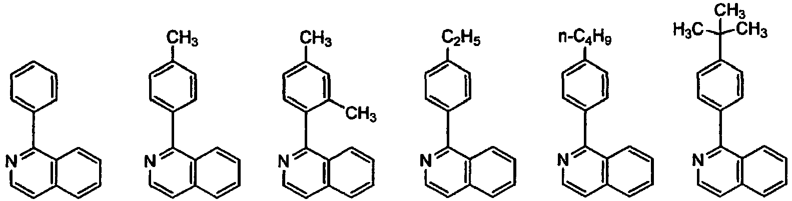

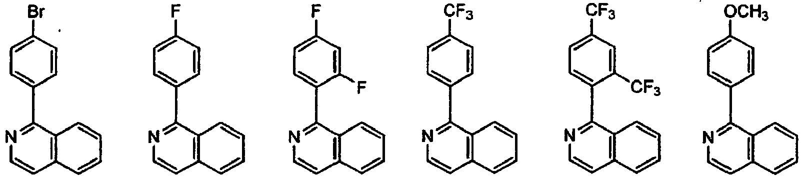

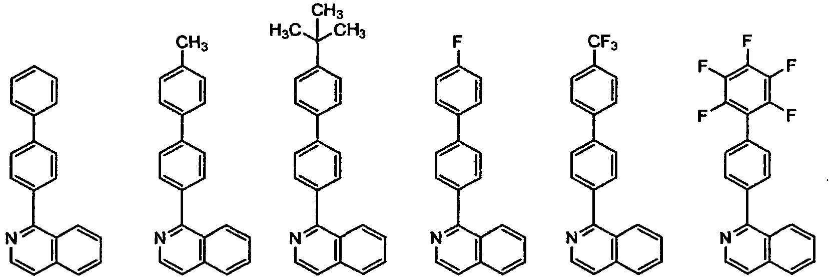

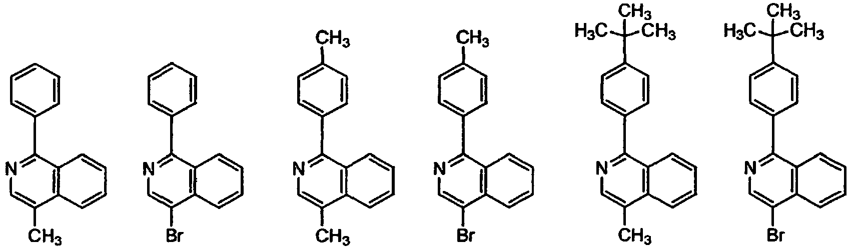

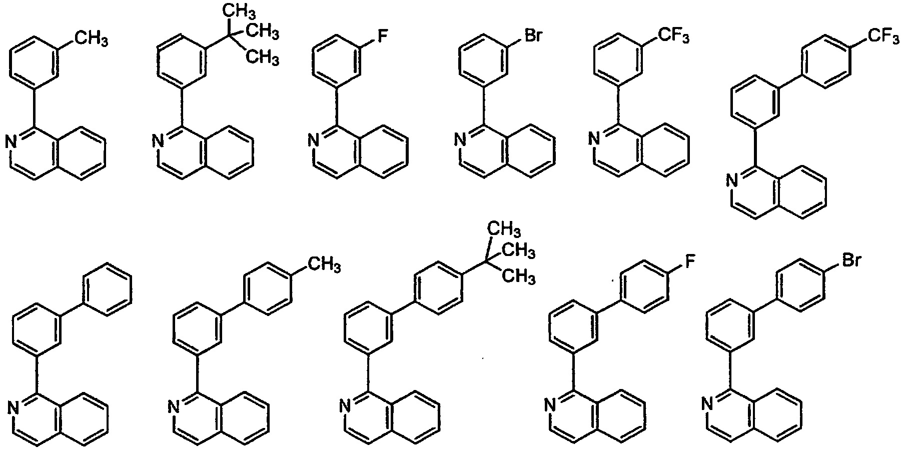

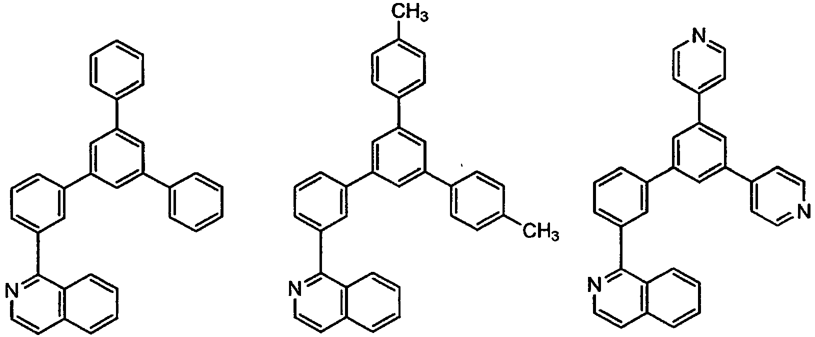

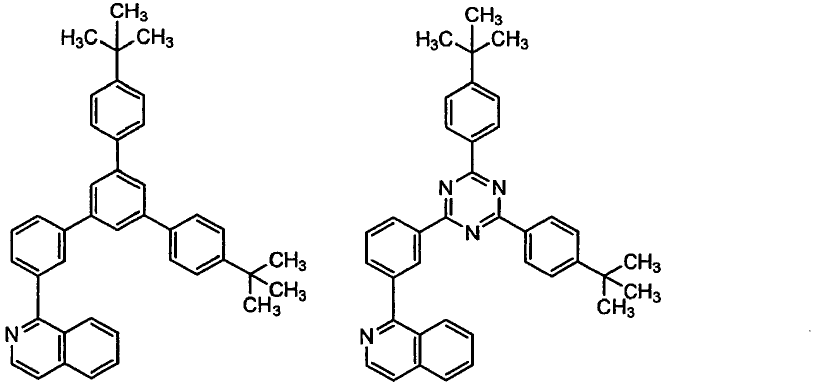

The metal complex represented by the formula (1a) includes the following metal complexes.

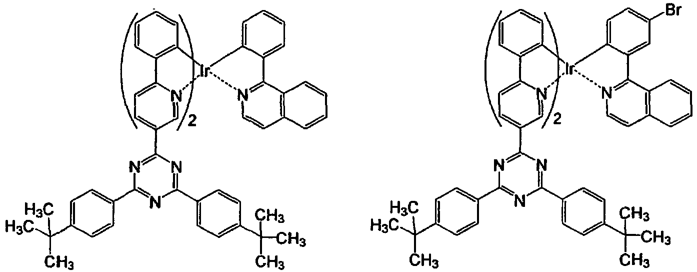

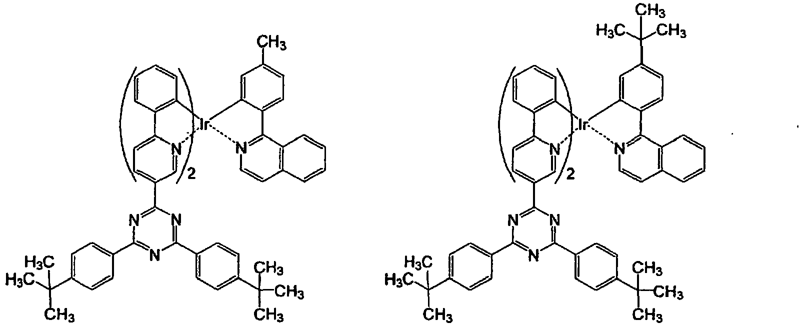

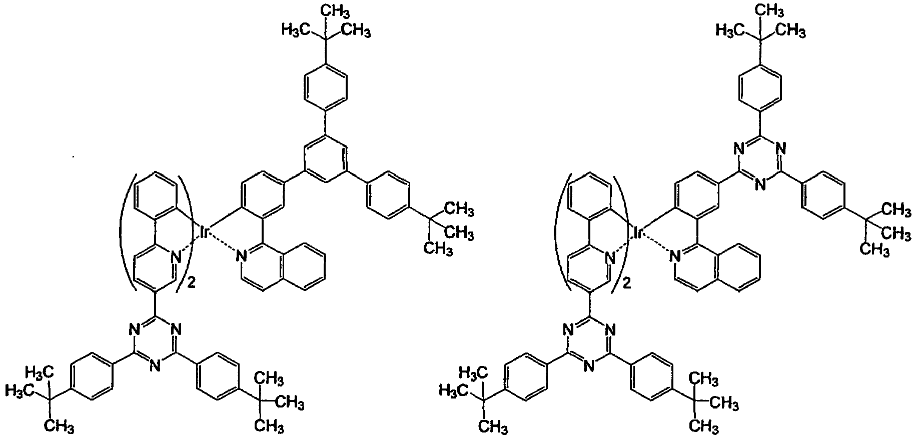

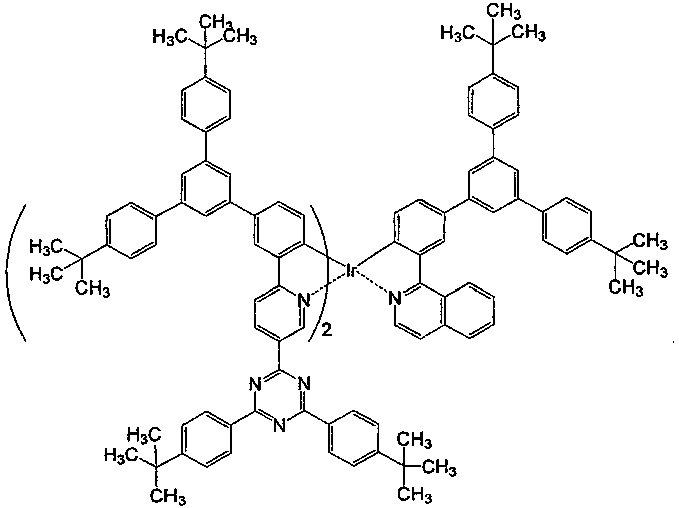

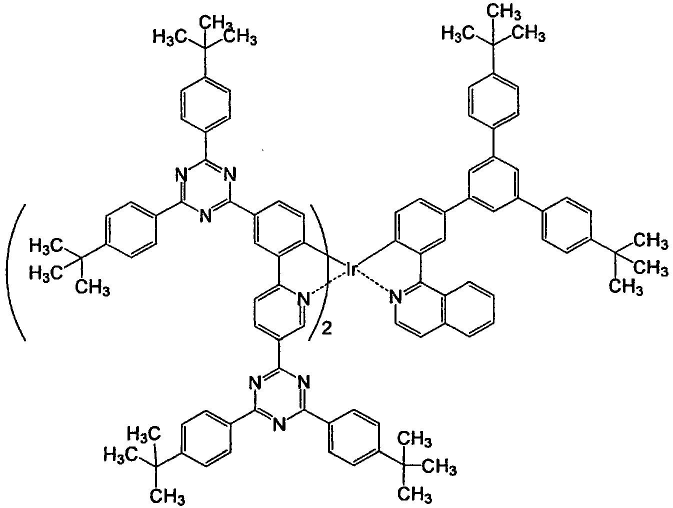

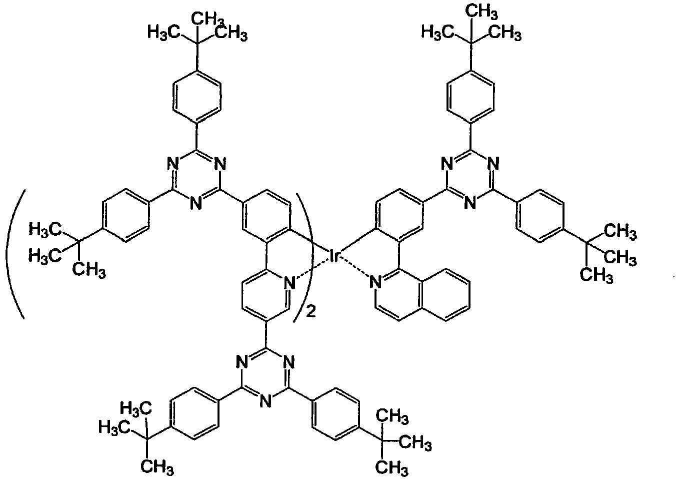

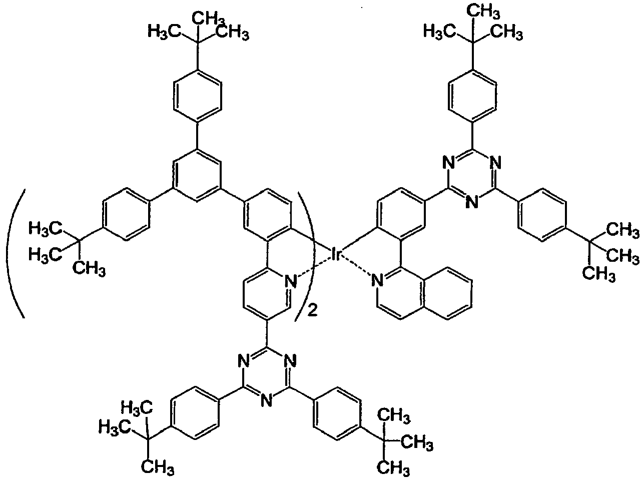

The metal complex represented by the formula (1b) includes the following metal complexes.

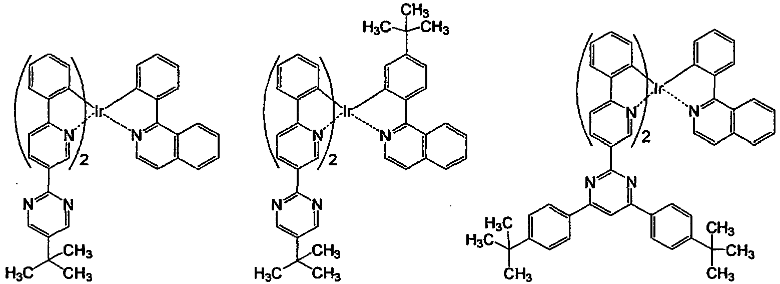

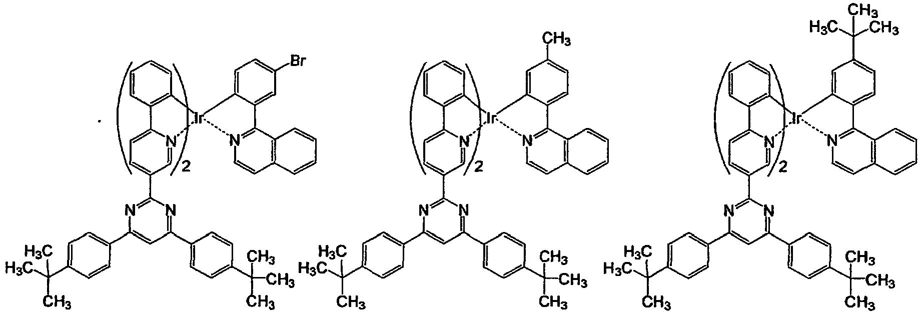

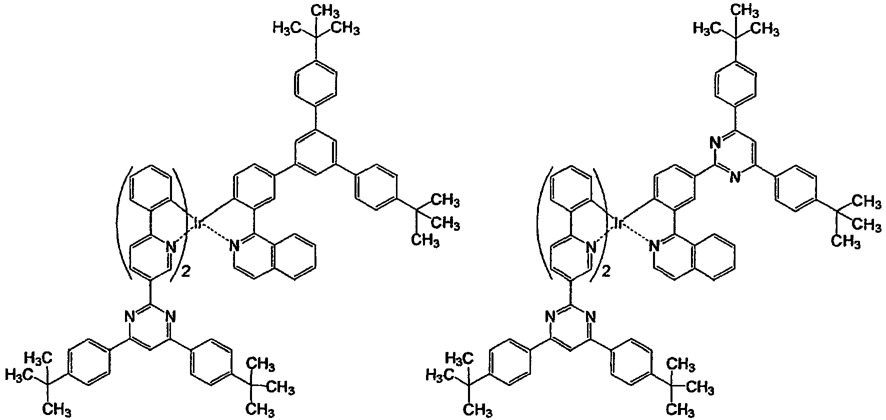

The metal complexes of the present invention may be used singly or in combination, or may also be combined with other components to give a composition.

### <First composition>

The first composition of the present invention contains a metal complex of the present invention. It is preferable that the composition contains only one metal complex of the present invention from the viewpoint of the light emission efficiency of a light emitting device obtained when used in fabrication of a light emitting device, and it is preferable that the composition contains two or more metal complexes of the present invention from the viewpoint of simplicity of purification of metal complexes.

When the first composition of the present invention contains two or more metal complexes of the present invention, the combination of two or more metal complexes includes
(i) a combination of a metal complex represented by the above-described formula (1a) and a metal complex represented by the above-described formula (1b),
(ii) a combination of different metal complexes represented by the above-described formula (1a),
(iii) a combination of different metal complexes represented by the above-described formula (1b),
(iv) a combination of a metal complex represented by the above-described formula (1c) and a metal complex represented by the following formula (1d): 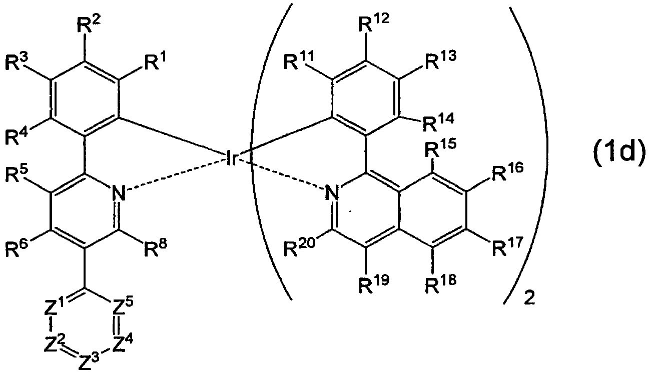
wherein R¹ R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above,
and the like, and the above-described combination (iv) is preferable since purification of metal complexes is easy.

In the above-described combination (iv), the amount of a metal complex represented by the above-described formula (1c) is preferably 80 to 99.99 parts by weight and more preferably 90 to 99.99 parts by weight and the amount a metal complex represented by the above-described formula (1d) is preferably 0.01 to 20 parts by weight and more preferably 0.01 to 10 parts by weight, with respect to 100 parts by weight of the total amount of the combination, since light emission efficiency is high in this range.

In the above-described combination (i), the amount of a metal complex represented by the above-described formula (1a) is preferably 50 to 99.99 parts by weight and more preferably 70 to 99.99 parts by weight and the amount of a metal complex represented by the above-described formula (1b) is preferably 0.01 to 50 parts by weight and more preferably 0.01 to 30 parts by weight, with respect to 100 parts by weight of the total amount of the combination.

The first composition of the present invention is preferably a composition comprising a metal complex of the present invention, and a metal complex represented by the following formula (2), a metal complex represented by the following formula (3) or a combination thereof, since a metal complex can be synthesized easily: 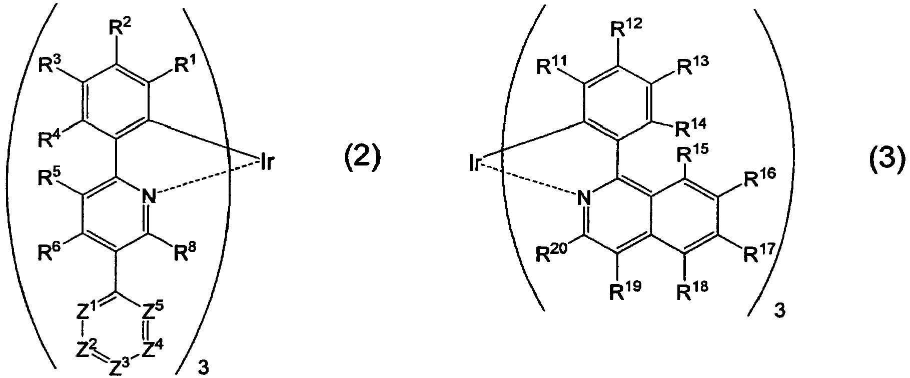 wherein R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above.

When the first composition of the present invention contains a metal complex represented by the formula (2) and/or a metal complex represented by the formula (3), the combination of metal complexes contained in the composition includes
(v) a combination of a metal complex represented by the formula (1), (1a), (1b) or (1c) and a metal complex represented by the formula (2),
(vi) a combination of a metal complex represented by the formula (1), (1a), (1b) or (1c) and a metal complex represented by formula (3),
(vii) a combination of a metal complex represented by the formula (1), (1a), (1b) or (1c), a metal complex represented by the formula (2) and a metal complex represented by the formula (3),
(viii) a combination of a metal complex represented by the formula (1c), a metal complex represented by the formula (1d) and a metal complex represented by the formula (2),
and the like, and preferable are the above-described combination (v) and the above-described combination (viii), since light emission efficiency is excellent.

In the above-described combination of (v), the amount of a metal complex represented by the formula (1), (1a), (1b) or (1c) is preferably 50 to 99.99 parts by weight and more preferably 80 to 99.99 parts by weight and the amount of a metal complex represented by formula (2) is preferably 0.01 to 50 parts by weight and more preferably 0.01 to 20 parts by weight, with respect to 100 parts by weight of the total amount of the combination, since synthesis of a metal complex is easy.

In the above-described combination (viii), the amount of a metal complex represented by the formula (1c) is preferably 50 to 99.98 parts by weight and more preferably 80 to 99.98 parts by weight, the amount of a metal complex represented by the formula (1d) is preferably 0.01 to 20 parts by weight and more preferably 0.01 to 10 parts by weight and the amount of a metal complex represented by formula (2) is preferably 0.01 to 49.99 parts by weight and more preferably 0.01 to 19.99 parts by weight, with respect to 100 parts by weight of the total amount of the combination.

In the above-described combination (vi), the amount of a metal complex represented by the formula (1), (1a), (1b) or (1c) is preferably 50 to 99.99 parts by weight and more preferably 80 to 99.99 parts by weight and the amount of a metal complex represented by the formula (3) is preferably 0.01 to 50 parts by weight and more preferably 0.01 to 20 parts by weight, with respect to 100 parts by weight of the total amount of the combination.

In the above-described combination (vii), the amount of a metal complex represented by the formula (1), (1a), (1b) or (1c) is preferably 50 to 99.98 parts by weight and more preferably 80 to 99.98 parts by weight, the amount of a metal complex represented by formula (2) is preferably 0.01 to 49.99 parts by weight and more preferably 0.01 to 19.99 parts by weight and the amount of a metal complex represented by the formula (3) is preferably 0.01 to 20 parts by weight and more preferably 0.01 to 10 parts by weight, with respect to 100 parts by weight of the total amount of the combination.

The first composition of the present invention may further contain at least one member selected from the group consisting of charge transporting materials, light emitting materials, solvents and dispersing media. The components contained in the first composition of the present invention may each be used singly or in combination.

The charge transporting materials are classified into hole transporting materials and electron transporting materials, further, these materials are each classified into low molecular weight organic compounds and high molecular weight organic compounds. The hole transporting material is a material mainly effecting transportation of holes from an anode to a light emitting layer. The electron transporting material is a material mainly effecting transportation of electrons from a cathode to a light emitting layer.

The low molecular weight organic compound as the hole transporting material includes compounds known as a hole transporting material of an organic EL device, such as arylamine derivatives, carbazole derivatives, pyrazoline derivatives, stilbene derivatives and the like. Specifically mentioned are compounds described in "Organic EL Display" (written under joint authorship by Shizuo Tokito, Chihaya Adachi and Hideyuki Murata, Ohmsha, Ltd.) p. 102 and p. 107.

The high molecular weight organic compound as the hole transporting material includes polymer compounds containing in its main chain a thienylene group, a pyrrolediyl group, a 2,5-thienylenevinylene group, a p-phenylenevinylene group, a group represented by the formula (8) described later or the like as a repeating unit, and copolymers with these repeating units, each of which may have a substituent. Also polyvinylcarbazole and derivatives thereof, polysilane and derivatives thereof, polysiloxane derivatives having an aromatic amine in the side chain or the main chain and polyaniline and derivatives thereof are mentioned as examples of the high molecular weight organic compound as the hole transporting material.

The low molecular weight organic compound as the electron transporting material includes compounds known as an electron transporting material of an organic EL device, for example, oxadiazole derivatives, triazole derivatives, phenylquinoxaline derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, and metal complexes of 8-hydroxyquinoline and derivatives thereof. Specifically mentioned are compounds described in "Organic EL Display" (written under joint authorship by Shizuo Tokito, Chihaya Adachi and Hideyuki Murata, Ohmsha, Ltd.) p. 105 and p. 107 and JP-A No. 2004-277377.

The high molecular weight organic compound as the electron transporting material includes polymer compounds containing in its main chain a quinolinediyl group, a quinoxalinediyl group, a fluorenediyl group or the like as a repeating unit, and copolymers with these repeating units, each of which may have a substituent.

The high molecular weight organic compound as the charge transporting material can also be a polymer compound having simultaneously hole transport properties and electron transport properties, containing the repeating unit of the polymer compound mentioned for the hole transporting material and the repeating unit of the polymer compound mentioned for the electron transporting material.

The high molecular weight organic compound includes polymer compounds having an optionally substituted benzene ring, and polymers described in JP-A No. 2003-231741, JP-A No. 2004-059899, JP-A No. 2004-002654, JP-A No. 2004-292546, WO99/54385, WO00/46321, WO02/077060, "Organic EL Display" (written under joint authorship by Shizuo Tokito, Chihaya Adachi and Hideyuki Murata, Ohmsha, Ltd.) pp. 111 to 113, and the like.

For obtaining excellent light emission from a metal complex, preferable among the charge transporting materials are compounds having lowest triplet excitation energy larger than the lowest triplet excitation energy of the metal complex.

In the case of use of a high molecular weight organic compound as the charge transporting material, the high molecular weight organic compound has a polystyrene-equivalent number-average molecular weight of preferably 1×10³ to 1×10⁸, more preferably 1×10⁴ to 1×10⁶. The high molecular weight organic compound has a polystyrene-equivalent weight-average molecular weight of preferably 1×10³ to 1×10⁸, more preferably 5×10⁴ to 5×10⁶.

The light emitting material includes naphthalene derivatives, anthracene and derivatives thereof, perylene and derivatives thereof, polymethine dyes, xanthene dyes, coumarin dyes, cyanine dyes, metal complexes of 8-hydroxyquinoline and derivatives thereof, aromatic amines, tetraphenylcyclopentadiene and derivatives thereof, tetraphenylbutadiene and derivatives thereof, and the like.

The solvent or dispersing medium may advantageously be one which is capable of uniformly dissolving or dispersing a solid component contained in a composition, and examples thereof include chlorine-based solvents (chloroform, methylene chloride, 1,2-dichloroethane, 1,1,2-trichloroethane, chlorobenzene, o-dichlorobenzene and the like), ether solvents (tetrahydrofuran, dioxane and the like), aromatic hydrocarbon solvents (benzene, toluene, xylene and the like), aliphatic hydrocarbon solvents (cyclohexane, methylcyclohexane, n-pentane, n-hexane, n-heptane, n-octane, n-nonane, n-decane and the like), ketone solvents (acetone, methyl ethyl ketone, cyclohexanone and the like), ester solvents (ethyl acetate, butyl acetate, ethyl cellosolve acetate and the like), polyhydric alcohols and derivatives thereof (ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexane diol and the like), alcohol solvents (methanol, ethanol, propanol, 2-propanol, cyclohexanol and the like), sulfoxide solvents (dimethyl sulfoxide and the like) and amide solvents (N-methyl-2-pyrrolidone, N,N-dimethylformamide and the like).

When the first composition of the present invention contains a solvent or a dispersing medium and the composition is applied to an inkjet method, it is preferable that the composition contains a solvent having high boiling point such as anisole, bicyclohexylbenzene and the like and it is more preferable that the composition has a viscosity at 25°C of 1 to 100 mPa·s, for suppressing vaporization of a solvent or a dispersing medium from a nozzle.

When the first composition of the present invention contains at least one member selected from the group consisting of a charge transporting material, a light emitting material, a solvent and a dispersing medium, the amount of the metal complex of the present invention is usually 0.1 to 90 parts by weight, preferably 0.1 to 80 parts by weight and more preferably 0.5 to 50 parts by weight, with respect to 100 parts by weight of the total amount of the composition.

### <Production method of metal complex>

The metal complex of the present invention may be produced by any method, and for example, can be produced by reacting a compound acting as a ligand and an iridium compound in a solution. In the above-described reaction, a base and a silver salt compound may be present in the reaction system.

The above-described reaction method includes methods described in J. Am. Chem. Soc. 1984, 106, 6647; Inorg. Chem. 1991, 30, 1685; Inorg. Chem. 1994, 33, 545; Inorg. Chem. 2001, 40, 1704; and Chem. Lett., 2003, 32, 252.

The reaction temperature of the above-described reaction is usually from the melting point to the boiling point of a solvent present in the reaction system, and preferably from -78°C to the boiling point of a solvent. In the case of use of a micro wave reaction apparatus in the above-described reaction, the reaction can be carried out at a temperature of not lower than the boiling point of a solvent.

The reaction time of the above-described reaction is usually 30 minutes to 150 hours. In the case of use of a micro wave reaction apparatus in the above-described reaction, the reaction time is usually several minutes to several hours.

The compound acting as a ligand can be synthesized by the Suzuki coupling, the Grignard coupling, the Stille coupling and the like of a 2-phenylpyridine derivative and a heterocyclic aromatic compound. For example, the above-described compound acting as a ligand can be synthesized by dissolving a 2-phenylpyridine derivative and a heterocyclic aromatic compound in an organic solvent, and reacting them at a temperature of the melting point or higher and the boiling point or lower of the organic solvent using an alkali and a suitable catalyst. This synthesis can refer to "Organic Syntheses", Collective Volume VI, pp. 407 to 411, John Wiley & Sons, Inc., 1988; Chem. Rev., vol. 106, p. 2651 (2006); Chem. Rev., vol. 102, p. 1359 (2002); Chem. Rev., vol. 95, p. 2457 (1995); and J. Organomet. Chem., vol. 576, p. 147 (1999).

The heterocyclic aromatic compound can be synthesized by methods described in "HOUBEN-WEYL METHODS OF ORGANIC CHEMISTRY 4TH EDITION", vol. E9b, p. 1, GEORG THIEME VERLAG STUTTGART; HOUBEN-WEYL METHODS OF ORGANIC CHEMISTRY 4TH EDITION, vol. E9c, p. 667, GEORG THIEME VERLAG STUTTGART, and the like.

As the catalyst used in the coupling reaction, palladium catalysts are preferable.

Preferable as the palladium catalyst are palladium acetate, bis(triphenylphosphine)palladium(II) dichloride, tetrakis(triphenylphosphine)palladium(0), [1,1'-bis(diphenylphosphino)ferrocene]dichloropalladium(II) and tris(dibenzylideneacetone)dipalladium(0), and preferable are tetrakis(triphenylphosphine)palladium(0), [1,1'-bis(diphenylphosphino)ferrocene]dichloropalladium(II) and tris(dibenzylideneacetone)dipalladium(0).

The palladium catalyst may also be used together with a phosphorus compound such as triphenylphosphine, tri(o-tolyl)phosphine, tri(tert-butyl)phosphine, tricyclohexylphosphine, 1,1'-bis(diphenylphosphino)ferrocene and the like.

The compound acting as a ligand includes the same examples as for the above-described compound acting as a bidentate ligand of which number is defined by a subscript m and the above-described compound acting as a bidentate ligand of which number is defined by a subscript 3-m.

The compound acting as a bidentate ligand of which number is defined by a subscript m can be synthesized, for example, by the following scheme.

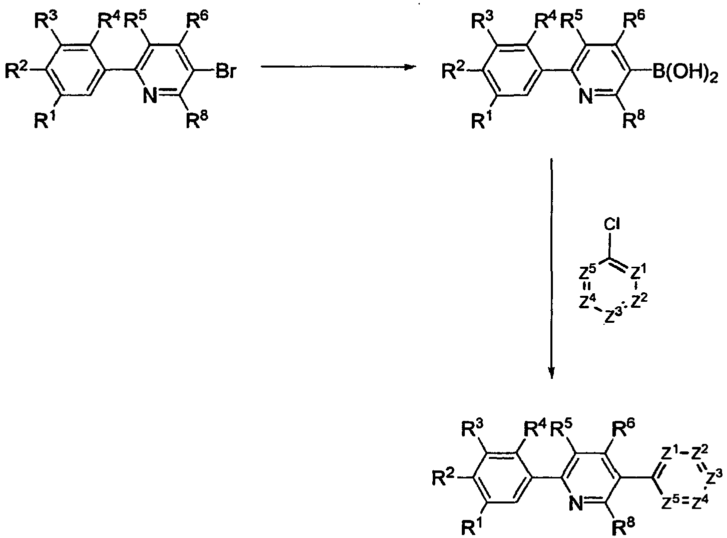

The compound acting as a bidentate ligand of which number is defined by a subscript 3-m (namely, the compound represented by the formula (5)) can be synthesized, for example, by the following scheme, and methods described in JP-A No. 2008-231042 and Advanced Functional Materials vol. 15, p. 387 (2005).

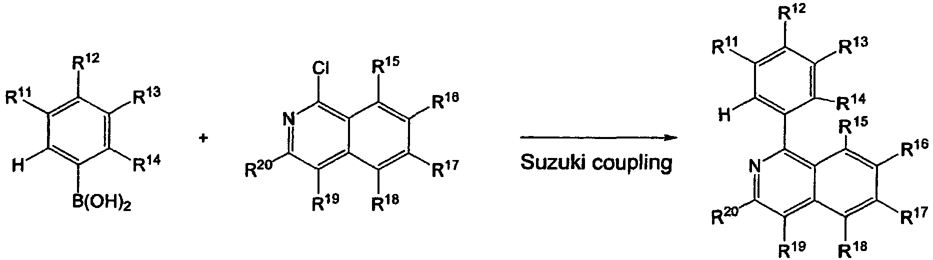

A method for producing the metal complex represented by the formula (1c) which is a typical metal complex among metal complexes of the present invention will be illustrated specifically using a general formula.

The metal complex represented by the formula (1c) can be produced, for example, by a method comprising reacting a metal complex represented by the formula (4) and a compound represented by the above-described formula (5). More specifically, a metal complex represented by the formula (4) and a compound represented by the formula (5) are reacted in the presence of a silver salt compound such as silver trifluoromethanesulfonate and the like. The amount of the compound represented by the formula (5) is usually 1.5 to 30-fold mol, preferably 2 to 10-fold mol, with respect to the metal complex represented by the formula (4). The amount of the silver salt compound is usually 1.5 to 20-fold mol, preferably 2 to 5-fold mol, with respect to the metal complex represented by the formula (4). The reaction temperature is usually in the range from the melting point to the boiling point of a solvent present in the reaction system, preferably from -78°C to the boiling point of a solvent, and from the viewpoint of the purity of a metal complex, more preferably in the range of 50°C to 160°C, further preferably in the range of 70°C to 130°C.

The reaction time is usually in the range of 30 minutes to 72 hours, preferably in the range of 1 hour to 48 hours.

The metal complex represented by the formula (4) can be produced, for example, by reacting a compound represented by 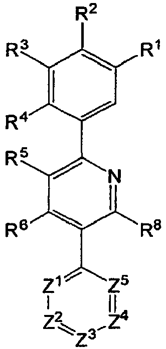 and iridium trichloride.

The reaction is carried out usually in a solvent. As the solvent, a polar solvent is preferably used for progressing the reaction smoothly. Such a solvent includes methanol, ethanol, 1-propanol, ethylene glycol, glycerin, 2-methoxyethanol, 2-ethoxyethanol, N,N-dimethylformamide and the like. The solvent may also be a mixed solvent composed of the above-described polar solvent and water. Examples of the mixed solvent include a water/2-ethoxyethanol mixed solvent. The polar solvent/water can be used at any mixing ratio, and the ratio is preferably in the range of 10/1 to 1/1 since a compound represented by the formula (5) can be dissolved at high temperatures in this range.

The reaction time is usually in the range of 30 minutes to 50 hours, preferably in the range of 30 minutes to 24 hours. The reaction temperature is usually in the range from the melting point to the boiling point of a solvent present in the reaction system, preferably from-78°C to the boiling point of a solvent, more preferably in the range of 50°C to 200°C.

The metal complex represented by the formula (1c) can also be produced, for example, by a method comprising reacting a metal complex represented by the formula (6) and a compound represented by the formula (5).

In the formula (6), the alkyl group, alkoxy group, aryl group and monovalent heterocyclic group represented by R^{e}, R^{f} and R^{g} have the same meaning as described above.

The metal complex represented by the formula (6) can be produced by reacting a metal complex represented by the formula (4) and a compound represented by 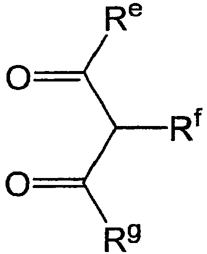 (1,3-dicarbonyl compound).

Specifically, to a solution of a metal complex represented by the formula (4) is added the above-described 1,3-dicarbonyl compound (for example, 1,3-diketone such as acetylacetone and the like), and a base such as sodium carbonate and the like is added and the mixture can be reacted. The amount of the 1,3-dicarbonyl compound is usually 2 to 50-fold mol, preferably 2 to 20-fold mol, with respect to the metal complex represented by the formula (4). The reaction time is usually in the range of 30 minutes to 50 hours, preferably in the range of 30 minutes to 24 hours. The reaction temperature is usually in the range from the melting point to the boiling point of a solvent present in the reaction system, preferably from-78°C to the boiling point of a solvent, more preferably in the range of 20°C to 100°C.

The metal complex represented by the formula (1d) can be synthesized, for example, by the following scheme. 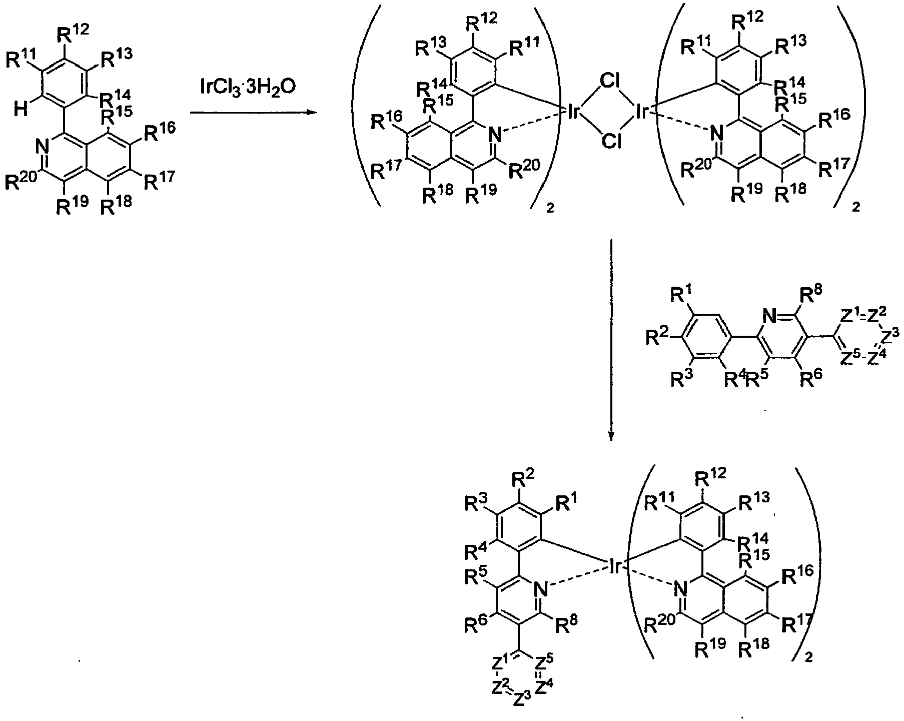 [wherein R¹ to R⁶, R⁸, R¹¹ to R²⁰ and Z¹ to Z⁵ have the same meaning as described above.]

The metal complex represented by the above-described formula (1d) can be synthesized also by the following scheme. 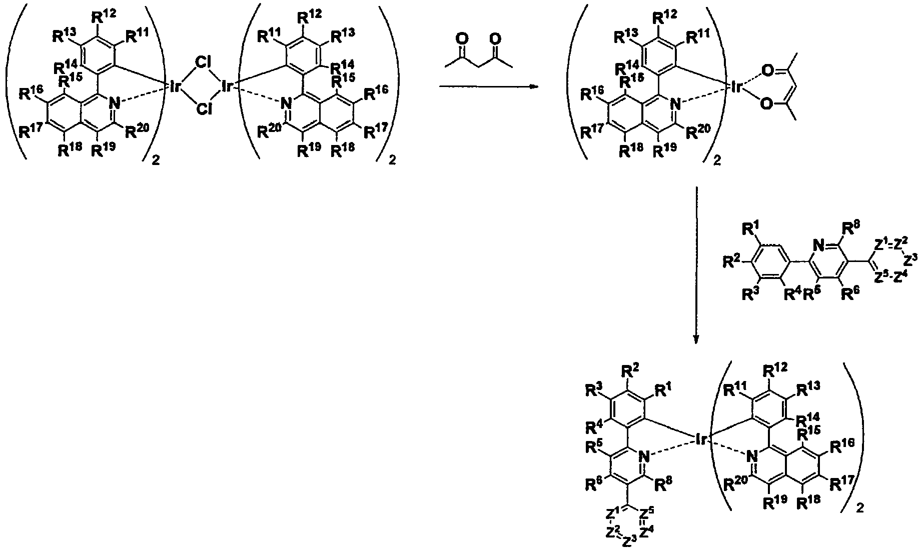 [wherein R¹ to R⁶, R⁸, R¹¹ to R²⁰ and Z¹ to Z⁵ have the same meaning as described above.]

Identification and analysis of thus produced metal complex of the present invention can be carried out by element analysis, NMR analysis and MS analysis.

### <Metal-containing polymer compound>

The metal-containing polymer compound of the present invention is a polymer compound containing a residue of the metal complex of the present invention (for example, the metal complex represented by the above-described formula (1)). In the present specification, "residue of metal complex" means an atomic group remaining after removal of k (k represents an integer of 1 or more) hydrogen atoms from a metal complex.

For example, the residue the metal complex represented by the above-described formula (1) means an atomic group remaining after removal of k hydrogen atoms from the metal complex represented by the above-described formula (1), and preferably is a mono- to tri-valent group.

The metal-containing polymer compound of the present invention may be a non-conjugated polymer compound or a conjugated polymer compound, and preferable are conjugated polymer compounds, more preferable are conjugated polymer compounds containing an aromatic ring in the main chain, since electric conductivity is high. The conjugated polymer compound as the metal-containing polymer compound of the present invention means a polymer compound in which 80 to 100%, preferably 85 to 100%, more preferably 90 to 100% of all bonds in the main chain of the polymer compound are conjugated.

The metal-containing polymer compound of the present invention has a polystyrene-equivalent number-average molecular weight of preferably 1×10³ to 1×10⁸, more preferably 1×10⁴ to 1×10⁷, since light emission efficiency and lifetime when used in a light emitting device are excellent in this range.

The metal-containing polymer compound of the present invention is preferably a polymer compound containing a residue of a metal complex represented by the above-described formula (1) and a divalent group represented by the following formula (7-1), (7-2), (7-3), (7-4) or (7-5): 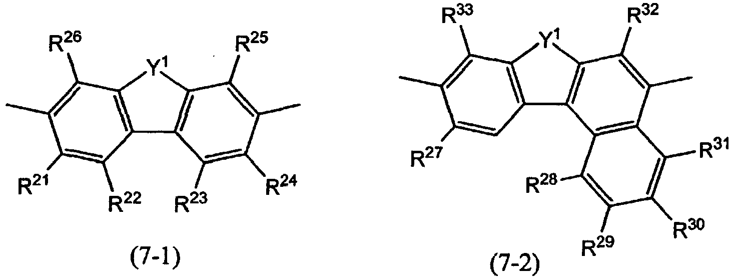 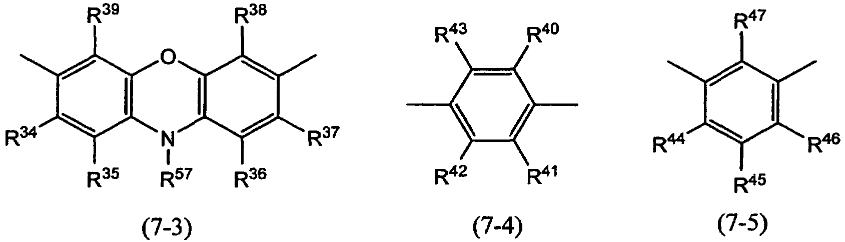
wherein Y¹ represents -C(R⁴⁸) (R⁴⁹)-, -O-C(R⁵⁰) (R⁵¹)-, -O-, -S-, -B(R⁵²)-, -Si(R⁵³) (R⁵⁴)-, -P(R⁵⁵)-, -P(R⁵⁶) ( = O)- or N(R⁵⁷)-;
R⁴⁸, R⁴⁹, R⁵⁰, R⁵¹, R⁵², R⁵³, R⁵⁴, R⁵⁵, R⁵⁶ and R⁵⁷ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group or a halogen atom, each of which may have a substituent;
R²¹, R²², R²³ R²⁴, R²⁵ R²⁶ , R²⁷, R²⁸ R²⁹, R³⁰, R³¹, R²¹, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶ and R⁴⁷ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group or a cyano group, each of which may have a substituent, more preferably a polymer compound containing a residue of a metal complex represented by the above-described formula (1) and a group represented by the above-described formula (7-1), since electric conductive is excellent.

In the metal-containing polymer compound of the present invention, the group represented by the above-described formulae (7-1) to (7-5) is preferably contained as a repeating unit, from the viewpoint of solubility and electric conductivity or easiness of synthesis of a polymer compound.

In the metal-containing polymer compound of the present invention, the residue of a metal complex represented by the formula (1) is preferably contained in a repeating unit, because of easiness of synthesis and enhanced light emission efficiency when used in a light emitting device. The repeating unit containing the residue of a metal complex represented by the formula (1) includes
an arylene group or a divalent heterocyclic group having as a substituent a residue (divalent) of a metal complex represented by the formula (1) or a residue (monovalent) of a metal complex represented by the formula (1), and the like. Examples of the arylene group or divalent heterocyclic group having as a substituent a residue (monovalent) of a metal complex include repeating units shown later as examples of a repeating unit represented by the formula: -(Ar')-.

In the metal-containing polymer compound of the present invention, the weight ratio of "a residue of a metal complex represented by the above-described formula (1)" to "a divalent group represented by the above-described formula (7-1), (7-2), (7-3), (7-4) or (7-5)" is preferably 1:2 to 1:1000, more preferably 1:3 to 1:400, since light emission efficiency, light emission color or electric conductivity is excellent in this range.

In the metal-containing polymer compound of the present invention, when a residue of a metal complex represented by the above-described formula (1) is contained in a repeating unit, the proportion of the repeating unit containing a residue of a metal complex represented by the above-described formula (1) is preferably 0.01 to 50 wt%, more preferably 0.1 to 30 wt%, further preferably 0.5 to 25 wt%, from the viewpoint of easiness of synthesis of a polymer compound or light emission efficiency when used in a light emitting device.

In the metal-containing polymer compound of the present invention, when the divalent group represented by the above-described formula (7-1), (7-2), (7-3), (7-4) or (7-5) is contained as a repeating unit, the proportion of the repeating unit is preferably 10 to 99.99 wt%, more preferably 30 to 99.99 wt%, further preferably 50 to 99.99 wt% with respect to the polymer compound, from the viewpoint of easiness of synthesis or electric conductivity.

In the metal-containing polymer compound of the present invention, the residue of a metal complex represented by the above-described formula (1) and the divalent group represented by the above-described formula (7-1), (7-2), (7-3), (7-4) or (7-5) may each be used singly or in combination.

The groups and atoms represented by R²¹ to R⁵⁷ in the formulae (7-1) to (7-5) have the same meaning as described above.

In the formulae (7-1) and (7-2), Y represents preferably -C(R⁴⁸) (R⁴⁹)-, -O-C(R⁵⁰) (R⁵¹)-, -O- or -S-, more preferably -C(R⁴⁸) (R⁴⁹)-, from the viewpoint of easiness of synthesis of a polymer compound or light emission efficiency when used in a light emitting device. Regarding R⁴⁸ and R⁴⁹, it is preferable that at least one of them is a phenyl group optionally having a substituent and it is more preferable that at least one them is a C₁ to C₁₂ alkoxyphenyl group or a C₁ to C₁₂ alkylphenyl group, because of excellent luminance lifetime of a light emitting device obtained when the metal-containing polymer compound of the present invention is used in the light emitting device.

The divalent group represented by the formula (7-1) includes groups represented by the following formulae, and the like. 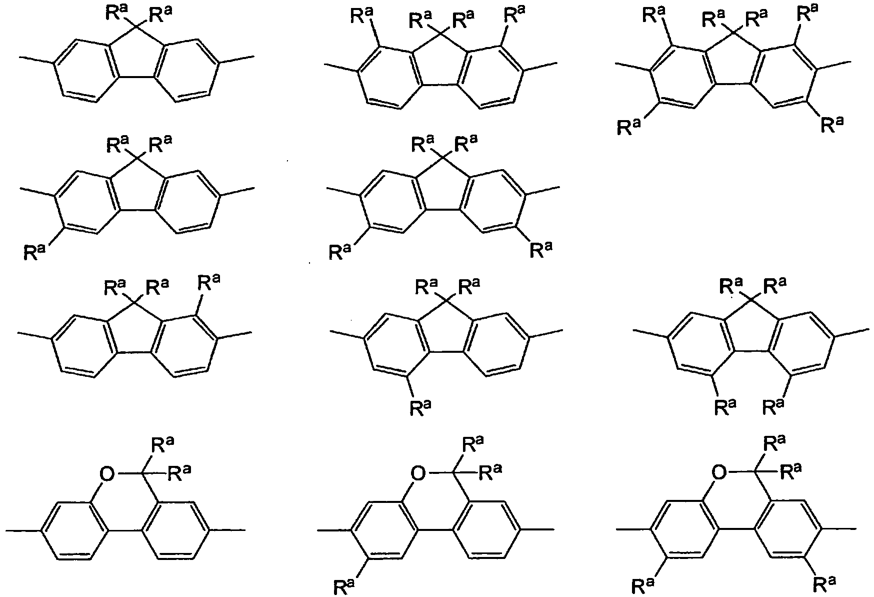 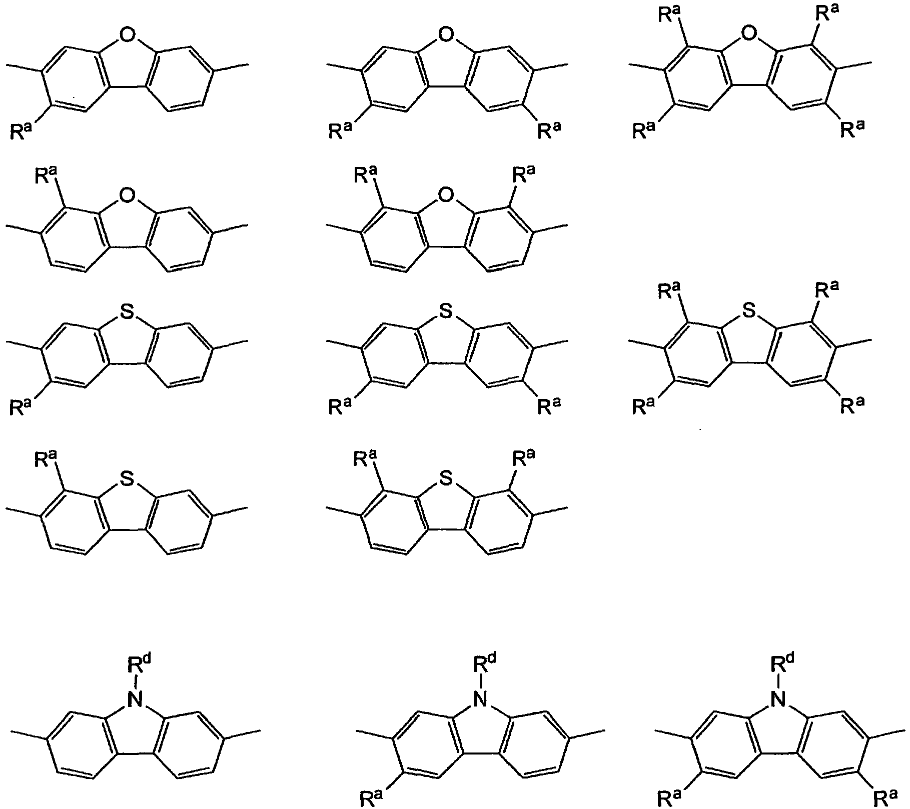 [wherein R^{a} represents an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group or a halogen atom. These groups may have a substituent. The plurality of R^{a} moieties may be the same or different.

R^{d} represents an alkyl group, an aryl group, an arylalkyl group, an arylalkenyl group, an arylalkynyl group or a monovalent heterocyclic group. These groups may have a substituent.]

The alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, silyloxy group, substituted silyloxy group, monovalent heterocyclic group or halogen atom represented by R^{a} has the same meaning as described above.

The alkyl group, aryl group, arylalkyl group, arylalkenyl group, arylalkynyl group or monovalent heterocyclic group represented by R^{d} has the same meaning as described above.

The divalent group represented by the formula (7-2) includes groups represented by the following formulae, and the like. 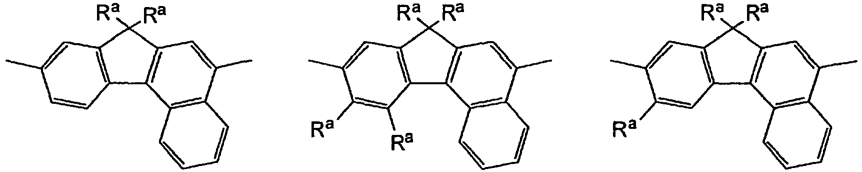 [wherein R^{a} has the same meaning as described above.]

The divalent group represented by the above-described formula (7-3) includes groups represented by the following formulae, and the like. 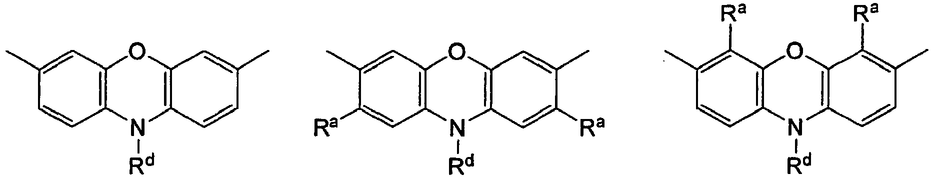 [wherein R^{a} and R^{d} have the same meaning as described above.]

The divalent group represented by the above-described formula (7-4) includes groups represented by the following formulae, and the like. 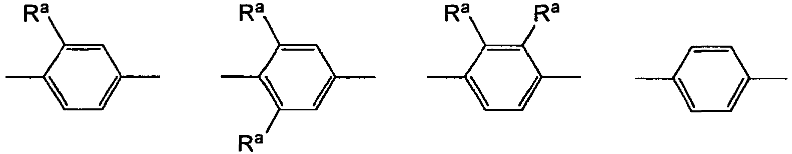 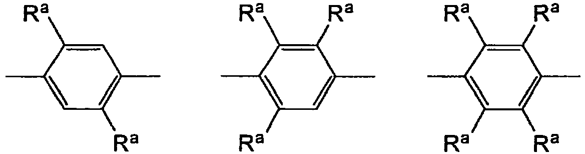 [wherein R^{a} has the same meaning as described above.]

The divalent group represented by the above-described formula (7-5) includes groups represented by the following formulae, and the like. 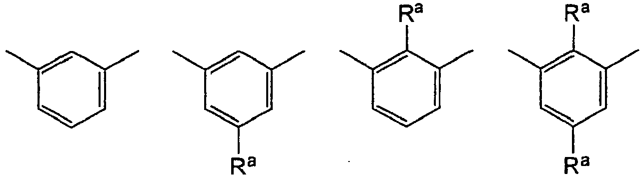 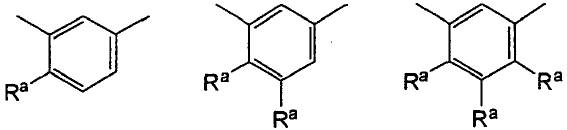 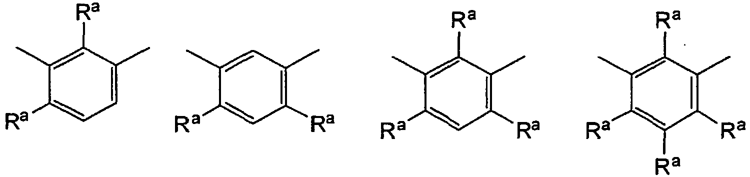 [wherein R^{a} has the same meaning as described above.]

The metal-containing polymer compound of the present invention may further contain a group represented by the following formula (8): 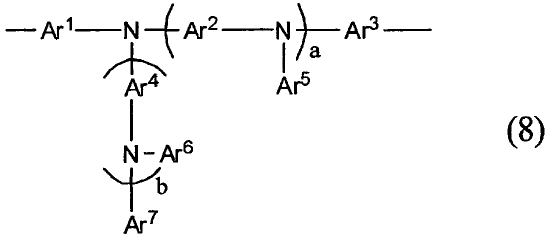 wherein Ar¹, Ar², Ar³ and Ar⁴ each independently represent an arylene group or a divalent heterocyclic group, and Ar⁵, Ar⁶ and Ar⁷ each independently represent an aryl group or a monovalent heterocyclic group, wherein Ar¹, Ar², Ar³, Ar⁴, Ar⁵, Ar⁶ and Ar⁷ may have a substituent; a and b each independently represent 0 or 1, and 0 ≤ a + b ≤ 1, since hole transport properties is excellent in this case. The group represented by the above-described formula (8) is preferably contained as a repeating unit in the metal-containing polymer compound.

In the above-described formula (8), the arylene group represented by Ar¹, Ar², Ar³ and Ar⁴ is an atomic group obtained by removing two hydrogen atoms from an aromatic hydrocarbon. The arylene group includes also groups having a condensed ring, and groups containing two or more independent benzene rings or condensed rings linked directly or via a vinylene group or the like. The arylene group may have a substituent. The carbon atom number of a portion obtained by removing the substituent from the arylene group is usually 6 to 60, preferably 6 to 20. The total carbon atom number of the arylene group including the substituent is usually 6 to 100. The arylene group includes a phenylene group, a biphenyl-4,4'-diyl group, a 1,1' :4',1"-terphenyl-4,4"-diyl group, a naphthalenediyl group, an anthracenediyl group, a phenanthrenediyl group, a pentalenediyl group, an indenediyl group, a heptalenediyl group, an indacenediyl group, a triphenylenediyl group, a binaphthyldiyl group, a phenylnaphthalenediyl group, a stilbenediyl group, a fluorenediyl group and the like.

In the above-described formula (8), the divalent heterocyclic group represented by Ar¹, Ar², Ar³ and Ar⁴ means an atomic group remaining after removal of two hydrogen atoms from a heterocyclic compound. The divalent heterocyclic group has a carbon atom number of usually 2 to 30, preferably 2 to 15. The carbon atom number of the divalent heterocyclic group does not include the carbon atom number of the substituent. The divalent heterocyclic group is preferably a divalent aromatic heterocyclic group. The divalent heterocyclic group includes a pyridinediyl group, a diazaphenylene group, a quinolinediyl group, a quinoxalinediyl group, an acridinediyl group, a bipyridyldiyl group, a phenanthrolinediyl group and the like.

In the formula (8), the aryl group and monovalent heterocyclic group represented by Ar⁵, Ar⁶ and Ar⁷ have the same meaning as described above.

In the formula (8), the substituent which the arylene group, divalent heterocyclic group, aryl group and monovalent heterocyclic group may have includes an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group, a cyano group, a nitro group and the like. These atoms and groups have the same meaning as described above.

The group represented by the formula (8) includes groups represented by the following formulae, and the like. 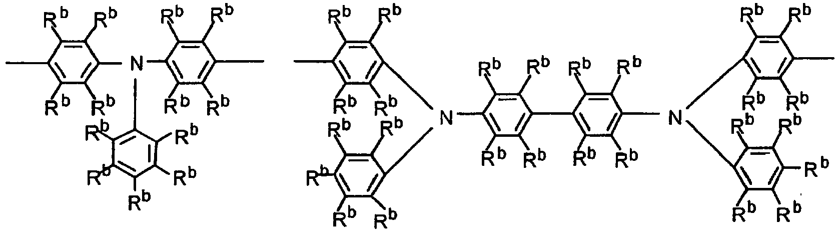 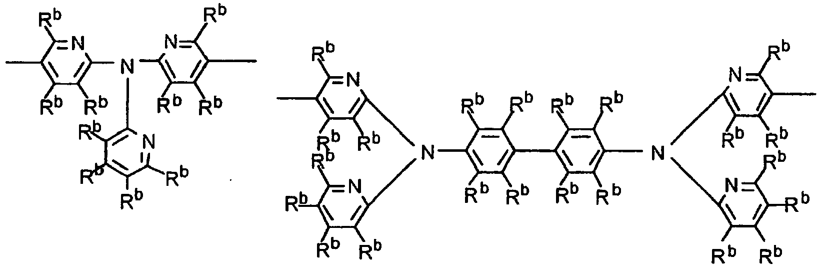 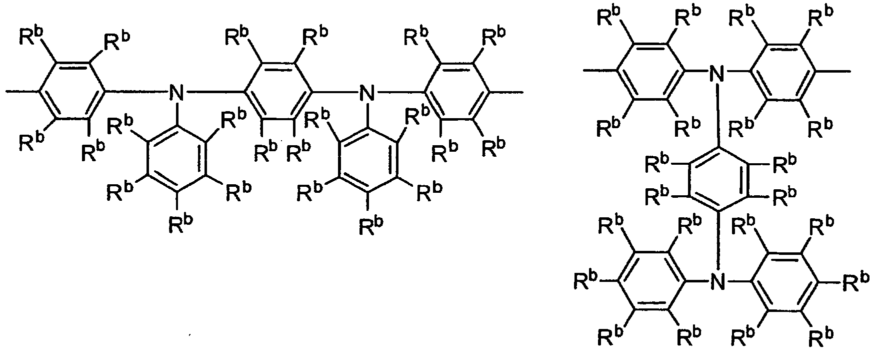 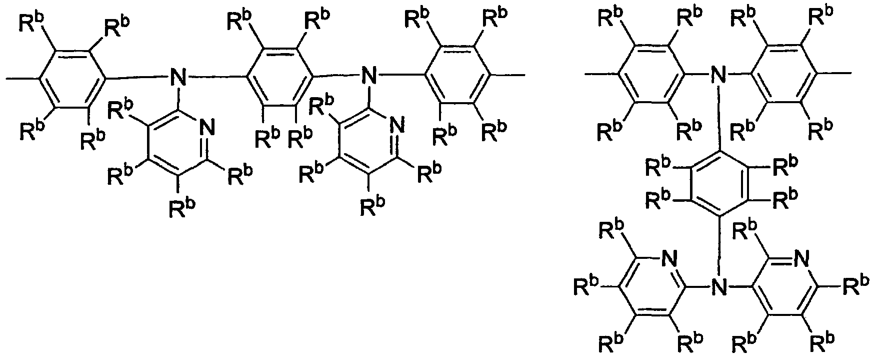 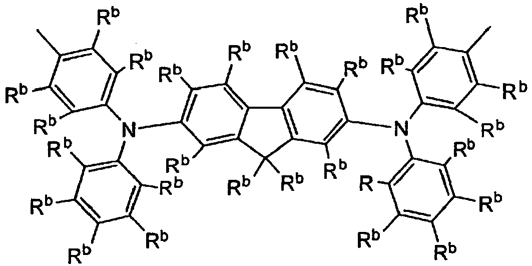 [wherein R^{b} represents a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group or a cyano group. A hydrogen atom contained in these groups may be substituted by a fluorine atom. These groups may have a substituent. The plurality of R^{b} moieties may be the same or different.]

### -Position of residue of metal complex-

The metal-containing polymer compound of the present invention includes
1. a polymer compound having a residue of a metal complex in the main chain of the molecular chain;
2. a polymer compound having a residue of a metal complex on the end of the molecular chain; and
3. a polymer compound having a residue of a metal complex in the side chain of the molecular chain.

The polymer compound having a residue of a metal complex in the main chain of the molecular chain is represented, for example, by any of the following formulae. 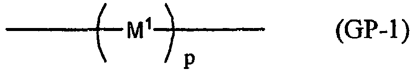 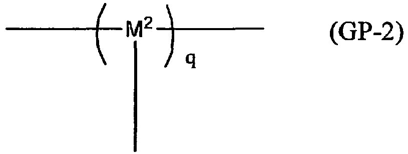 [wherein M¹ and M² each independently represent a residue of a metal complex. p and q each independently represent the degree of polymerization.

In the formula (GP-1) and formula (GP-2), a solid line represents a molecular chain.]

In the above-described formula (GP-1), when p≥2, inter-residual parts of a metal complex represented by adjacent two M¹s (hereinafter, referred to as "M¹ inter-residual parts") are linked directly or linked via a molecular chain represented by a solid line. In the above-described formula (GP-1), when p≥3, two or more M¹ inter-residual parts bonds may be the same or different.

In the above-described formula (GP-2), when q≥2, inter-residual parts of a metal complex represented by adjacent two M²s (hereinafter, referred to as "M² inter-residual parts") are linked directly or linked via a molecular chain represented by a solid line. In the above-described formula (GP-2), when q≥3, two or more M² inter-residual parts bonds may be the same or different.

M¹ includes (divalent) residues of metal complexes represented by the following formulae. 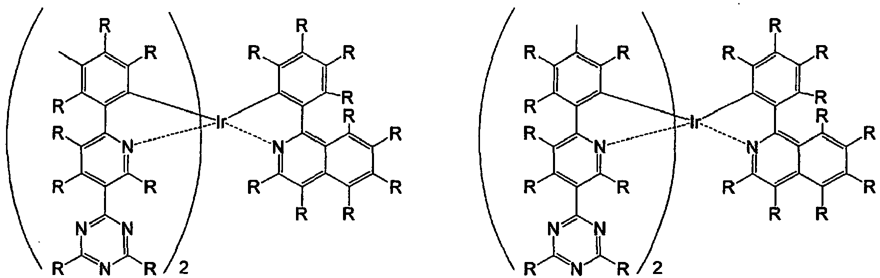 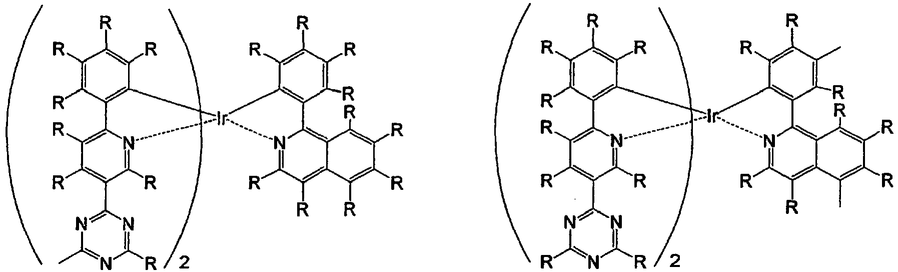 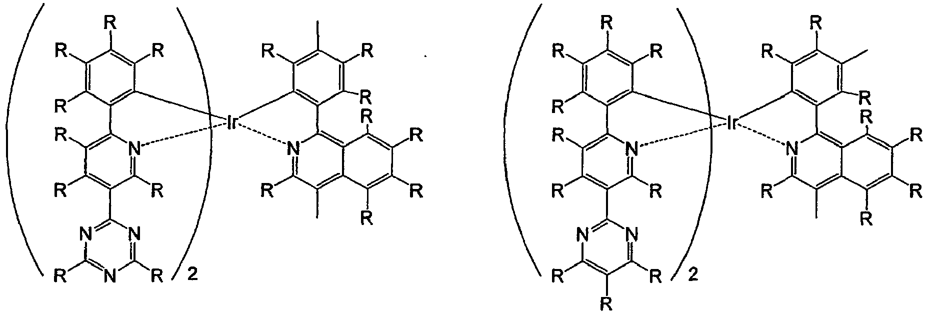 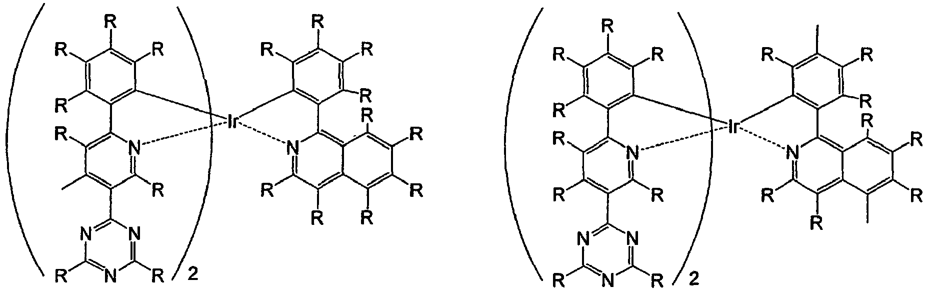 [wherein R has the same meaning as described above.]

The above-described M² includes (trivalent) residues of metal complexes represented by the following formulae. 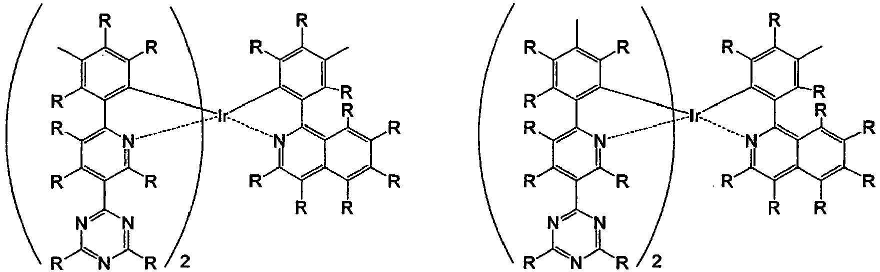 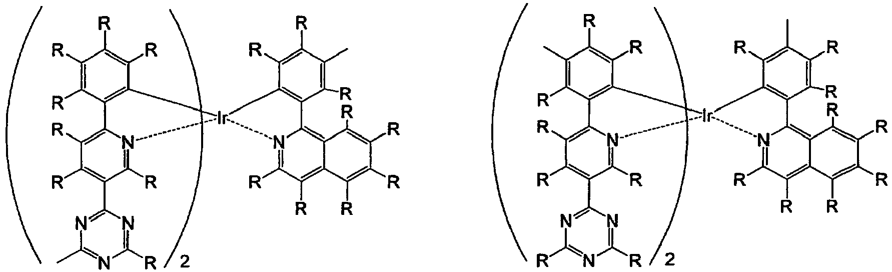 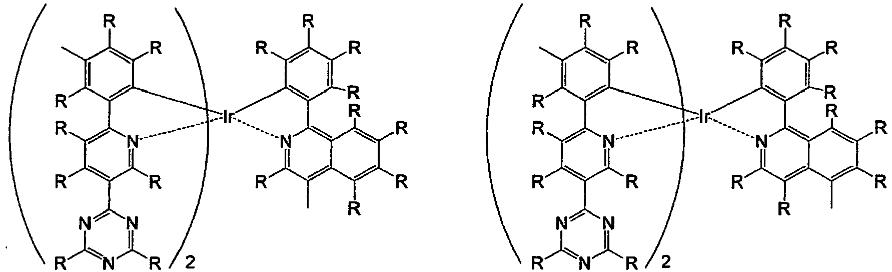 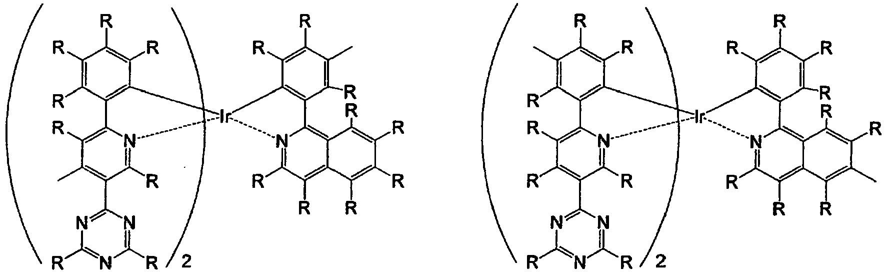 [wherein R has the same meaning as described above.]

The polymer compound having a residue of a metal complex on the end of the molecular chain is represented, for example, by the following formulae. 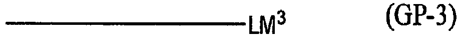 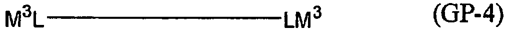 [wherein M³ represents a residue of a metal complex. In the formula (GP-3) and formula (GP-4), a solid line represents a molecular chain. L represents a single bond, -O-, -S-, -C( = O)-, -C( = O)O-, -S( = O)-, -S( = O)₂ -, -Si(R⁶⁸) (R⁶⁹)-, N(R⁷⁰)-, -B(R⁷¹)-, -P(R⁷²)-, -P( = O) (R⁷³)-, an optionally substituted alkylene group, an optionally substituted alkenylene group, an optionally substituted alkynylene group, an optionally substituted arylene group or an optionally substituted divalent heterocyclic group. R⁶⁸, R⁶⁹, R⁷⁰, R⁷¹, R⁷² and R⁷³ each independently represent a hydrogen atom, an alkyl group, an aryl group, a monovalent heterocyclic group or a cyano group.]

M³ includes (monovalent) residues of metal complexes represented by the following formulae. 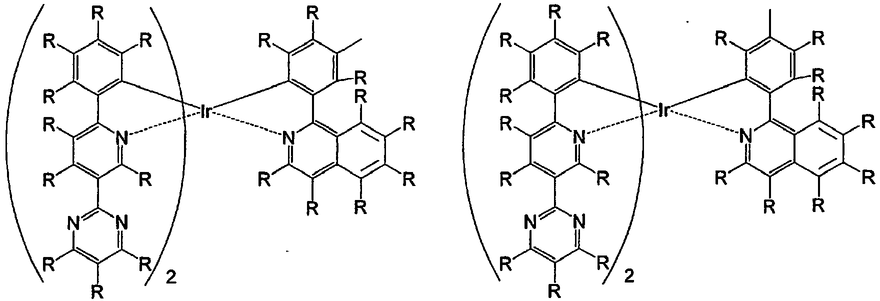 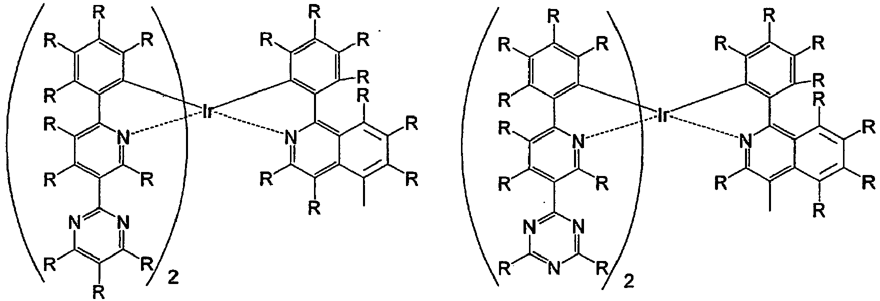 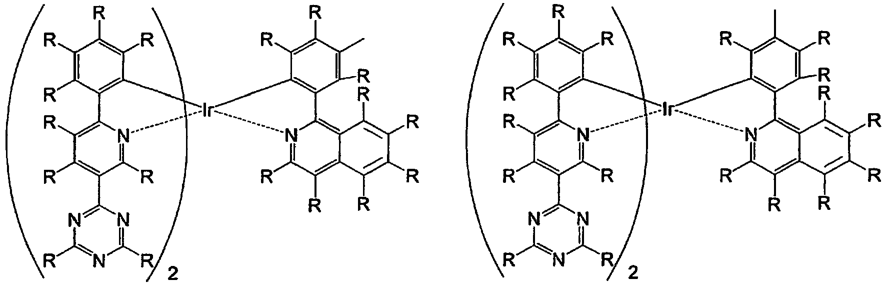 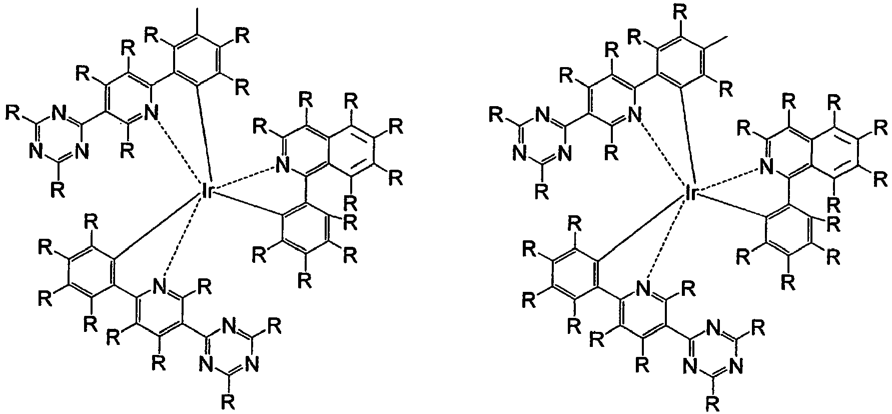 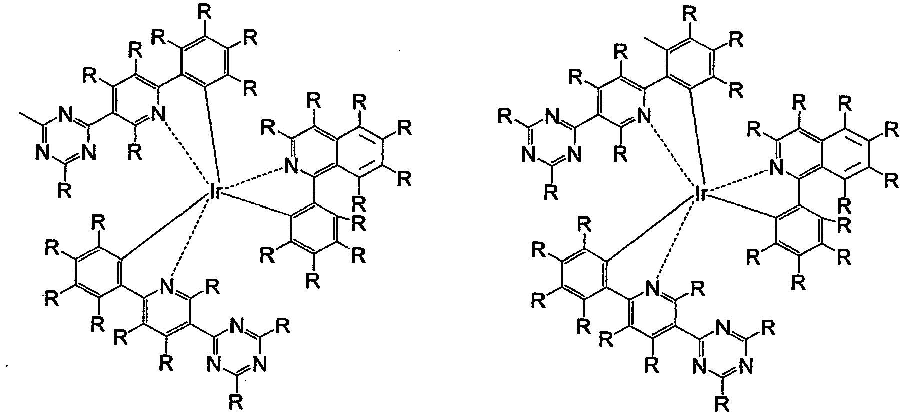 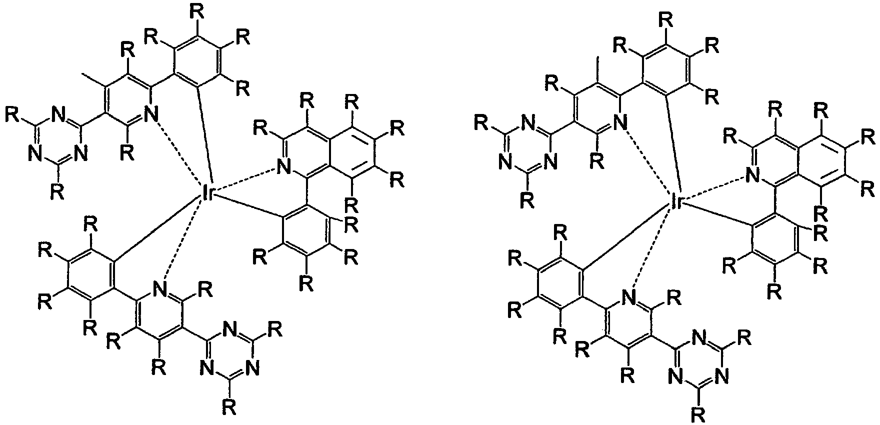 [wherein R has the same meaning as described above.]

The polymer compound having a residue of a metal complex in the side chain of the molecular chain includes polymer compounds in which the residue of a metal complex is a monovalent group.

The molecular chain has, for example, a repeating unit represented by the formula: -(Ar')-. In the formula, Ar' represents a divalent aromatic group having 1 to 4 groups represented by -L-M³, or a divalent heterocyclic group having an atom selected from the group consisting of an oxygen atom, a silicon atom, a phosphorus atom, a boron atom and a sulfur atom and 1 to 4 groups represented by -L-M³. L and M³ have the same meaning as described above. When the alkylene group, alkenylene group and alkynylene group represented by L contain -CH₂- groups, one or more -CH₂- groups contained in the alkylene group, one or more -CH₂- groups contained in the alkenylene group and one or more -CH₂- groups contained in the alkynylene group may be substituted by a group represented by -O-, -S-, -C( = O)-, -C( = O) O-, -S( = O)-, -S( = O)₂-, -Si(R⁷⁴) (R⁷⁵)-, N(R⁷⁶)-, -B(R⁷⁷)-, -P(R⁷⁸)- or P( = O) (R⁷⁹)-. R⁷⁴, R⁷⁵, R⁷⁶, R⁷⁷, R⁷⁸ and R⁷⁹ each independently represent a hydrogen atom, an alkyl group, an aryl group, a monovalent heterocyclic group or a cyano group. Ar' may have a substituent selected from the group consisting of an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group and a cyano group, in addition to the group represented by -L-M₃. When Ar' has a plurality of substituents, these may be the same or different.

In the formula, the alkyl group, aryl group, monovalent heterocyclic group and cyano group represented by R⁶⁸, R⁶⁹, R⁷⁰, R⁷¹, R⁷², R⁷³, R⁷⁴, R⁷⁵, R⁷⁶, R⁷⁷, R⁷⁸ and R⁷⁹, and the alkyl group, alkoxy group, alkylthio group, aryl group, aryloxy group, arylthio group, arylalkyl group, arylalkoxy group, arylalkylthio group, arylalkenyl group, arylalkynyl group, amino group, substituted amino group, silyl group, substituted silyl group, halogen atom, acyl group, acyloxy group, imine residue, amide group, acid imide group, monovalent heterocyclic group, carboxyl group, substituted carboxyl group and cyano group which are substituents optionally carried on Ar' have the same meaning as described above.

Ar' includes groups represented by the following formulae, and the like. 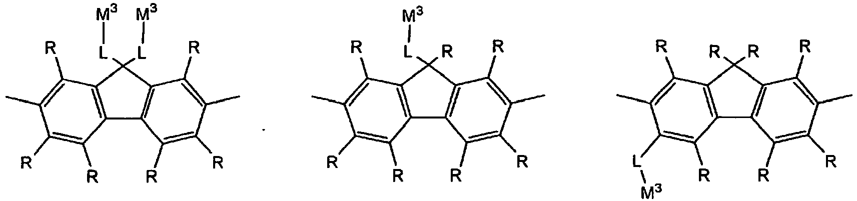 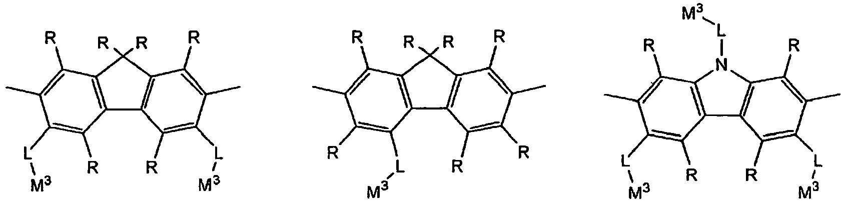 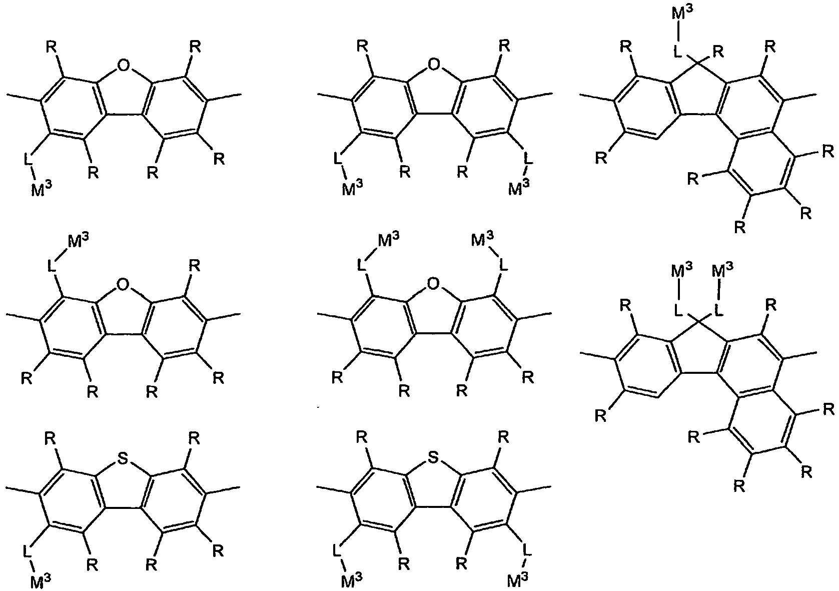 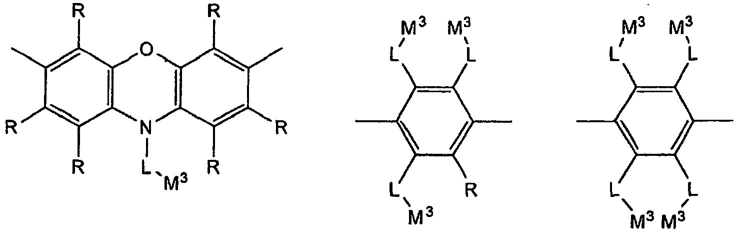 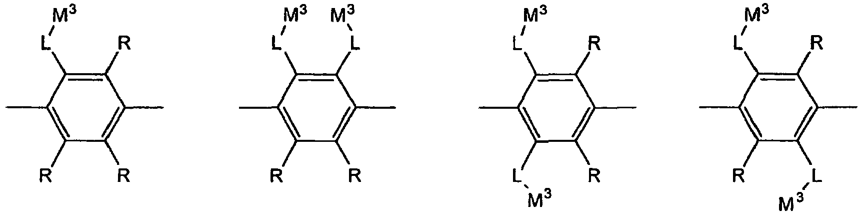 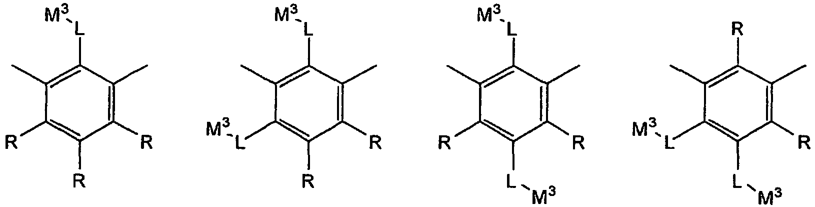 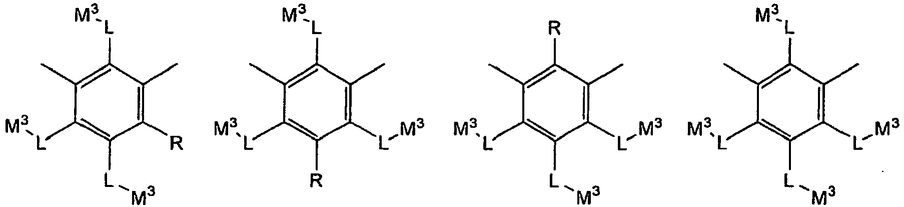 [wherein L, R and M³ have the same meaning as described above.]

The alkylene group represented by L has a carbon atom number of usually 1 to 30, preferably 1 to 15. The alkylene group includes a methylene group, an ethylene group, a propylene group, a tetramethylene group, a pentamethylene group, a hexamethylene group, an octamethylene group, a 1,3-cyclopentylene group, a 1,4-cyclohexylene group and the like.

The alkenylene group represented by L has a carbon atom number of usually 2 to 30, preferably 2 to 15. The alkenylene group includes a vinylene group, a propenediyl group and the like. The alkenylene group includes also alkadienylene groups such as a 1,3-butadienediyl group.

The alkynylene group represented by L has a carbon atom number of usually 2 to 30, preferably 2 to 15. The alkynylene group includes an ethynylene group and the like. The alkynylene group includes also groups having two triple bonds, and a 1,3-butadiyne-1,4-diyl group and the like are listed.

The arylene group and divalent heterocyclic group represented by L have the same meaning as described above.

L includes preferably a single bond, -O-, a methylene group, an ethylene group, a hexamethylene group, an octamethylene group, a vinylene group, a phenylene group, a biphenyl-4,4'-diyl group and a pyridinediyl group, more preferably a phenylene group and a pyridinediyl group, from the viewpoint of easiness of synthesis of a polymer compound, light emission properties.

The metal-containing polymer compound of the present invention is preferably a metal-containing polymer compound causing no significant deterioration of charge transport properties, charge injection properties and the like, and more preferably is a conjugated metal-containing polymer compound because of excellent charge transport properties.

When the metal-containing polymer compound of the present invention has a repeating unit containing a residue of a metal complex represented by the above-described formula (1) and a repeating unit containing a divalent group represented by the above-described formulae (7-1) to (7-5), the metal-containing polymer compound of the present invention may contain other repeating units in a range not deteriorating light emission properties and charge transport properties. In this case, the total amount of the repeating unit containing a residue of a metal complex represented by the above-described formula (1) and the repeating unit containing a divalent group represented by the above-described formulae (7-1) to (7-5) is preferably 10 mol% or more, more preferably 50 mol% or more, further preferably 80 mol% or more, based on all repeating units.

### -Production method of metal-containing polymer compound-

The metal-containing polymer compound of the present invention can be produced, for example, by a production method including a step of reacting a metal complex represented by the following formula (9) and a compound represented by the following formula (12-1), (12-2), (12-3), (12-4) or (12-5). 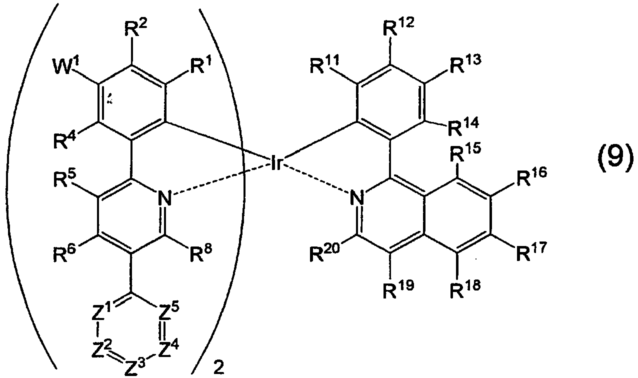 [wherein, W¹ represents a polymerization reactive group. R¹, R², R⁴, R⁵, R⁶, R⁸ R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above.] 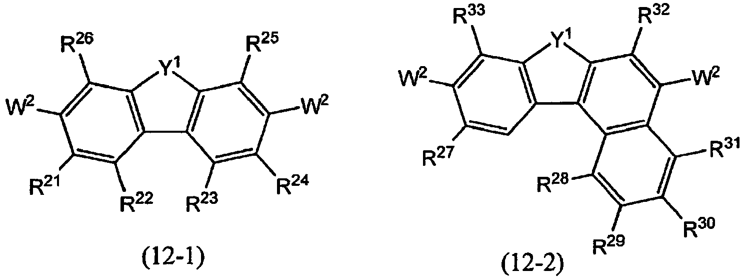 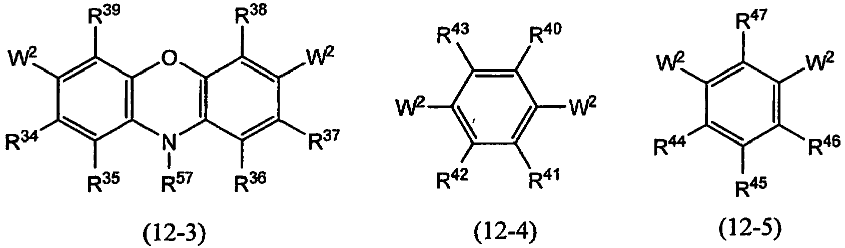 [wherein, W² represents a polymerization reactive group. The plurality of W² moieties may be the same or different. Y¹, R²¹, R²², R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶ and R⁴⁷ have the same meaning as described above.]

In the above-described formula (9), (12-1), (12-2), (12-3), (12-4) or (12-5), the polymerization reactive group represented by W¹ and W² includes a halogen atom, an alkylsulfonyloxy group, an arylsulfonyloxy group, an arylalkylsulfonyloxy group, a boric acid ester residue, a sulfonium methyl group, a phosphonium methyl group, a phosphonate methyl group, a monohalogenated methyl group, -MgX (X represents a halogen atom), a stannyl group, -B(OH)₂, a formyl group, a cyano group and the like, and preferable are -B(OH)₂, a boric acid ester residue, -MgX, a stannyl group and a halogen atom.

The halogen atom as the polymerization reactive group includes a fluorine atom, a chlorine atom, a bromine atom and an iodine atom, preferably a chlorine atom, a bromine atom and an iodine atom, more preferably a bromine atom.

The alkylsulfonyloxy group as the polymerization reactive group includes a methylsulfonyloxy group, an ethylsulfonyloxy group, a trifluoromethylsulfonyloxy group and the like.

The arylsulfonyloxy group as the polymerization reactive group includes a phenylsulfonyloxy group, a p-tolylsulfonyloxy group and the like.

The arylalkylsulfonyloxy group as the polymerization reactive group includes a benzylsulfonyloxy group and the like.

The boric acid ester residue as the polymerization reactive group includes a dialkyl ester residue, a diaryl ester residue, a diaryl alkyl ester residue and the like, and preferable are groups represented by the following formulae. 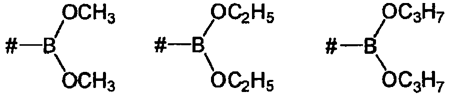 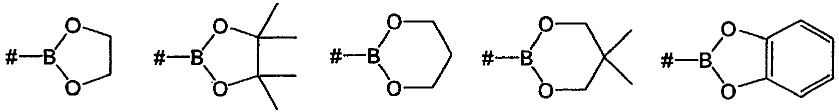 [wherein, # represents a connecting bond.]

The sulfonium methyl group includes groups represented by the following formulae.

-CH₂ S⁺ Me₂X⁻, -CH₂ S⁺ Ph₂ X⁻

[wherein, X represents a halogen atom and Ph represents a phenyl group.].

The phosphonium methyl group includes groups represented by the following formula.

-CH₂ P⁺ Ph₃ X⁻

[wherein, X represents a halogen atom.]

The phosphonate methyl group includes groups represented by the following formula.

-CH₂PO(OR^{c})₂

[wherein, X represents a halogen atom. R^{c} represents an alkyl group, an aryl group or an arylalkyl group.]

The monohalogenated methyl group includes a monofluoromethyl group, a monochloromethyl group, a monobromomethyl group and a monoiodomethyl group.

-MgX includes -MgCl, -MgBr and -MgI.

The stannyl group includes optionally substituted stannyl groups, and includes a stannyl group, a trichlorostannyl group, a trimethylstannyl group, a triethylstannyl group, a tri-n-butylstannyl group, a triphenylstannyl group and a tribenzylstannyl group.

The polymerization reactive group includes preferably a halogen atom, an alkylsulfonyloxy group, an arylsulfonyloxy group and an arylalkylsulfonyloxy group in a reaction using a zerovalent nickel complex or the like such as in the Yamamoto coupling reaction and the like, and includes preferably an alkylsulfonyloxy group, a halogen atom, a boric acid ester residue and -B(OH)₂ in a reaction using a nickel catalyst or a palladium catalyst such as in the Suzuki coupling reaction and the like.

Production of the metal-containing polymer compound of the present invention can be carried out, for example, by dissolving a raw material compound having several polymerization reactive groups in an organic solvent as required, and using an alkali or a suitable solvent at a temperature of the melting point or higher and the boiling point of lower of the organic solvent. For producing the metal-containing polymer compound, known methods described in "Organic Reactions", vol. 14, pp. 270 to 490, John Wiley & Sons, Inc., 1965, "Organic Syntheses", Collective Volume VI, pp. 407-411, John Wiley & Sons, Inc., 1988, Chem. Rev., vol. 95, p. 2457 (1995), Journal of Organometallic Chemistry (J. Organomet. Chem.), vol. 576, p. 147 (1999), Makromolecular Chemistry, Macromolecular Symposium (Makromol. Chem., Macromol. Symp.), vol. 12, p. 229 (1987), and the like can be used.

In the method of producing the metal-containing polymer compound of the present invention, known condensation reactions can be used depending on a polymerization reactive group of a compound represented by the formula (9) and a polymerization reactive group of a compound represented by the above-described formulae (12-1) to (12-5). By carrying out polymerization under coexistence of a compound having two or more polymerization reactive groups, a metal-containing polymer compound which is a copolymer can be produced, and by copolymerizing a compound having three or more polymerization reactive groups, a metal-containing polymer compound having a branched structure can be produced.

When the condensation reaction is a reaction of generating a double bond, the method includes methods described in JP-A No. 5-202355, that is, methods of polymerization according to the Wittig reaction of a compound having a formyl group with a compound having a phosphonium methyl group, or a compound having a formyl group and a phosphonium methyl group, polymerization according to the Heck reaction of a compound having a vinyl group with a compound having a halogen atom, polycondensation according to the dehydrohalogenation method of a compound having two or more monohalogenated methyl groups, polycondensation according to the sulfonium salt decomposition method of a compound having two or more sulfonium methyl groups, polymerization according to the Knoevenagel reaction of a compound having a formyl group with a compound having a cyano group, and the like, and methods of polymerization according to the McMurry reaction of a compound having two or more formyl groups, and the like.

When the condensation reaction is a reaction of generating a triple bond, the reaction includes the Heck reaction and the Sonogashira reaction.

When the condensation reaction is a reaction of not generating a double bond or a triple bond, the reaction includes a method of polymerization according to the Suzuki coupling reaction, a method of polymerization according to the Grignard reaction, a method of polymerization with a Ni(0) complex, a method of polymerization with an oxidizing agent such as FeCl₃ and the like, a method of electrochemical oxidation polymerization, a method according to decomposition of an intermediate polymer having a suitable leaving group, and the like, from the correspondent raw material compounds.

Of them, methods of polymerization according to the Wittig reaction, polymerization according to the Heck reaction, polymerization according to the Knoevenagel reaction and polymerization according to the Suzuki coupling reaction, a method of polymerization according to the Grignard reaction and a method of polymerization with a nickel zerovalent complex are preferable since control of molecular weight is easy and control of composition ratio in copolymerization is easy.

In the method of producing the metal-containing polymer compound of the present invention, it is preferable that the polymerization reactive groups W¹ and W² represent -B(OH)₂, a boric acid ester residue or a halogen atom, the ratio of the total mol number (J) of a halogen atom to the total mol number (K) of -B(OH)₂ and a boric acid ester residue, in all raw material compounds, is substantially 1 (usually, K/J is 0.7 to 1.2) and condensation polymerization is carried out using a nickel catalyst or a palladium catalyst.

In these production methods, the raw material compounds include a combination of a dihalogenated compound with a diboric acid compound or a diborate compound; a halogen-boric acid compound, a halogen-borate compound, and the like.

In the method of producing the metal-containing polymer compound of the present invention, it is preferable to progress the reaction under an inert atmosphere using an organic solvent which has been subjected to a sufficient deoxidation treatment before use, for suppressing side reactions. If necessary, a dehydration treatment may be carried out. However, this is not the case in a reaction in a two-phase system with water such as in the Suzuki coupling reaction.

The organic solvent which may be used in the method of producing the metal-containing polymer compound includes saturated hydrocarbons such as pentane, hexane, heptane, octane, cyclohexane and the like, unsaturated hydrocarbons such as benzene, toluene, ethylbenzene, xylene and the like, halogenated saturated hydrocarbons such as carbon tetrachloride, chloroform, dichloromethane, chlorobutane, bromobutane, chloropentane, bromopentane, chlorohexane, bromohexane, chlorocyclohexane, bromocyclohexane and the like, halogenated unsaturated hydrocarbons such as chlorobenzene, dichlorobenzene, trichlorobenzene and the like, alcohols such as methanol, ethanol, propanol, isopropanol, butanol, t-butylalcohol and the like, carboxylic acids such as formic acid, acetic acid, propionic acid and the like, ethers such as dimethyl ether, diethyl ether, methyl t-butyl ether, tetrahydrofuran, tetrahydropyran, dioxane and the like, amines such as trimethylamine, triethylamine, N,N,N',N'-tetramethylethylenediamine, pyridine and the like, amides such as N,N-dimethylformamide, N,N-dimethylacetamide, N,N-diethylacetamide, N-methylmorpholine oxide and the like, etc. These organic solvents may be used singly or in combination.

In the method of producing the metal-containing polymer compound of the present invention, an alkali and a catalyst may be added for promoting the reaction. As the alkali and catalyst, those which are dissolved sufficiently in the solvent to be used in the reaction are preferable. As the method of mixing the alkali and catalyst, there are methods in which a solution of an alkali or a catalyst is added slowly while stirring the reaction solution under an inert atmosphere such as argon, nitrogen and the like, or the reaction solution is added slowly to a solution of an alkali or a catalyst.

The compound represented by the formula (9) can be produced, for example, from a compound represented by the above-described formula (1c). First, a compound represented by the above-described formula (1c) is dissolved in a solvent, and N-bromosuccinimide is added and they are reacted, to generate a compound represented by the following formula (9a). In this reaction, halogen-based hydrocarbon solvents such as methylene chloride and the like can be used. 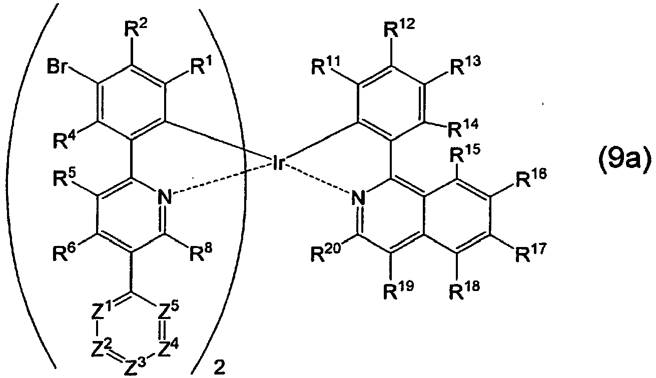 [wherein R¹, R², R⁴ R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above.]

### -Non-metal-containing polymer compound-

The first composition of the present invention may contain a non-metal-containing polymer compound, since when used in fabrication of a light emitting device, the fabrication cost of the device can be reduced. When the first composition of the present invention contains a non-metal-containing polymer compound, the proportion of the non-metal-containing polymer compound is preferably 180 to 20000 parts by weight, more preferably 400 to 3300 parts by weight, with respect to 100 parts by weight a metal complex represented by the above-described formula (1).

The non-metal-containing polymer compound may be a non-conjugated non-metal-containing polymer compound or a conjugated non-metal-containing polymer compound, and because of excellent electric conductivity, preferable are conjugated non-metal-containing polymer compounds, more preferable are conjugated non-metal-containing polymer compounds containing an aromatic ring in the main chain.

The non-metal-containing polymer compound has a polystyrene-equivalent number-average molecular weight of preferably 1×10³ to 1×10⁸, more preferably 1×10⁴ to 1×10⁷, since device properties such as light emission efficiency, lifetime and the like when used in a light emitting device are excellent.

The non-metal-containing polymer compound preferably has at least one member selected from the group consisting of a divalent group represented by the above-described formula (7-1), a divalent group represented by the above-described formula (7-2), a divalent group represented by the above-described formula (7-3), a divalent group represented by the above-described formula (7-4), a divalent group represented by the above-described formula (7-5) and a divalent group represented by the above-described formula (8) as a repeating unit, more preferably has a divalent group represented by the above-described formula (7-1) as a repeating unit, since electric conductivity is excellent in this case.

The non-metal-containing polymer compound preferably has further a repeating unit represented by the following formula (10), since electron injectability is excellent when fabricated into a light emitting device. 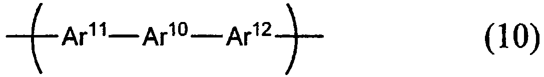 [wherein Ar¹⁰ represents a divalent nitrogen-containing aromatic heterocyclic group. Ar¹¹ and Ar¹² each independently represent an arylene group or a divalent heterocyclic group.]

In the formula (10), the arylene group and divalent heterocyclic group represented by Ar¹¹ and Ar¹² have the same meaning as described above.

In the formula (10), the divalent nitrogen-containing aromatic heterocyclic group represented by Ar¹⁰ means an atomic group remaining after removal of two hydrogen atoms from a nitrogen-containing aromatic heterocyclic compound, corresponding to a divalent group represented by the above-described formula (7-1) or the above-described formula (7-2) in which Y¹ is -N(R⁵⁷)-, and the atomic group not corresponding to a divalent group represented by the above-described formula (7-3). The above-described divalent nitrogen-containing aromatic heterocyclic group has a carbon atom number of usually 2 to 30, preferably 2 to 15. The divalent nitrogen-containing aromatic heterocyclic group does not include the carbon atom number of the substituent.

In the formula (10), the substituent which the divalent nitrogen-containing aromatic heterocyclic group may have includes an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group, a halogen atom and the like. These atoms and groups have the same meaning as described above.

The divalent nitrogen-containing aromatic heterocyclic group includes groups represented by the following formulae, and the like. 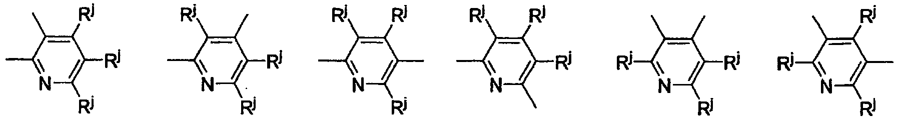 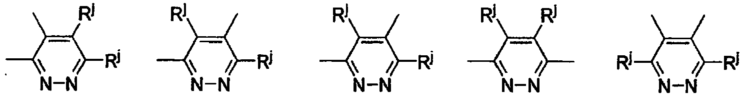 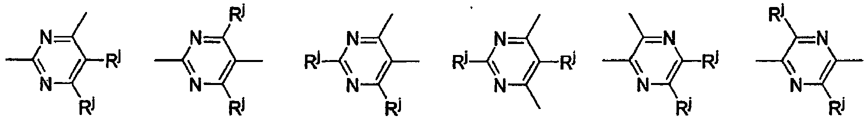 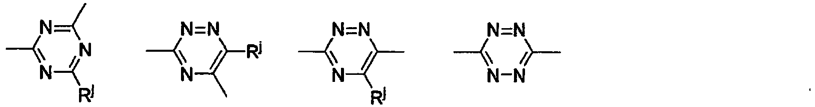 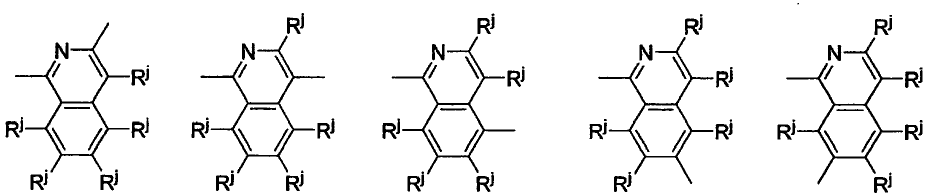 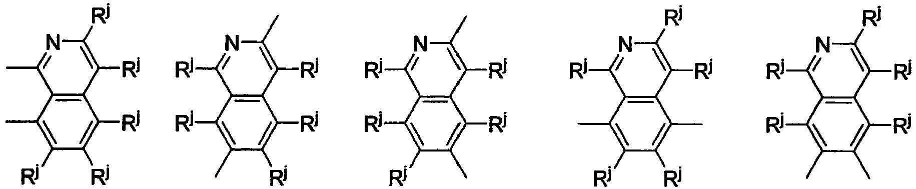 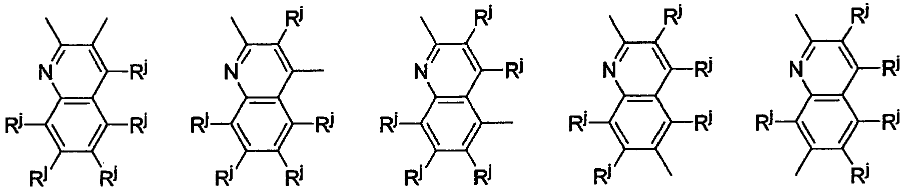 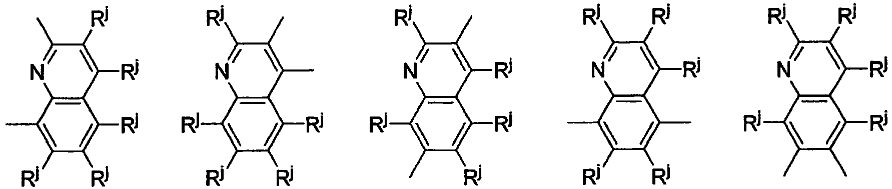 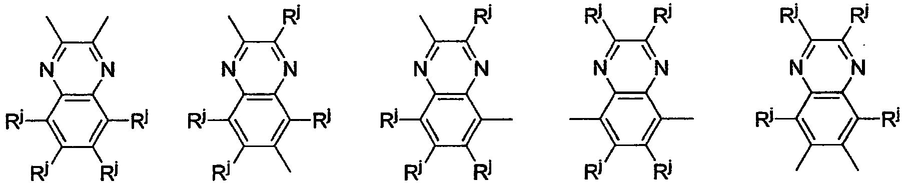 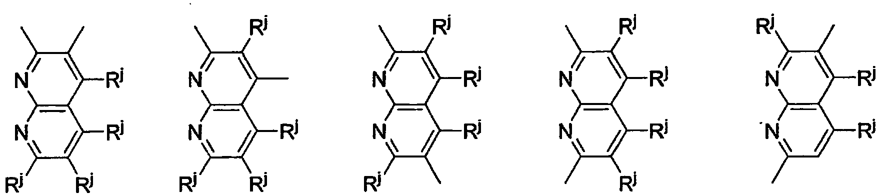 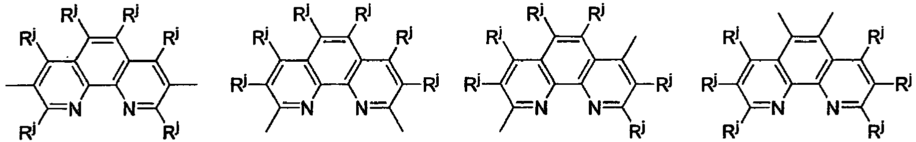 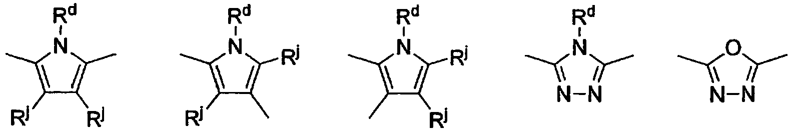 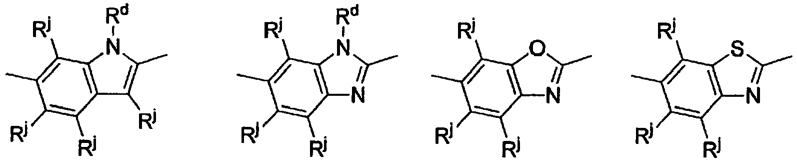 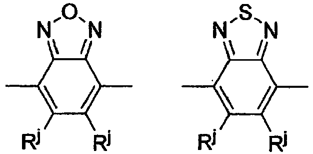 [wherein R^{d} has the same meaning as described above. R^{j} represents a hydrogen atom, an alkyl group, an alkoxy group, an aryl group, an aryloxy group, an arylalkyl group, an arylalkoxy group, an arylalkenyl group, an arylalkynyl group, an amino group, a substituted amino group, a monovalent heterocyclic group or a halogen atom, each of which may have a substituent. The plurality of R^{j} moieties may be the same or different.]

In the non-metal-containing polymer compound, the repeating unit represented by the above-described formula (10) is preferably a repeating unit represented by the following formula (11): 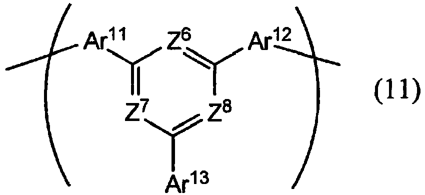
wherein Ar¹¹ and Ar¹² have the same meaning as described above and Ar¹³ represents an aryl group or a monovalent heterocyclic group, wherein Ar¹¹, Ar¹² and Ar¹³ may have a substituent;
Z⁶, Z⁷ and Z⁸ each independently represent -C(R^{k})= or a nitrogen atom and R^{k} represents a hydrogen atom or a substituent, provided that at least two of Z⁶, Z⁷ and Z⁸ are nitrogen atoms.

In the formula (11), the aryl group and monovalent heterocyclic group represented by Ar¹³ have the same meaning as described above.

It is preferable for the non-metal-containing polymer compound that Z⁶, Z⁷ and Z⁸ are a nitrogen atom in the above-described formula (11), since driving voltage can be reduced when used in a light emitting device.

Exemplified as the repeating unit represented by the formula (11) are the following repeating units.

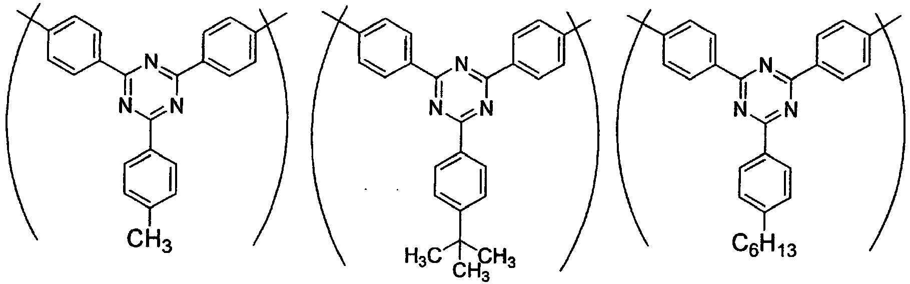

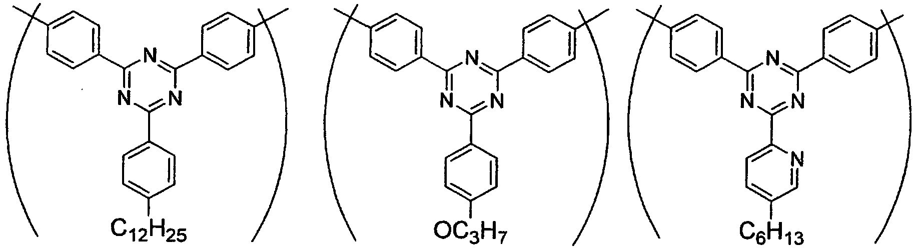

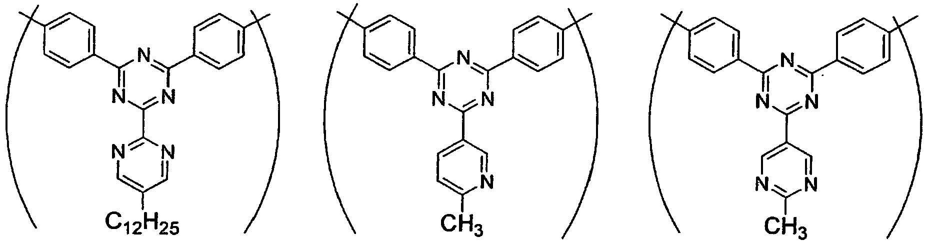

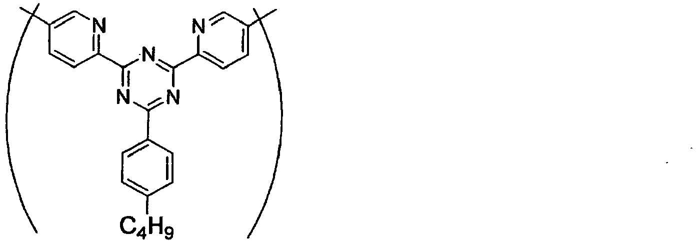

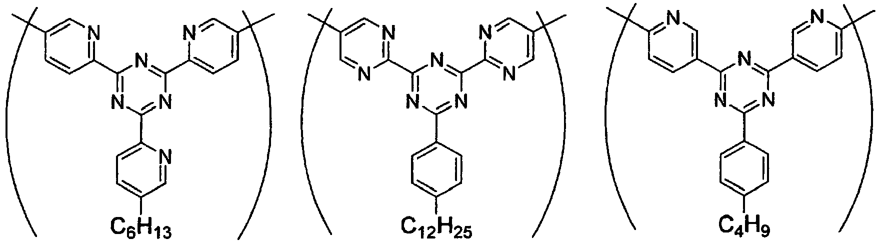

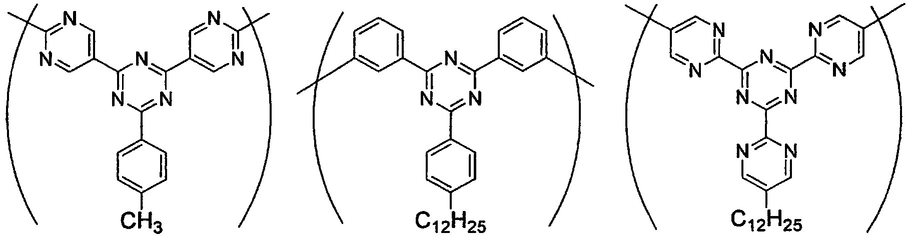

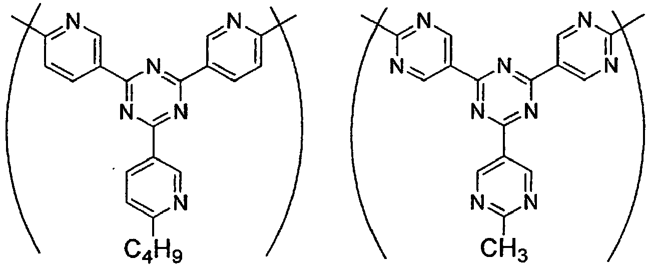

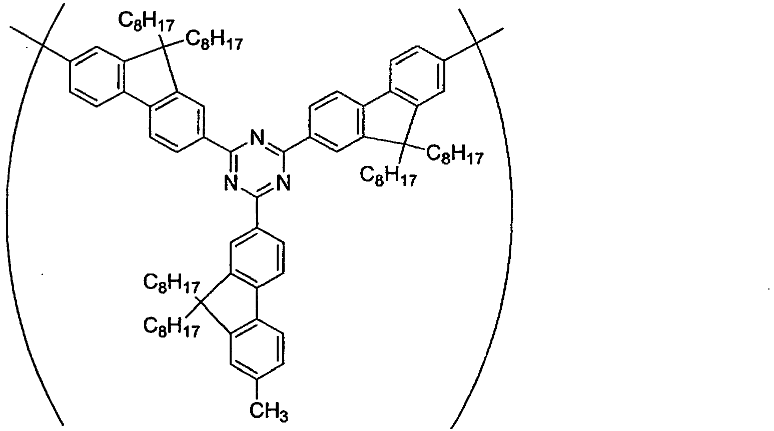

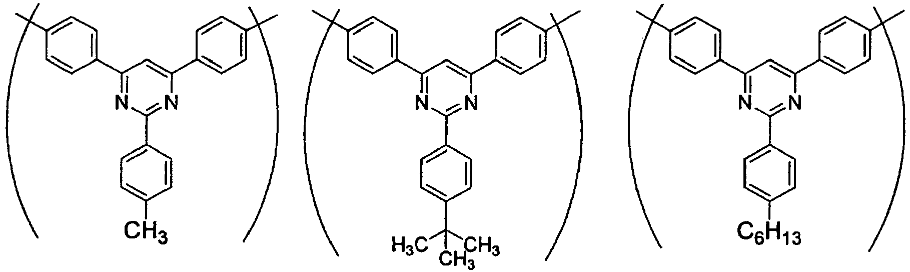

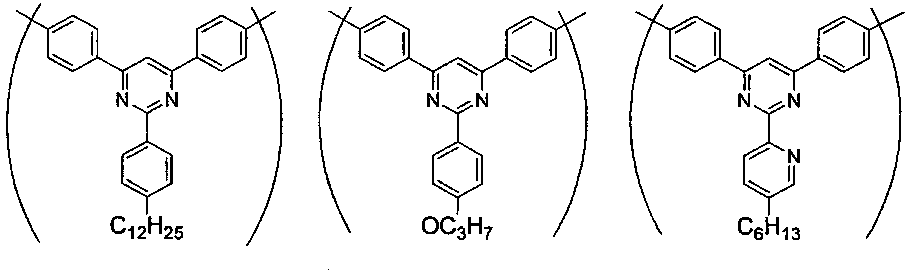

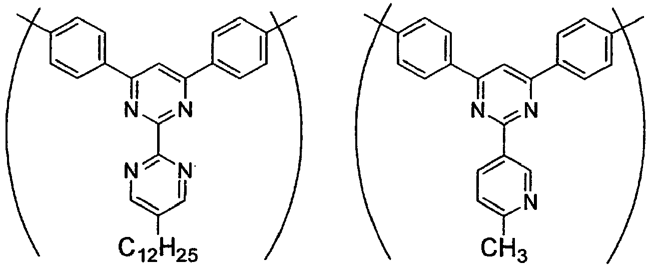

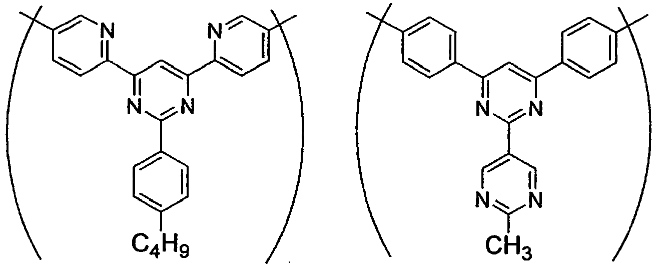

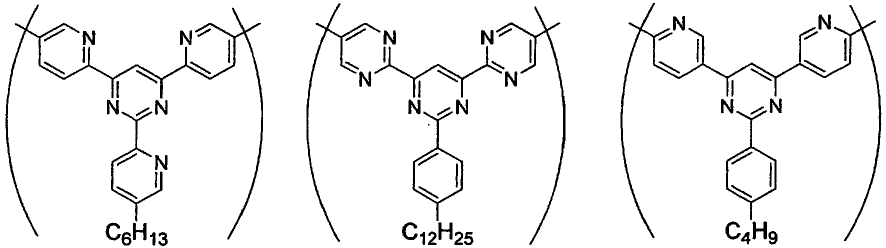

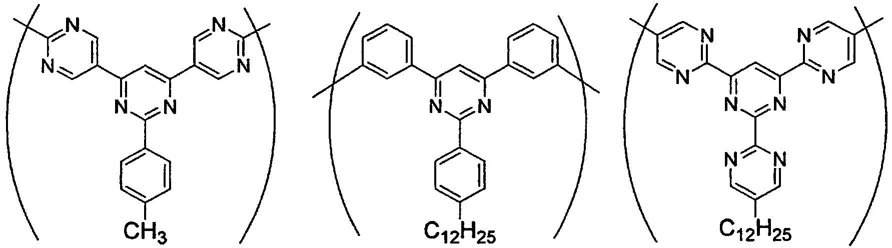

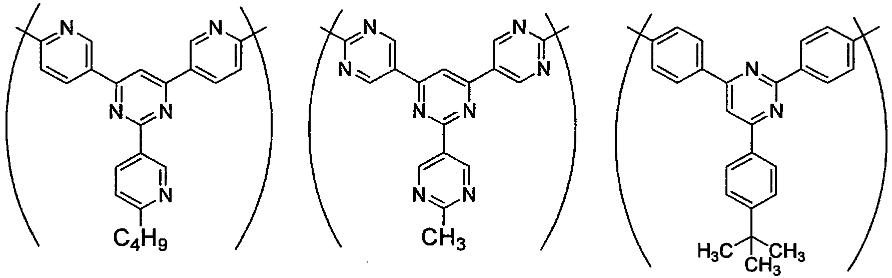

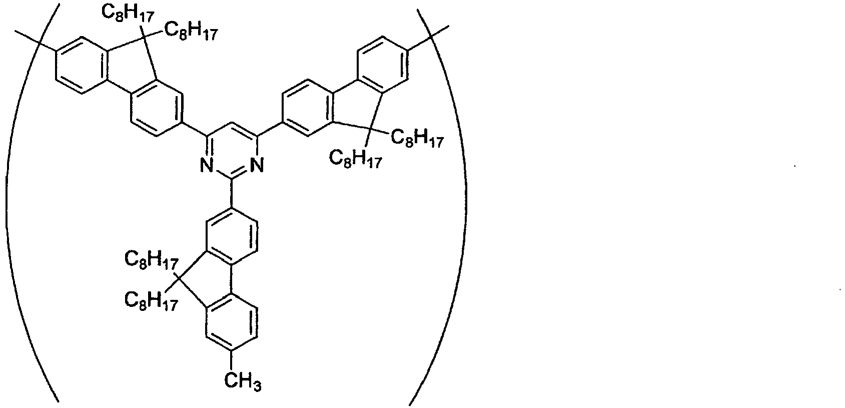

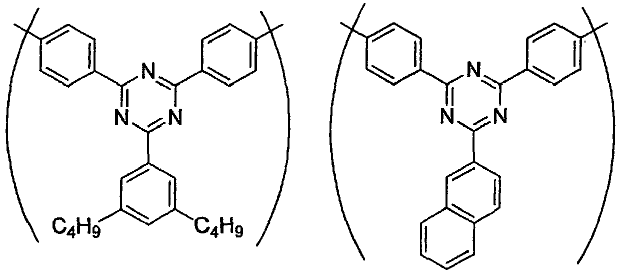

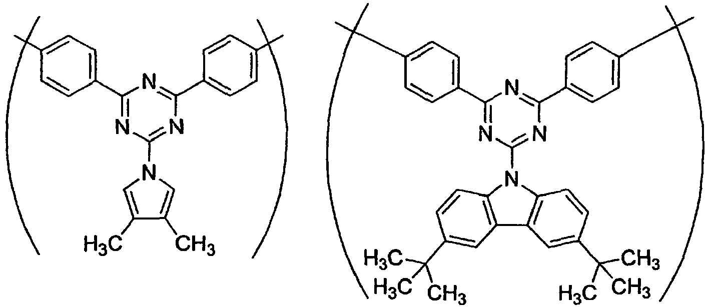

Specific examples of the non-metal-containing polymer compound include non-metal-containing polymer compounds represented by the following formulae. In the formulae, u1 to u179 are numbers satisfying the following formulae, and represent the number of each repeating unit when the number of all repeating units contained in each non-metal-containing polymer compound is 100. 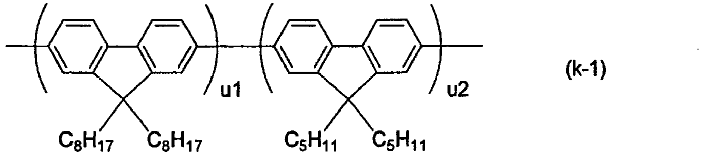 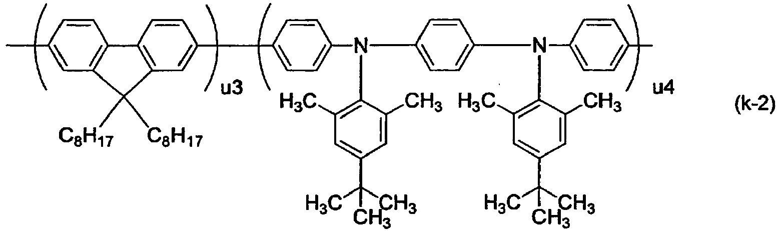 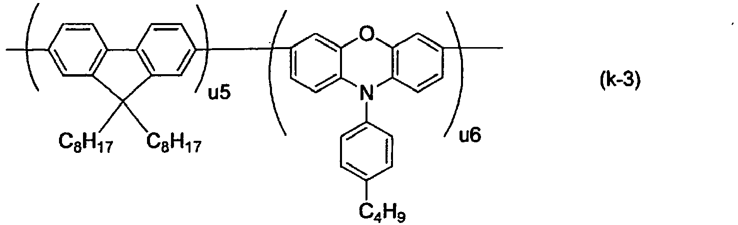 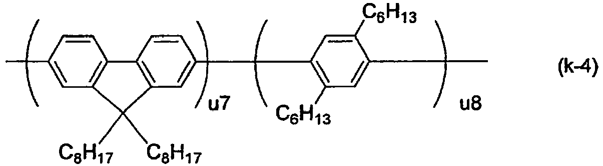 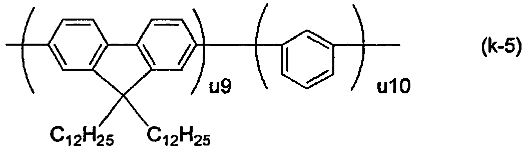 (in the formula (k-1), 5 ≤ u1 ≤ 95, 5 ≤ u2 ≤ 95, u1 + u2 = 100)
(in the formula (k-2), 5 ≤ u3 ≤ 99, 1 ≤ u4 ≤ 95, u3 + u4 = 100)
(in the formula (k-3), 5 ≤ u5 ≤ 99, 1 ≤ u6 ≤ 95, u5 + u6 = 100)
(in the formula (k-4), 5 ≤ u7 ≤ 95, 5 ≤ u8 ≤ 95, u7 + u8 = 100)
(in the formula (k-5), 5 ≤ u9 ≤ 95, 5 ≤ u10 ≤ 95, u9 + u10 = 100) 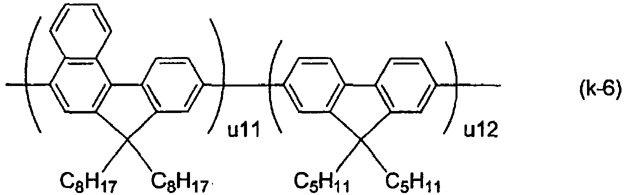 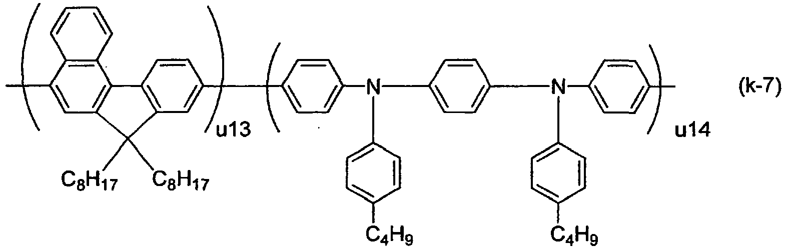 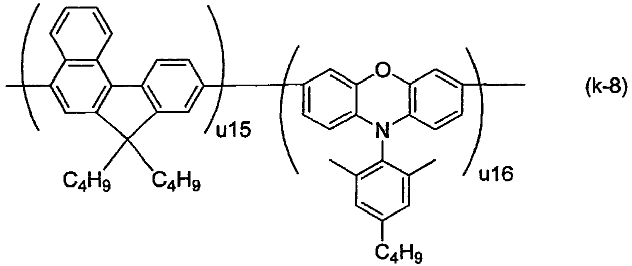 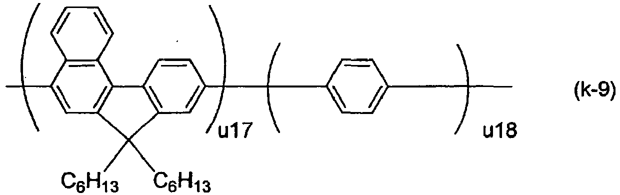 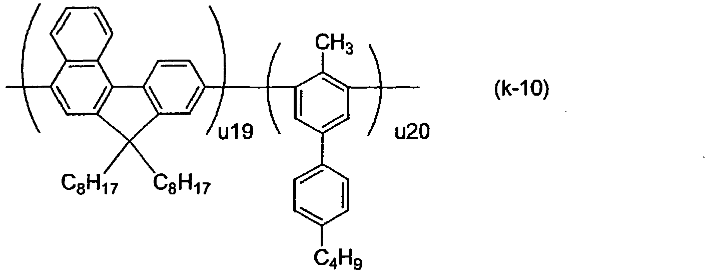 (in the formula (k-6), 5 ≤ n11 ≤ 95, 5 ≤ u12 ≤ 95, u11 + u12 = 100)
(in the formula (k-7), 5 ≤ u13 ≤ 99, 1 ≤ u14 ≤ 95, u13 + u14 = 100)
(in the formula (k-8), 5 ≤ u15 ≤ 99, 1 ≤ u16 ≤ 95, u15 + u16 = 100)
(in the formula (k-9), 5 ≤ u17 ≤ 95, 5 ≤ u18 ≤ 95, u17 + u18 = 100)
(in the formula (k-10), 5 ≤ u19 ≤ 95, 5 ≤ u20 ≤ 95, u19 + u20 = 100) 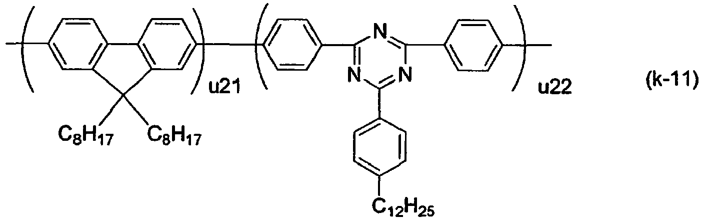 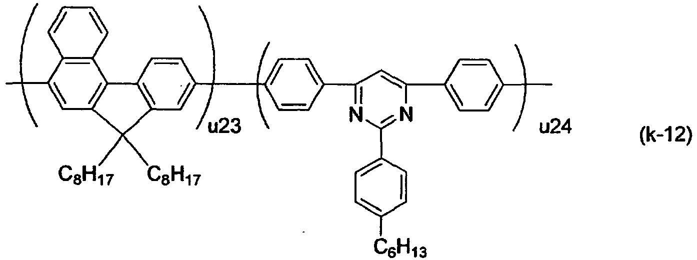 (in the formula (k-11), 5 ≤ u21 ≤ 99, 1 ≤ u22 ≤ 95, u21 + u22 = 100)
(in the formula (k-12), 5 ≤ u23 ≤ 99, 1 ≤ u24 ≤ 95, u23 + u24 = 100) 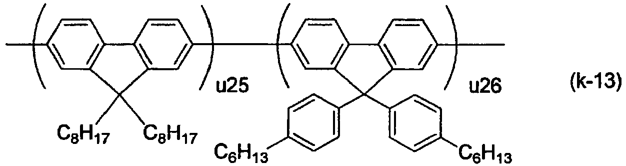 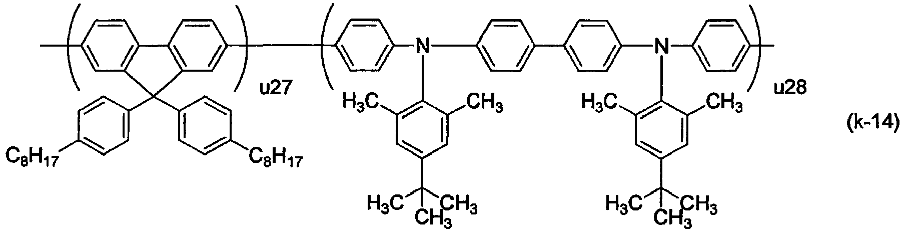 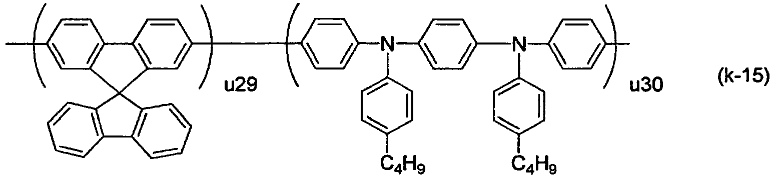 (in the formula (k-13), 5 ≤ u25 ≤ 95, 5 ≤ u26 ≤ 95, u25 + u26 = 100)
(in the formula (k-14), 5 ≤ u27 ≤ 99, 1 ≤ u28 ≤ 95, u27 + u28 = 100)
(in the formula (k-15), 5 ≤ u29 ≤ 99, 1 ≤ u30 ≤ 95, u29 + u30 = 100) 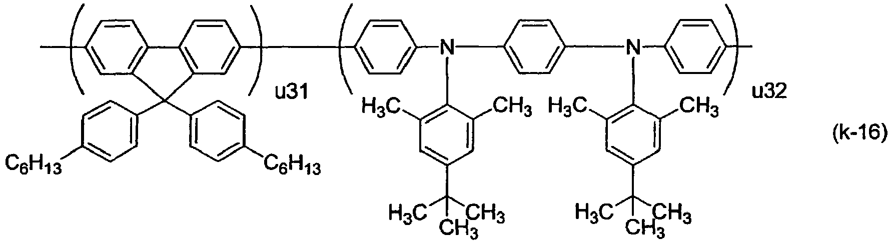 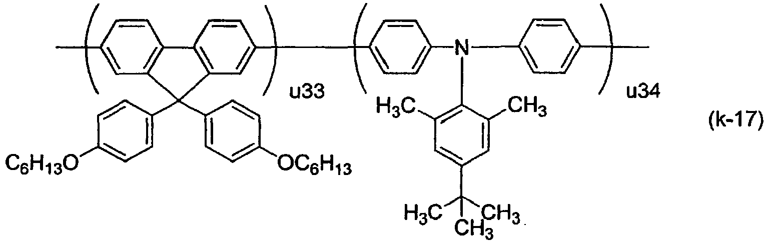 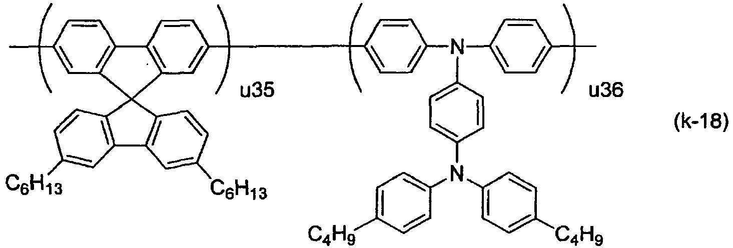 (in the formula (k-16), 5 ≤ u31 ≤ 99, 1 ≤ u32 ≤ 95, u31 + u32 = 100)
(in the formula (k-17), 5 ≤ u33 ≤ 99, 1 ≤ u34 ≤ 95, u33 + u34 = 100)
(in the formula (k-18), 5 ≤ u35 ≤ 99, 1 ≤ u36 ≤ 95, u35 + u36 = 100) 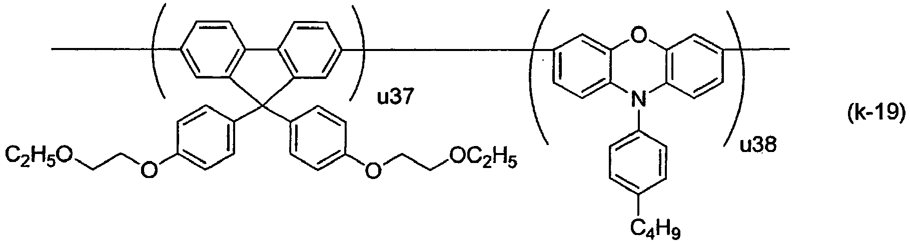 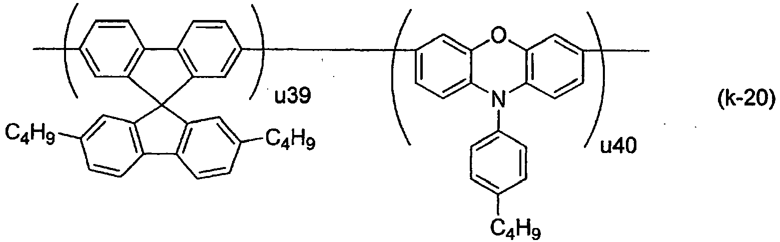 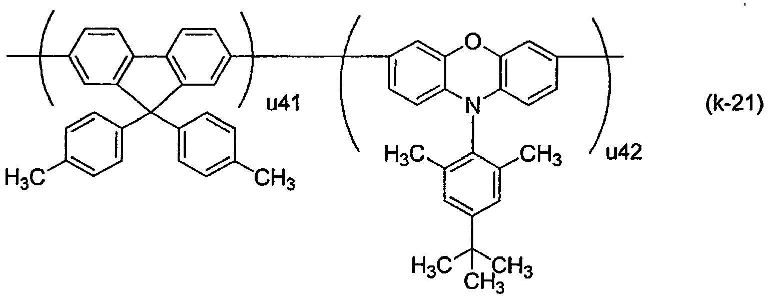 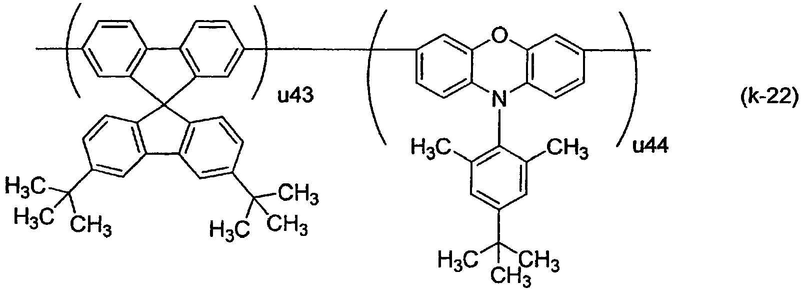 (in the formula (k-19), 5 ≤ u37 ≤ 99, 1 ≤ u38 ≤ 95, u37 + u38 = 100)
(in the formula (k-20), 5 ≤ u39 ≤ 99, 1 ≤ u40 ≤ 95, u39 + u40 = 100)
(in the formula (k-21), 5 ≤ u41 ≤ 99, 1 ≤ u42 ≤ 95, u41 + u42 = 100)
(in the formula (k-22), 5 ≤ u43 ≤ 99, 1 ≤ u44 ≤ 95, u43 + u44 = 100) 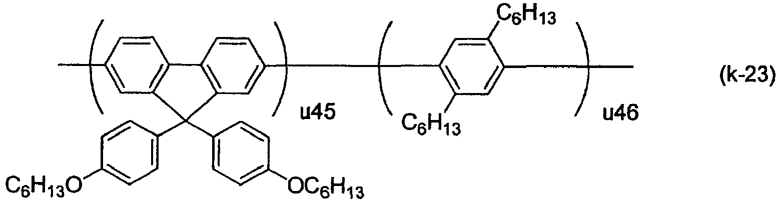 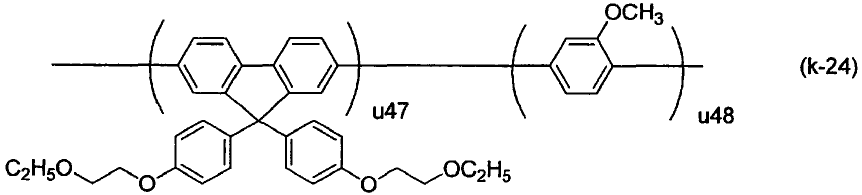 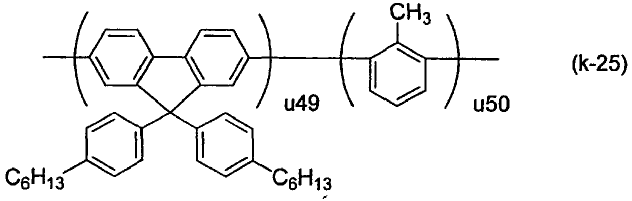 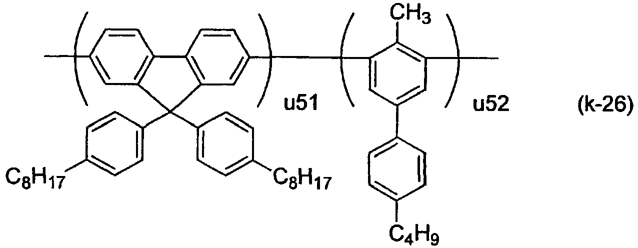 (in the formula (k-23), 5 ≤ u45 ≤ 95, 5 ≤ u46 ≤ 95, u45 + u46 = 100)
(in the formula (k-24), 5 ≤ u47 ≤ 95, 5 ≤ u48 ≤ 95, u47 + u48 = 100)
(in the formula (k-25), 5 ≤ u49 ≤ 95, 5 ≤ u50 ≤ 95, u49 + u50 = 100)
(in the formula (k-26), 5 ≤ u51 ≤ 95, 5 ≤ u52 ≤ 95, u51 + u52 = 100) 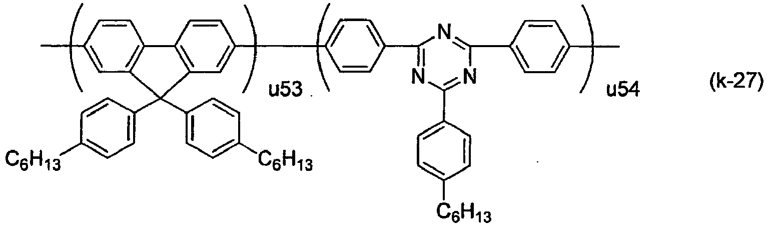 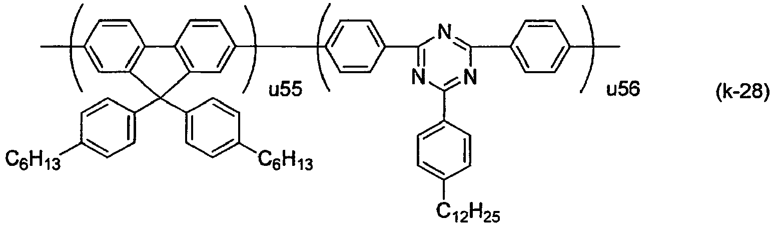 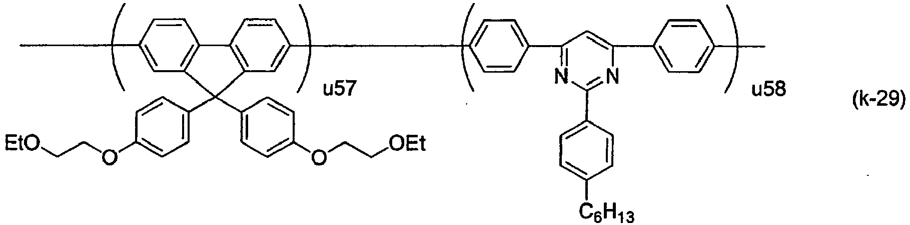 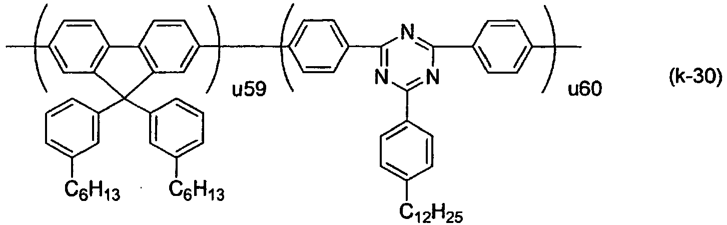 (in the formula (k-27), 5 ≤ u53 ≤ 99, 1 ≤ u54 ≤ 95, u53 + u54 = 100)
(in the formula (k-28), 5 ≤ u55 ≤ 99, 1 ≤ u56 ≤ 95, u55 + u56 = 100)
(in the formula (k-29), 5 ≤ u57 ≤ 99, 1 ≤ u58 ≤ 95, u57 + u58 = 100)
(in the formula (k-30), 5 ≤ u59 ≤ 99, 1 ≤ u60 ≤ 95, u59 + u60 = 100) 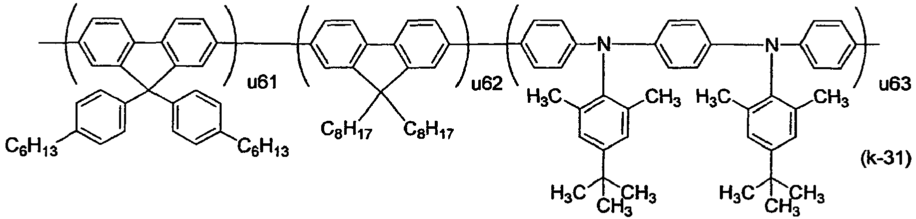 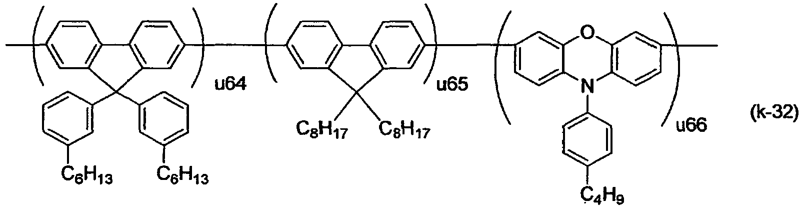 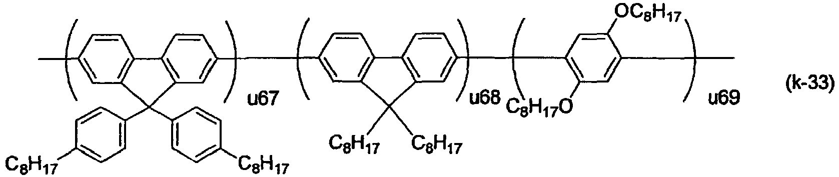 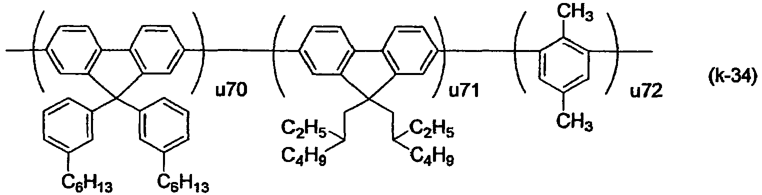 (in the formula (k-31), 5 ≤ u61 ≤ 94, 5 ≤ u62 ≤ 94, 1 ≤ u63 ≤ 50, u61 + u62 + u63 = 100)
(in the formula (k-32), 5 ≤ u64 ≤ 94, 5 ≤ u65 ≤ 94, 1 ≤ u66 ≤ 50, u64 + u65 + u66 = 100)
(in the formula (k-33), 5 ≤ u67 ≤ 90, 5 ≤ u68 ≤ 90, 5 ≤ u69 ≤ 90, u67 + u68 + u69 = 100)
(in the formula (k-34), 5 ≤ u70 ≤ 90, 5 ≤ u71 ≤ 90, 5 ≤ u72 ≤ 90, u70 + u71 + u72 = 100) 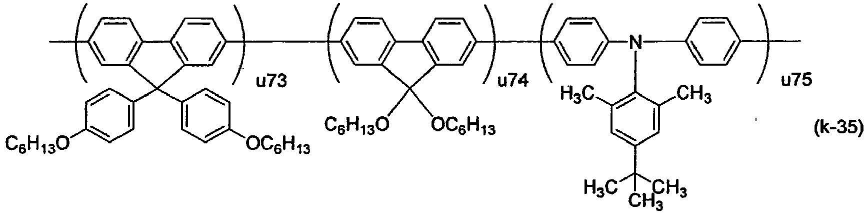 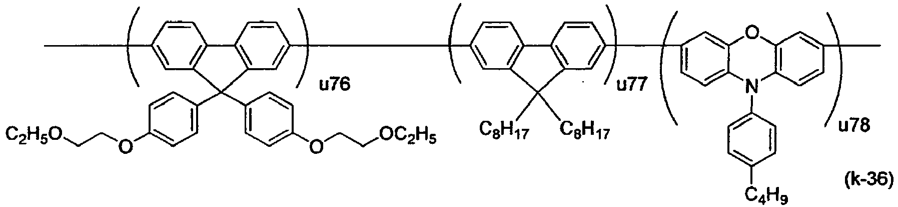 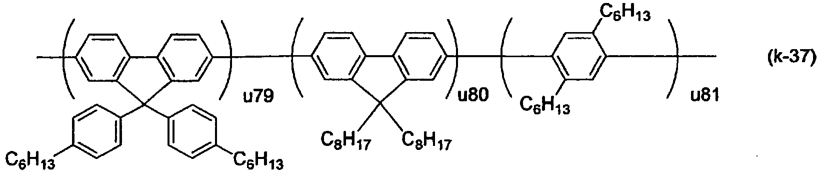 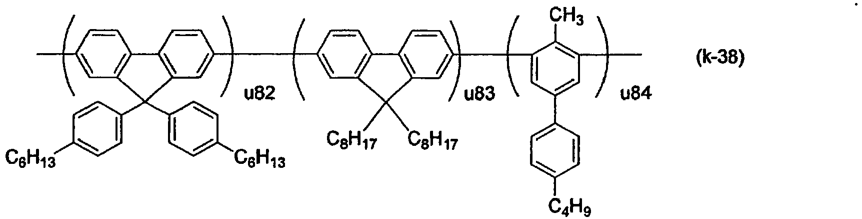 (in the formula (k-35), 5 ≤ u73 ≤ 94, 5 ≤ u74 ≤ 94, 1 ≤ u75 ≤ 50, u73 + u74 + u75 = 100)
(in the formula (k-36), 5 ≤ u76 ≤ 94, 5 ≤ u77 ≤94, 1 ≤ u78 ≤ 50, u76 + u77 + u78 = 100)
(in the formula (k-37), 5 ≤ u79 ≤ 90, 5 ≤ u80 ≤ 90, 5 ≤ u81 ≤ 90, u79 + u80 + u81 = 100)
(in the formula (k-38), 5 ≤ u82 ≤ 90, 5 ≤ u83 ≤ 90, 5 ≤ u84 ≤ 90, u82 + u83 + u84 = 100) 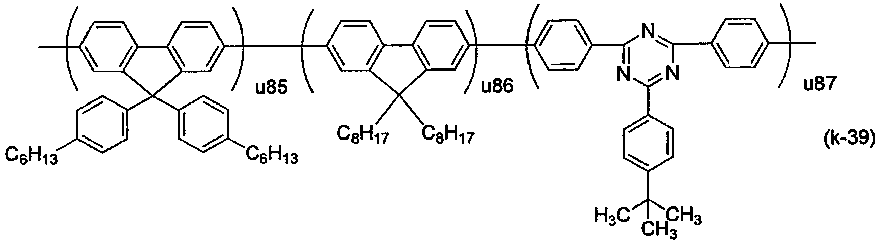 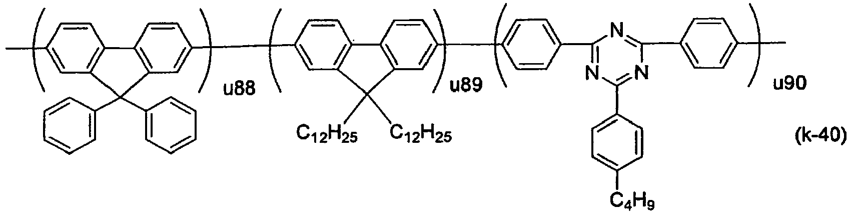 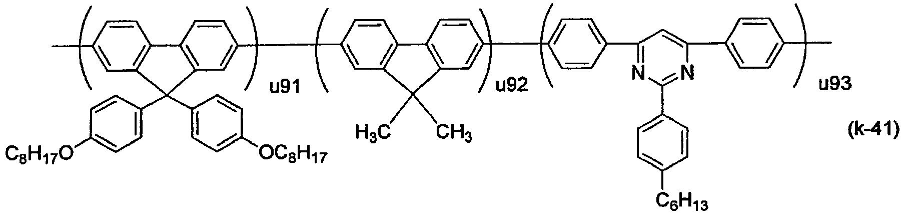 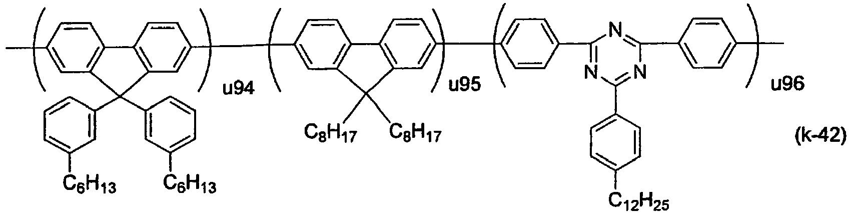 (in the formula (k-39), 5 ≤ u85 ≤ 94, 5 ≤ u86 ≤ 94, 1 ≤ u87 ≤ 30, u85 + u86 + u87 = 100)
(in the formula (k-40), 5 ≤ u88 ≤ 94, 5 ≤ u89 ≤ 94, 1 ≤ u90 ≤ 30, u88 + u89 + u90 = 100)
(in the formula (k-41), 5 ≤ u91 ≤ 94, 5 ≤ u92 ≤ 94, 1 ≤ u93 ≤ 30, u91 + u92 + u93 = 100)
(in the formula (k-42), 5 ≤ u94 ≤ 94, 5 ≤ u95 ≤ 94, 1 ≤ u96 ≤ 30, u94 + u95 + u96 = 100) 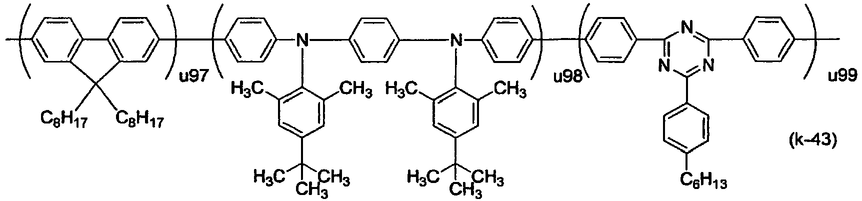 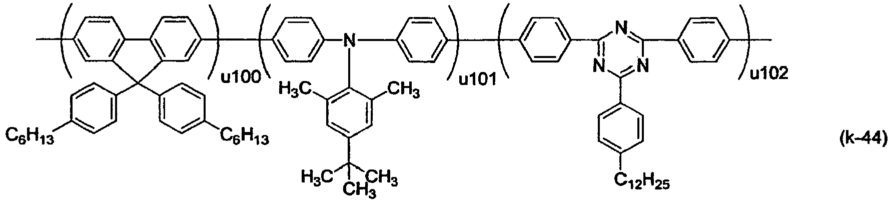 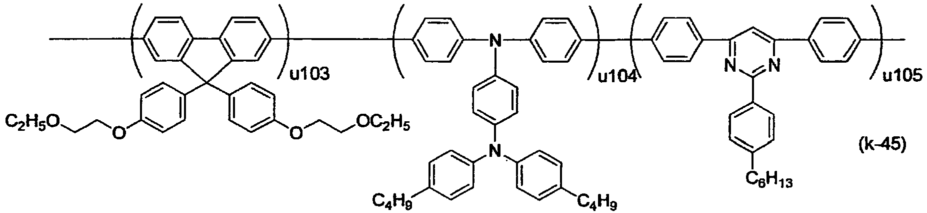 (in the formula (k-43), 5 ≤ u97 ≤ 98, 1 ≤ u98 ≤ 50, 1 ≤ u99 ≤ 45, u97 + u98 + u99 = 100)
(in the formula (k-44), 5 ≤ u100 ≤ 98, 1 ≤ u101 ≤ 50, 1 ≤ u102 ≤ 45, u100 + u101 + u102 = 100)
(in the formula (k-45), 5 ≤ u103 s 98, 1 ≤ u104 ≤ 50, 1 ≤ u105 ≤ 45, u103 + u104 + u105 = 100) 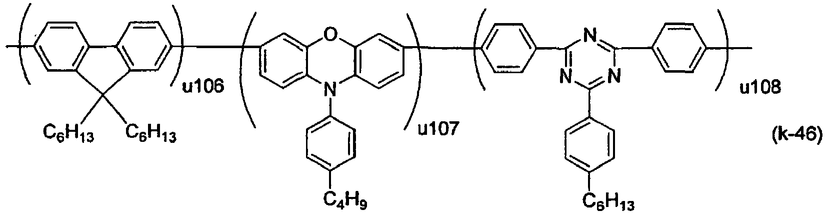 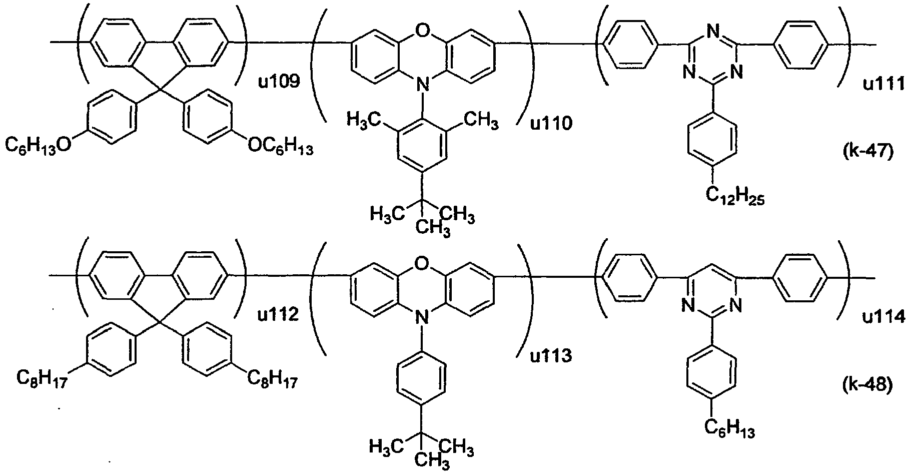 (in the formula (k-46), 5 ≤ u106 ≤ 98, 1 ≤ u107 ≤ 50, 1 ≤ u108 ≤ 45, u106 + u107 + u108 = 100)
(in the formula (k-47), 5 ≤ u109 ≤ 98, 1 ≤ u110 ≤ 50, 1 ≤ u111 ≤ 45, u109 + u110 + u111 = 100)
(in the formula (k-48), 5 ≤ u112 ≤ 98, 1 ≤ u113 ≤ 50, 1≤ u114 ≤ 45, u112 + u113 + u114 = 100) 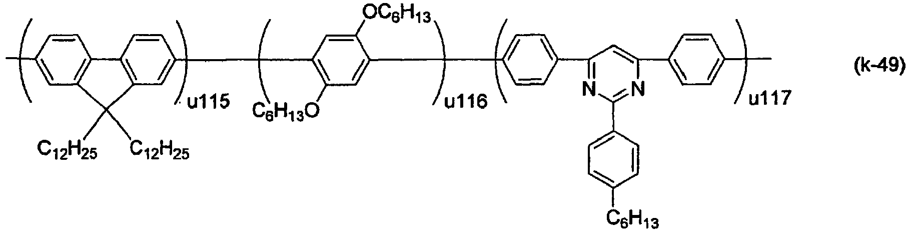 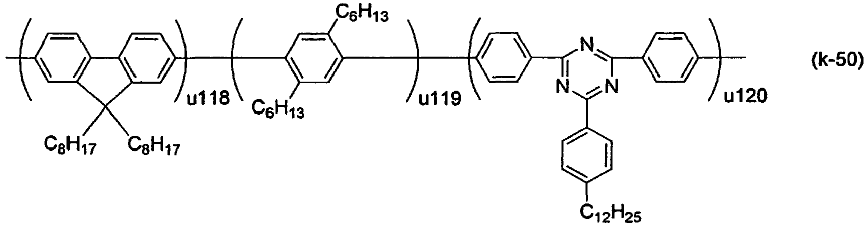 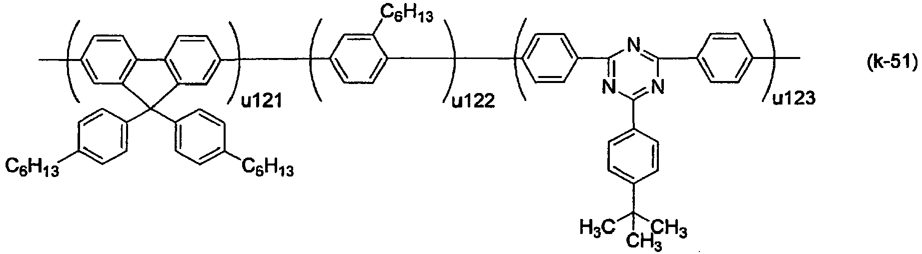 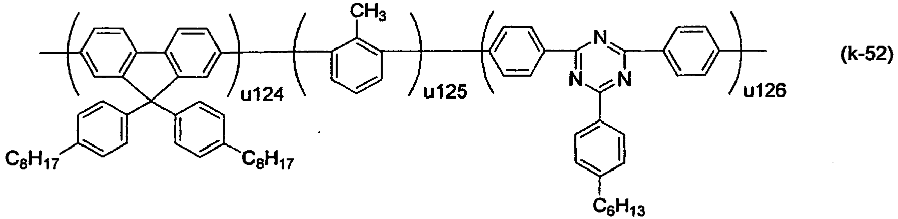 (in the formula (k-49), 5 ≤ u115 ≤ 94, 5 ≤ u116 ≤ 94, 1 ≤ u117 ≤ 30, u115 + u116 + u117 = 100)
(in the formula (k-50), 5 ≤ u118 ≤ 94, 5 ≤ u119 ≤ 94, 1 ≤ u120 ≤ 30, u118 + u119 + u120 = 100)
(in the formula (k-51), 5 ≤ u121 ≤ 94, 5 ≤ u122 ≤ 94, 1 ≤ u123 ≤ 30, u121 + u122 + u123 = 100)
(in the formula (k-52), 5 ≤ u124 ≤ 94, 5 ≤ u125 ≤ 94, 1 ≤ u126 ≤ 30, u124 + u125 + u126 = 100) 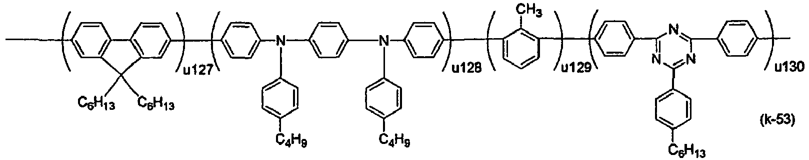 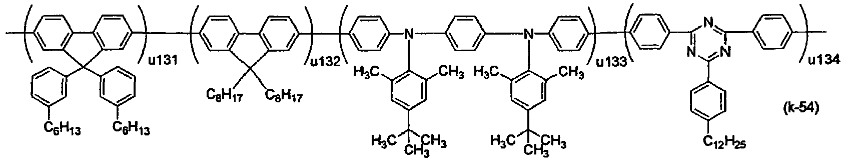 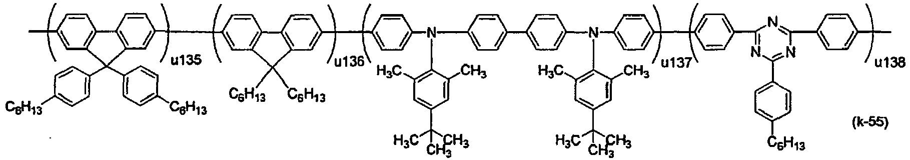 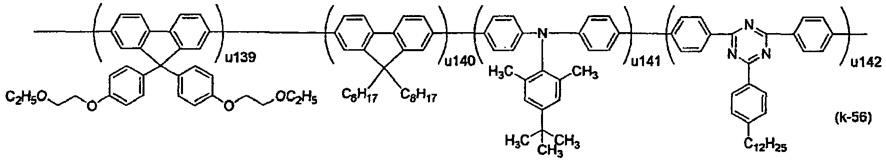 (in the formula (k-53), 5 ≤ u127 ≤ 93, 1 ≤ u128 ≤ 50, 5 ≤ u129 ≤ 93, 1 ≤ u130 ≤ 30, u127 + u128 + u129 + u130 = 100)
(in the formula (k-54), 5 ≤ u131 ≤ 93, 5 ≤ u132 ≤ 93, 1 ≤ u133 ≤ 50, 1 ≤ u134 ≤ 30, u131 + u132 + u133 + u134 = 100)
(in the formula (k-55), 5 ≤ u135 ≤ 93, 5 ≤ u136 ≤ 93, 1 ≤ u137 ≤ 50, 1 ≤ u138 ≤ 30, u135 + u136 + u137 + u138 = 100)
(in the formula (k-56), 5 ≤ u139 ≤ 93, 5 ≤ u140 ≤ 93, 1 ≤ u141 ≤ 50, 1 ≤ u142 ≤ 30, u139 + u140 + u141 + u142 = 100) 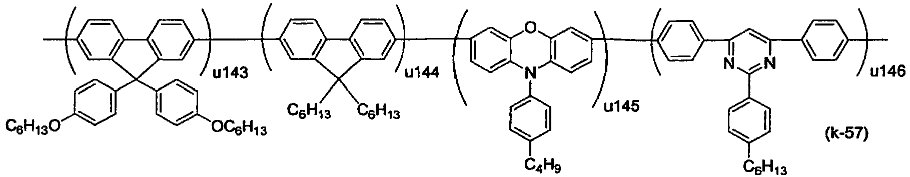 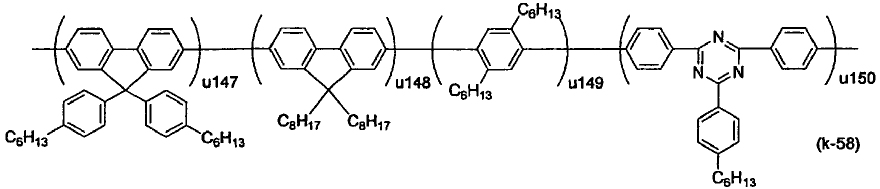 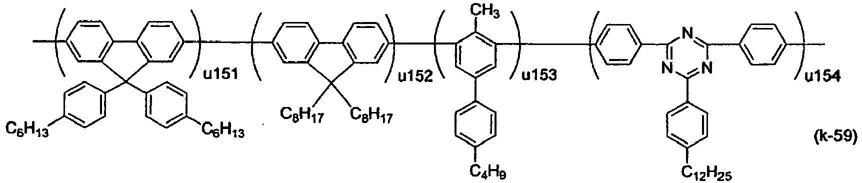 (in the formula (k-57), 5 ≤ u143 ≤ 93, 5 ≤ u144 ≤ 93, 1 ≤ u145 ≤ 50, 1 ≤ u146 ≤ 30, u143 + u144 + u145 + u146 = 100)
(in the formula (k-58), 5 ≤ u147 ≤ 89, 5 ≤ u148 ≤ 89, 5 ≤ u149 ≤ 89, 1 ≤ u150 ≤ 30, u147 + u148 + u149 + u150 = 100)
(in the formula (k-59), 5 ≤u151 5 89, 5 ≤ u152 ≤ 89, 5 ≤ u153 ≤ 89, 1 ≤ u154 ≤ 30, u151 + u152 + u153 + u154 = 100) 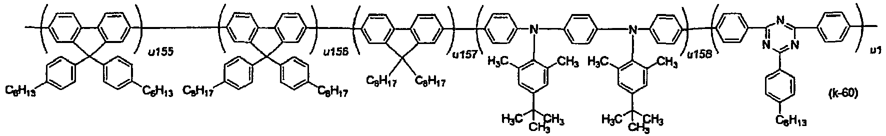 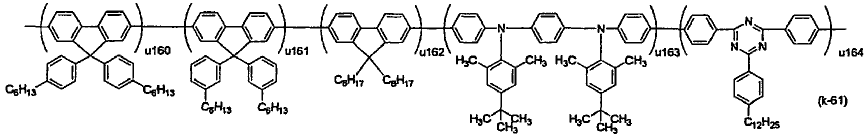 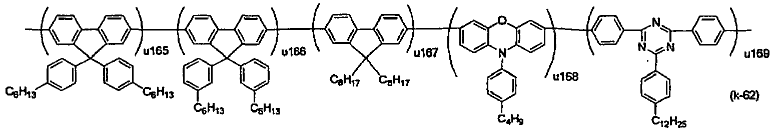 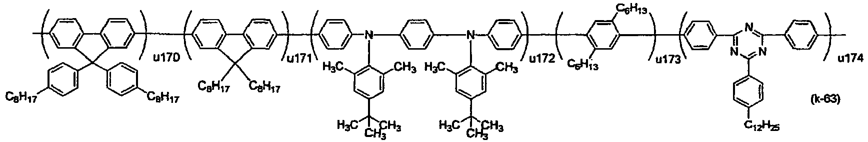 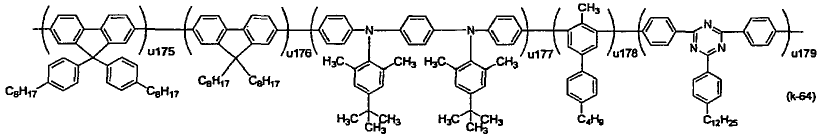 (in the formula (k-60), 5 ≤ u155 ≤ 88, 5 ≤ u156 ≤ 88, 5 ≤ u157 ≤ 88, 1 ≤ u158 ≤ 50, 1 ≤ u159 ≤ 30, u155 + u156 + u157 + u158 + u159 = 100)
(in the formula (k-61), 5 ≤ u160 ≤ 88, 5 ≤ u161 ≤ 88, 5 ≤ u162 ≤ 88, 1 ≤ u163 ≤ 50, 1 ≤ u164 ≤ 30, u160 + u161 + u162 + u163 + u164 = 100)
(in the formula (k-62), 5 ≤ u165 ≤ 88, 5 ≤ u166 ≤ 88, 5 ≤ u167 ≤ 88, 1 ≤ u168 ≤ 50, 1 ≤ u169 ≤ 30, u165 + u166 + u167 + u168 + u169 = 100)
(in the formula (k-63), 5 ≤ u170 ≤ 88, 5 ≤ u171 ≤ 88, 1 ≤ u172 ≤ 50, 5 ≤ u173 ≤ 88, 1 ≤ u174 ≤ 30, u170 + u171 + u172 + u173 + u174 = 100)
(in the formula (k-64), 5 ≤ u175 ≤ 88, 5 ≤ u176 ≤ 88, 1 ≤ u177 ≤ 50, 5 ≤ u178 ≤ 88, 1 ≤ u179 ≤ 30, u175 + u176 + u177 + u178 + u179 = 100)

The polymer compound represented by the above-described (k-1) to (k-64) may be any of a random copolymer, an alternative copolymer and a block copolymer.

### <Second composition>

The second composition of the present invention is a composition containing the metal-containing polymer compound of the present invention. The second composition of the present invention may contain at least one member selected from the group consisting of a charge transporting material, a light emitting material, a solvent and a dispersing medium. The second composition of the present invention may contain a non-metal-containing polymer compound. Components contained in the second composition of the present invention may each be used singly or in combination.

In the second composition of the present invention, the above-described charge transporting material, the above-described light emitting material, the above-described solvent, the above-described dispersing medium and the above-described non-metal-containing polymer compound are the same as in the above-described the first composition unless otherwise stated.

When the second composition of the present invention contains at least one member selected from the group consisting of a charge transporting material, a light emitting material, a solvent and a dispersing medium, the proportion of the above-described metal-containing polymer compound is usually 0.1 to 90 parts by weight, preferably 0.5 to 80 parts by weight, more preferably 0.5 to 50 parts by weight, with respect to 100 parts by weight of the total amount of the composition.

The second composition of the present invention may contain a non-metal-containing polymer compound since the device fabrication cost can be reduced when fabricated into a light emitting device. When the second composition of the present invention contains a non-metal-containing polymer compound, the proportion of the non-metal-containing polymer compound is preferably 0.1 to 100 parts by weight, more preferably 0.1 to 30 parts by weight, with respect to 100 parts by weight of the above-described metal-containing polymer compound.

### <Device>

The device of the present invention is a device comprising the metal complex of the present invention, the polymer compound of the present invention or the composition of the present invention, and examples thereof include devices having electrodes consisting of an anode and a cathode, and a layer disposed between the electrodes and containing the metal complex of the present invention, the polymer compound of the present invention or the composition of the present invention. Hereinafter, a case in which the device of the present invention is a light emitting device will be illustrated as a typical example.

The light emitting device of the present invention is a device having a pair of electrodes consisting of an anode and a cathode, and a film composed of one layer (single layer type) or several layers (multi-layer type) having a light emitting layer between the electrodes. At least one of layers constituting the above-described film contains the metal complex of the present invention, the polymer compound of the present invention or the composition of the present invention. The content of the above-described metal complex, the above-described polymer compound or the above-described composition in the above-described film is usually 0.1 to 100 wt%, preferably 0.1 to 80 wt%, more preferably 0.5 to 60 wt%, with respect to the whole light emitting layer. It is preferable for the light emitting device of the present invention that the above-described light emitting layer contains the above-described metal complex, the above-described polymer compound or the above-described composition.

When the light emitting device of the present invention is of single layer type, the above-described film is a light emitting layer and this light emitting layer contains the above-described metal complex or the above-described polymer compound.

When the light emitting device of the present invention is of multi-layer type, for example, the following constitutions are listed.
(a) anode/hole transporting layer/light emitting layer/cathode
(b) anode/hole injection layer/hole transporting layer/light emitting layer/cathode
(c) anode/light emitting layer/electron transporting layer/cathode
(d) anode/light emitting layer/electron transporting layer/electron injection layer/cathode
(e) anode/hole transporting layer/light emitting layer/electron transporting layer/cathode
(f) anode/hole injection layer/hole transporting layer/light emitting layer/electron transporting layer/cathode
(g) anode/hole injection layer/hole transporting layer/light emitting layer/electron transporting layer/electron injection layer/cathode

The anode of the light emitting device of the present invention feeds holes to a hole injection layer, a hole transporting layer, a light emitting layer and the like, and preferably has a work function of 4.5 eV or more.

As the anode material, metals, alloys, metal oxides, electric conductive compounds, mixtures thereof and the like can be used. The anode material includes electric conductive metal oxides such as tin oxide, zinc oxide, indium oxide, indium tin oxide (ITO) and the like, metals such as gold, silver, chromium, nickel and the like, further, mixtures or laminates of these electric conductive metal oxides with metals, inorganic electric conductive substances such as copper iodide, copper sulfide and the like, organic electric conductive materials such as polyanilines, polythiophenes (PEDOT and the like), polypyrrole and the like, laminates of these materials with ITO, and the like.

The cathode of the light emitting device of the present invention feeds electrons to an electron injection layer, an electron transporting layer, a light emitting layer and the like.

As the cathode material, metals, alloys, metal halides, metal oxides, electric conductive compounds or mixtures thereof can be used, and examples thereof include alkali metals (lithium, sodium, potassium, cesium and the like) and fluorides and oxides thereof, alkaline earth metals (magnesium, calcium, barium and the like) and fluorides and oxides thereof, gold, silver, lead, aluminum, alloys and mixed metals (sodium-potassium alloy, sodium-potassium mixed metal, lithium-aluminum alloy, lithium-aluminum mixed metal, magnesium-silver alloy, magnesium-silver mixed metal and the like), rare earth metals (ytterbium and the like), etc.

The hole injection layer and the hole transporting layer of the light emitting device of the present invention have a function of injecting holes from an anode, a function of transporting holes or a function of blocking electrons injected from a cathode.

As the material of the hole injection layer and the hole transporting layer, known materials can be used, and examples thereof include carbazole derivatives, triazole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styrylanthracene derivatives, fluorenone derivatives, hydrazone derivatives, stilbene derivatives, silazane derivatives, aromatic tertiary amine compounds, styrylamine compounds, aromatic dimethylidyne compounds, porphyrin compounds, polysilane compounds, poly(N-vinylcarbazole) derivatives, organosilane derivatives, polymers containing them, and the like. Additionally, aniline copolymers, and electric conductive high molecular weight oligomers such as thiophene oligomers, polythiophene and the like are listed. These materials may be used singly or in combination. The above-described hole injection layer and the above-described hole transporting layer may have a single layer structure composed of one or more of the above-described materials, or may have a multi-layer structure composed of several layers of the same composition or different compositions.

As the film formation method of the hole transporting layer, a method by film formation from a solution is exemplified in the case of use of a compound having high molecular weight.

As the solvent used in film formation from a solution, preferable are solvents capable of dissolving or uniformly dispersing a material used in the hole transporting layer. Exemplified as the solvent are chlorine-based solvents such as chloroform, methylene chloride, 1,2-dichloroethane, 1,1,2-trichloroethane, chlorobenzene, o-dichlorobenzene and the like, ether solvents such as tetrahydrofuran, dioxane, anisole and the like, aromatic hydrocarbon solvents such as toluene, xylene, mesitylene, ethylbenzene, cyclohexylbenzene and the like, aliphatic hydrocarbon solvents such as cyclohexane, methylcyclohexane, n-pentane, n-hexane, n-heptane, n-octane, n-nonane, n-decane and the like, ketone solvents such as acetone, methyl ethyl ketone, cyclohexanone and the like, ester solvents such as ethyl acetate, butyl acetate, ethyl cellosolve acetate and the like, polyhydric alcohols such as ethylene glycol, ethylene glycol monobutyl ether, ethylene glycol monoethyl ether, ethylene glycol monomethyl ether, dimethoxyethane, propylene glycol, diethoxymethane, triethylene glycol monoethyl ether, glycerin, 1,2-hexane diol and the like and derivatives thereof, alcohol solvents such as methanol, ethanol, propanol, isopropanol, cyclohexanol and the like, sulfoxide solvents such as dimethyl sulfoxide and the like, and amide solvents such as N-methyl-2-pyrrolidone, N,N-dimethylformamide and the like. These solvents may be used singly or in combination.

For film formation from a solution, coating methods such as a spin coat method, a casting method, a micro gravure coat method, a gravure coat method, a bar coat method, a roll coat method, a wire bar coat method, a dip coat method, a spray coat method, a screen printing method, a flexo printing method, an offset printing method, an inkjet print method, a capillary coat method, a nozzle coat method and the like can be used.

The thickness of a hole transporting layer shows the optimum value varying depending on a material to be used, and may advantageously be selected so as to give suitable values of driving voltage and light emission efficiency, and is usually 1 nm to 1 µm, preferably 2 to 500 nm, more preferably 5 to 200 nm.

When a hole transporting layer is disposed adjacent to a light emitting layer, particularly when both layers are formed by a coating method, the materials of the two layers are mixed to exert an undesirable effect on device properties and the like in some cases. When a hole transporting layer is formed by a coating method before formation of a light emitting layer by a coating method, the method of reducing mixing of the materials of the two layers includes a method of forming a hole transporting layer by a coating method, heating the hole transporting layer to become insoluble in an organic solvent used in fabrication of a light emitting layer, then, forming a light emitting layer. The temperature of the above-described heating is usually 150 to 300°C. The time of the above-described heating is usually 1 minute to 1 hour. In this case, for removal of components not insolubled in a solvent by heating, the hole transporting layer may advantageously be rinsed with a solvent used for formation of a light emitting layer, before formation of a light emitting layer after heating. If insolubilization in a solvent by heating is carried out sufficiently, the rinse process can be skipped. For sufficient insolubilization in a solvent by heating, it is preferable to use a compound containing at least one polymerizable group in the molecule as the compound of high molecular weight to be used in the hole transporting layer. Further, the number of the above-described polymerizable group is preferably 5% or more based on the number of constituent units in the molecule.

Specific examples of the compound of high molecular weight used in the hole transporting layer include compounds represented by the following formulae. In the formulae, v, w, x and y represent composition ratio.

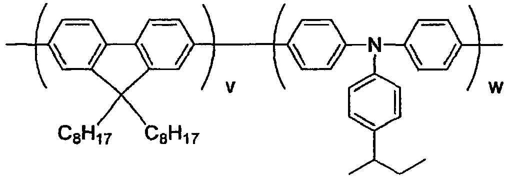

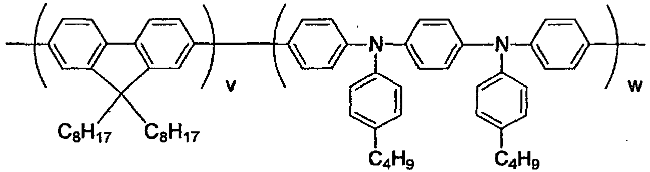

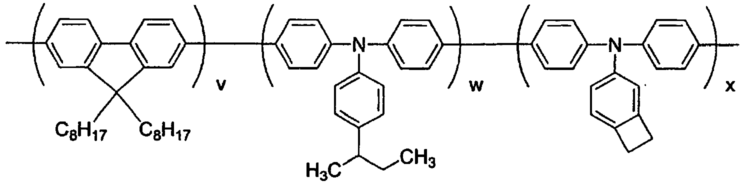

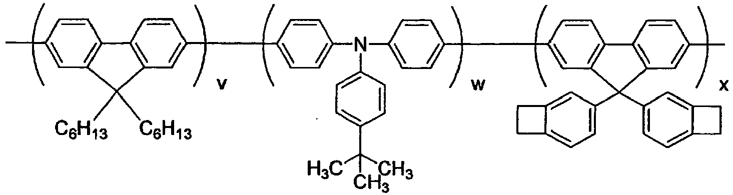

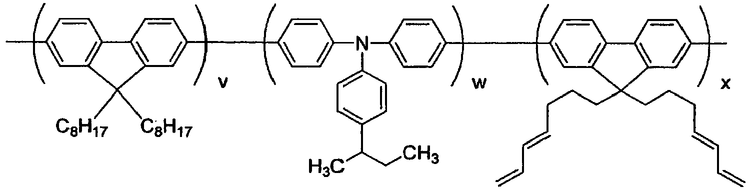

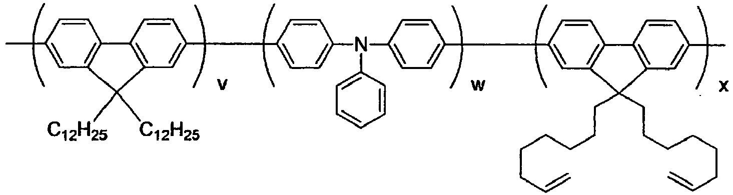

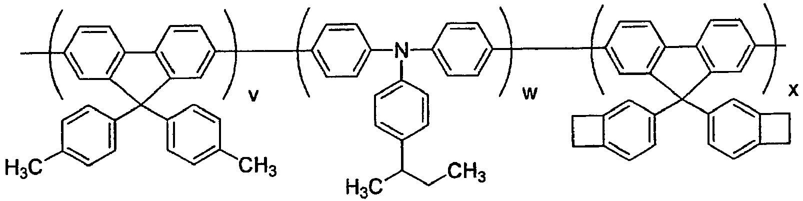

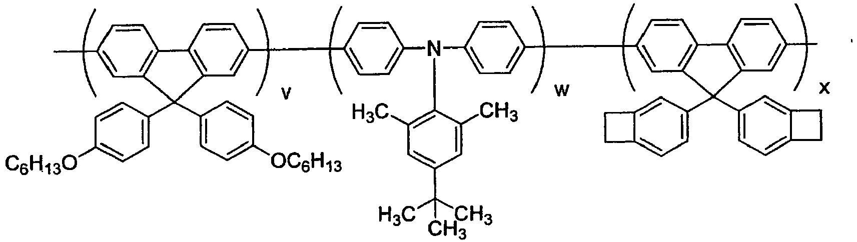

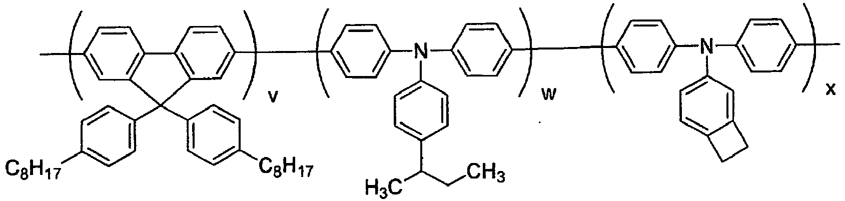

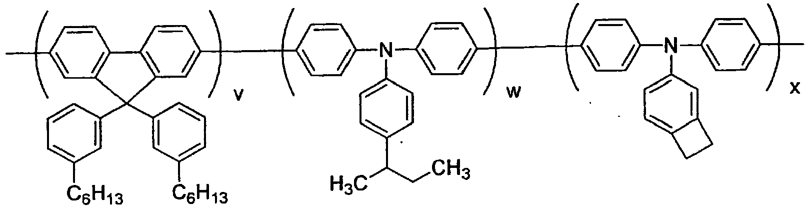

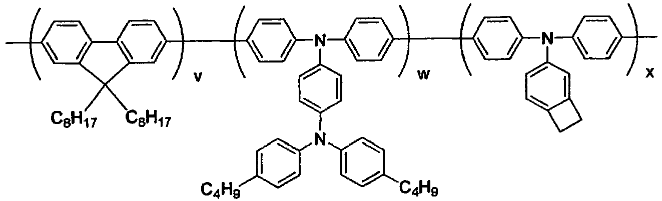

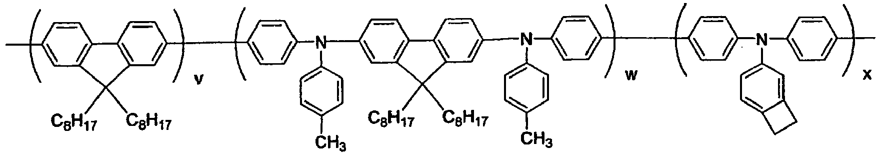

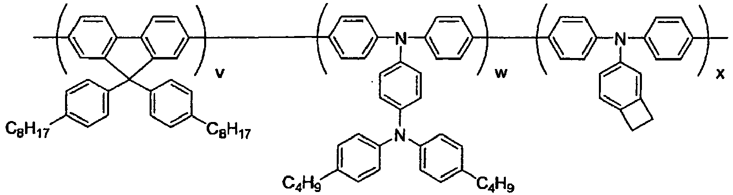

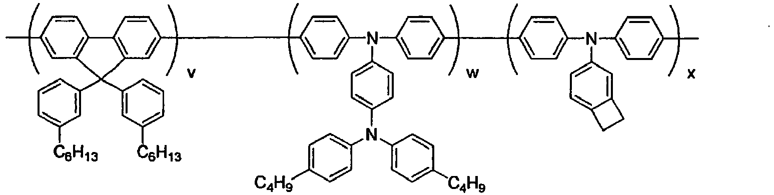

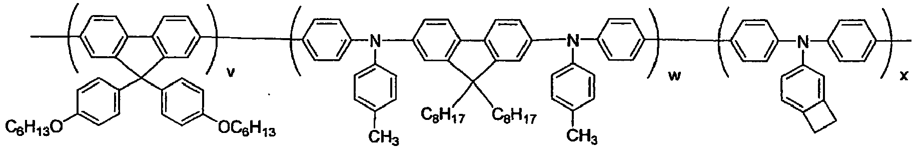

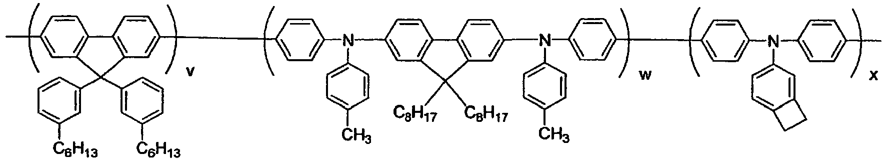

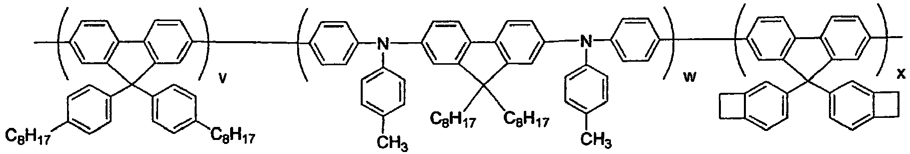

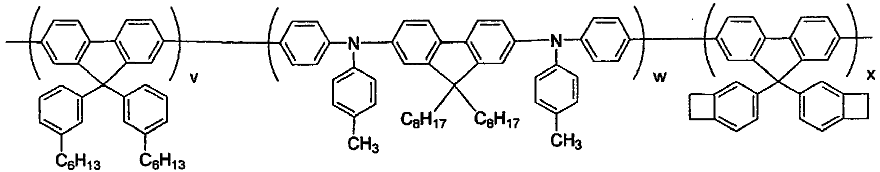

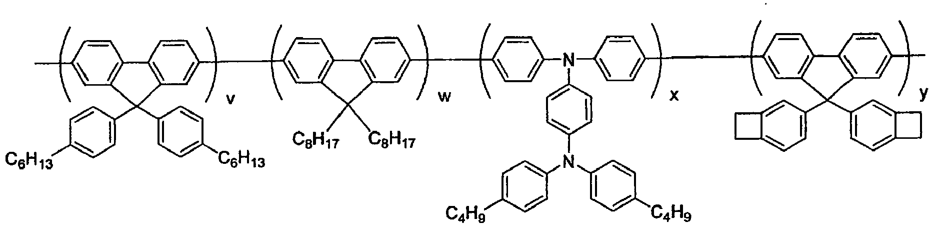

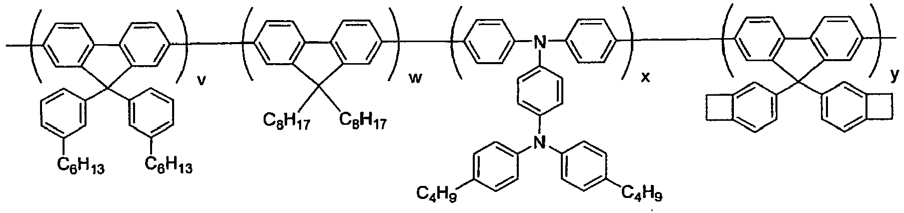

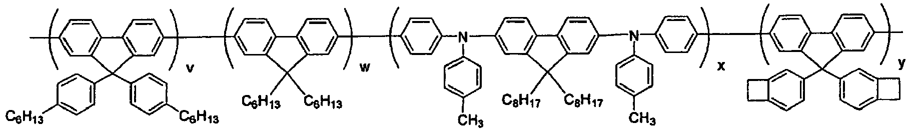

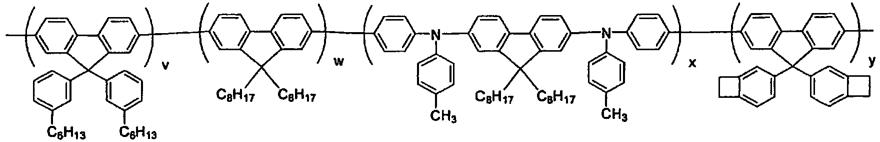

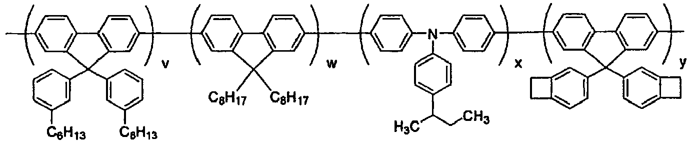

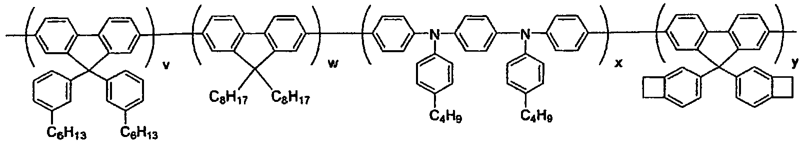

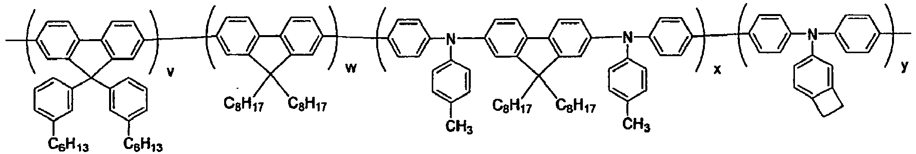

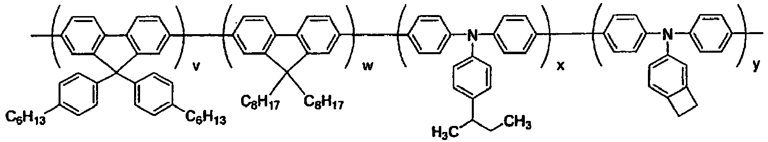

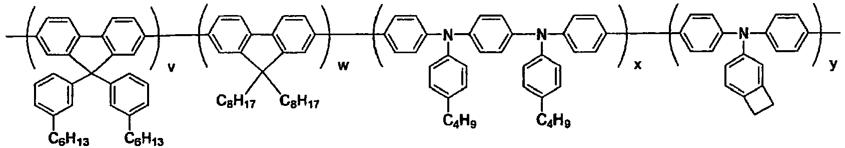

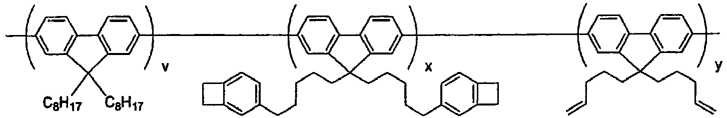

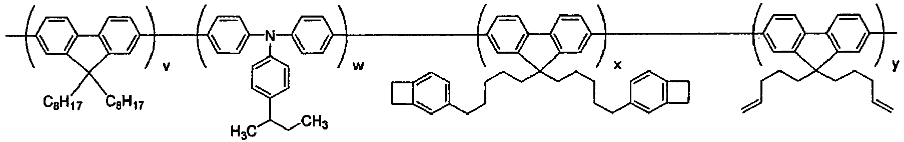

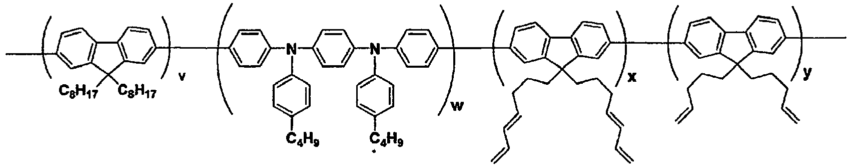

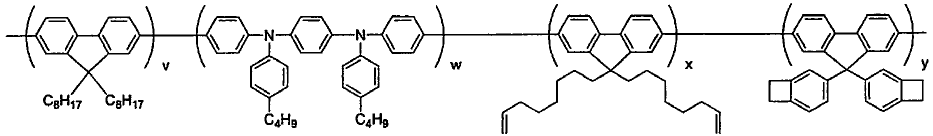

The electron injection layer and the electron transporting layer of the light emitting device of the present invention have a function of injecting electrons from a cathode, a function of transporting electrons or a function of blocking holes injected from an anode.

The materials of the electron injection layer and the electron transporting layer include triazole derivatives, oxazole derivatives, oxadiazole derivatives, imidazole derivatives, fluorenone derivatives, anthraquinodimethane derivatives, anthrone derivatives, diphenylquinone derivatives, thiopyran dioxide derivatives, carbodiimide derivatives, fluorenylidenemethane derivatives, distyrylpyrazine derivatives, aromatic ring tetracarboxylic acid anhydrides such as naphthalene, perylene and the like, phthalocyanine derivatives; metal complexes of 8-quinolinol derivatives; various metal complexes typified by metal complexes containing metallophthalocyanine, benzooxazole or benzothiazole as a ligand; and organosilane derivatives. These materials may be used singly or in combination. The above-described electron injection layer and the above-described electron transporting layer may have a single layer structure composed of one or more of the above-described materials, or may have a multi-layer structure composed of several layers of the same composition or different compositions.

In the light emitting device of the present invention, insulating or semiconductive inorganic compounds can also be used as the material of the electron injection layer and the electron transporting layer. If the electron injection layer and the electron transporting layer are constituted of an insulating body or a semiconductor, current leakage can be prevented effectively and electron injectability can be improved.

The above-described insulating body includes alkali metal chalcogenides, alkaline earth metal chalcogenides, alkali metal halides or alkaline earth metal halides.

As the alkaline earth metal chalcogenide, CaO, BaO, SrO, BeO, BaS and CaSe are preferable.

The above-described semiconductor includes oxides, nitrides and oxynitrides containing at least one element selected from the group consisting of Ba, Ca, Sr, Yb, Al, Ga, In, Li, Na, Cd, Mg, Si, Ta, Sb and Zn.

These materials may be used singly or in combination.

In the light emitting device of the present invention, a reducing dopant may be added to an interface region of a film in contact with a cathode.

As the reducing dopant, at least one compound selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, alkali metal complexes, alkaline earth metal complexes and rare earth metal complexes is preferable.

The light emitting layer of the light emitting device of the present invention has a function capable of injecting holes from an anode or a hole injection layer and capable of injecting electrons from a cathode or an electron injection layer in application of voltage, a function of transferring injected charges (electrons and holes) by the force of electric field, and a function of providing a field for recombination of electrons and holes, thereby causing light emission.

The above-described light emitting layer preferably contains the above-described metal complex, the above-described polymer compound or the above-described composition, and a host material for the above-described metal complex, the above-described polymer compound or the above-described composition as a guest material may also be contained.

The above-described host material includes a compound having a fluorine skeleton, a compound having a carbazole skeleton, a compound having a diarylamine skeleton, a compound having a pyridine skeleton, a compound having a pyrazine skeleton, a compound having a triazine skeleton and a compound having an arylsilane skeleton. It is preferable that T1 (energy level of lowest triplet excited state) of the above-described host material is larger than that of the guest material, and it is further preferable that its difference is larger than 0.2 eV. The above-described host material may be a low molecular weight compound or a high molecular weight compound. A light emission layer in which the above-described metal complex, the above-described polymer compound or the above-described composition is doped in the above-described host material can be formed, by mixing the above-described host material with the above-described metal complex, the above-described polymer compound or the above-described composition and coating the mixture, or co-evaporation-depositing them, or the like.

The method of forming each layer used in the light emitting device of the present invention includes vapor deposition methods (resistance heating deposition method, electron beam method and the like), sputtering methods, LB methods, molecule lamination methods, coating methods [wet process] (casting method, spin coat method, bar coat method, blade coat method, roll coat method, gravure printing, screen printing, inkjet method and the like), and the like. Of them, coating methods are preferable since the production process can be simplified. In the above-described coating methods, each layer can be formed by preparing a coating solution (composition) containing the metal complex of the present invention or the polymer compound of the present invention, coating the coating solution on a desired layer (or electrode), and drying this. In the above-described coating solution, a host material, an antioxidant, a viscosity modifier and a resin as a binder may be contained.

The above-described resin may be in the state of dissolution or dispersion in a solvent. The above-described resin includes polymer compounds such as polyvinylcarbazoles, polyolefins and the like, and specific examples thereof include polyvinyl chloride, polycarbonates, polystyrene, polymethyl methacrylate, polybutyl methacrylate, polyesters, polysulfones, polyphenylene oxides, polybutadiene, poly(N-vinylcarbazole), hydrocarbon resins, ketone resins, phenoxy resins, polyamides, ethyl cellulose, vinyl acetate, ABS resins, polyurethanes, melamine resins, unsaturated polyester resins, alkyd resins, epoxy resins and silicon resins.

The thickness of each layer in the light emitting device of the present invention varies depending on the kind of a material and the layer constitution, and preferably is several nm to 1 µm.

The application of the light emitting device of the present invention includes surface light sources, illuminating systems, signs, back lights, displays, printer heads and the like. For the above-described display, known driving technologies, driving circuits and the like can be used and constitutions such as a segment type, a dot matrix type and the like can be selected.

### EXAMPLES

Examples for illustrating the present invention in more detail are shown below, but the present invention is not limited to them.

The polystyrene-equivalent number-average molecular weight and weight-average molecular weight of a polymer compound were measured by size exclusion chromatography (SEC) (manufactured by Shimadzu Corp., trade name: LC-10Avp). The measurement conditions of the SEC are as described in the following [Measurement condition 1] or [Measurement condition 2].

### [Measurement condition 1]

A polymer compound to be measured was dissolved in tetrahydrofuran at a concentration of about 0.05 wt%, and 50 uL of the solution was injected into SEC. Tetrahydrofuran was used as the mobile phase of SEC, and flowed at a flow rate of 0.6 mL/min. As the column, two columns of TSKgel SuperHM-H (manufactured by Tosoh Corp.) and one column of TSKgel SuperH2000 (manufactured by Tosoh Corp.) were serially connected and used. As the detector, a differential refractive index detector (manufactured by Shimadzu Corp., trade name: RID-10A) was used.

### [Measurement condition 2]

A polymer compound to be measured was dissolved in tetrahydrofuran at a concentration of about 0.05 wt%, and 10 µL of the solution was injected into SEC. Tetrahydrofuran was used as the mobile phase of SEC, and flowed at a flow rate of 2.0 mL/min. As the column, PLgel MIXED-B (manufactured by Polymer Laboratories Ltd.) was used. As the detector, an UV-VIS detector (manufactured by Shimadzu Corp., trade name: SPD-10Avp) was used.

Measurement of LC-MS was carried out according to the following method. A measurement sample was dissolved in chloroform or tetrahydrofuran at a concentration of about 2 mg/mL, and about 1 µL of the solution was injected into LC-MS (manufactured by Agilent Technologies, trade name: 1100LCMSD). As the mobile phase of LC-MS, acetonitrile and tetrahydrofuran were used while changing the ratio thereof and flowed at a flow rate of 0.2 mL/min, unless otherwise stated. As the column, L-column 2 ODS (3 µm) (manufactured by Chemicals Evaluation and Research Institute, Japan, internal diameter: 2.1 mm, length: 100 mm, particle size: 3 µm) was used.

Measurement of NMR was carried out according to the following method. A measurement sample (5 to 10 mg) was dissolved in about 0.5 mL of deuterated chloroform or deuterated tetrahydrofuran, and NMR thereof was measured using (MERCURY 300 (trade name), manufactured by Varian, or AVANCE600 (trade name) TCI cryoprobe, manufactured by Bruker).

Measurement of MALDI-TOFMS was carried out according to the following [Measurement method 1] or [Measurement method 2].

### [Measurement method 1]

α-cyano-4-hydroxycinnamic acid was dissolved in methanol to prepare a saturated solution as a matrix solution. To about 4 mg of a polymer compound to be measured was added 200 µL of chloroform thereby dissolving the compound, and 20 µL of this solution was diluted with 200 µL of chloroform, to obtain a sample solution. The matrix solution (20 µL) and the sample solution (20 µL) were mixed, this mixture was applied on a MALDI plate, and measurement of MALDI-TOFMS was carried out. The measurement was carried out using a MALDI-TOFMS apparatus (Voyager-DE STR (manufactured by Applied Biosystems)) under measurement mode of Reflector, at an acceleration voltage of 20 kV, with a laser (N₂ (337 nm)).

### [Measurement method 2]

To about 4 mg of 1,1,4,4-tetraphenyl-1,3-butadiene was added 400 µL of tetrahydrofuran thereby dissolving the compound, to prepare a matrix solution. To about 0.1 mg of a polymer compound to be measured was added 200 µL of tetrahydrofuran thereby dissolving the compound, to prepare a sample solution. The matrix solution (50 µL) and the sample solution (10 µL) were mixed, this mixture was applied on a MALDIplate, and measurement of MALDI-TOFMS was carried out. The measurement was carried out using a MALDI-TOFMS apparatus (REFLEX III (manufactured by Bruker)) under measurement mode of Reflectron, at an acceleration voltage of 27.5 kV, with a laser (N₂ (337 nm)).

### <Synthesis Example 1> (Synthesis of polymer compound P-1)

To a 200 mL separable flask connected to a Dimroth condenser was added 3.18 g (6.0 mmol) of 9,9-dioctylfluorene-2,7-diboric acid ethylene glycol ester, 3.06 g (5.4 mmol) of 9,9-dioctyl-2,7-dibromofluorene, 0.44 g (0.6 mmol) of N,N'-bis(4-bromophenyl)-N,N'-bis(2,6-dimethyl-4-tert-butylphenyl)-1,4-phenylenediamine, 0.82 g of methyltrioctylammonium chloride (trade name:Aliquat336, manufactured by Aldrich) and 60 mL of toluene. Under a nitrogen atmosphere, 4.2 mg of bistriphenylphosphinepalladium dichloride was added, and the mixture was heated at 85°C. The resultant solution was heated up to 105°C while dropping 16.3 mL of a 17.5 wt% sodium carbonate aqueous solution into the solution, then, the mixture was stirred for 1.5 hours. Next, 0.74 g of phenylboric acid, and 4.2 mg of bistriphenylphosphinepalladium dichloride and 30 mL of toluene were added, and the mixture was stirred at 105°C for 17 hours. The aqueous layer was removed from the resultant solution, then, 3.65 g of sodium N,N-diethyldithiocarbamate trihydrate and 36 mL of ion-exchanged water, and the mixture was stirred at 85°C for 2 hours. The organic layer was separated from the aqueous layer, then, the organic layer was washed with 80 mL of ion-exchanged water (twice), 80 mL of a 3 wt% acetic acid aqueous solution (twice) and 80 mL of ion-exchanged water (twice) in this order. The washed organic layer was dropped into 930 mL of methanol, to find generation of a precipitate, and the precipitate was filtrated, then, dried to obtain a solid. This solid was dissolved in 190 mL of toluene to prepare a solution, this solution was passed through a silica gel/alumina column through which toluene had been passed previously, the resultant solution was dropped into 930 mL of methanol, to find generation of a precipitate, and the precipitate was filtrated, then, dried, to obtain a polymer compound P-1 (4.17 g) having repeating units represented by the following formulae at the following molar ratio. The polymer compound P-1 had a polystyrene-equivalent number-average molecular weight Mn of 2.7×10⁵ and a polystyrene-equivalent weight-average molecular weight Mw of 7.1×10⁵, measured under [Measurement condition 1].

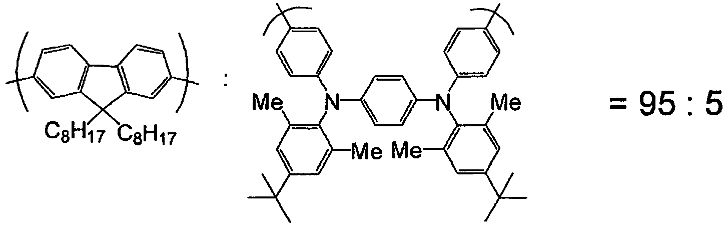

### <Example 1> (Synthesis and evaluation of metal complex MC-1)

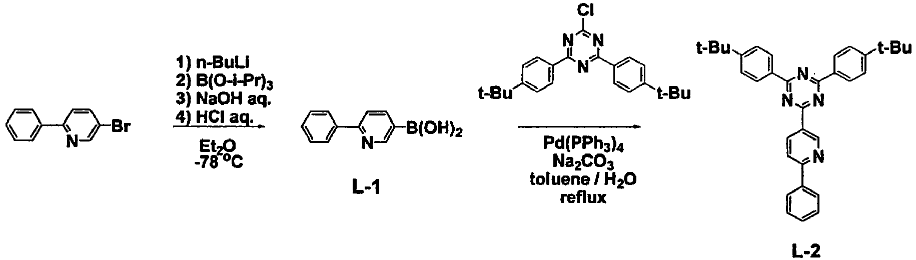

First, 5-bromo-2-phenylpyridine and 4,6-bis(4-tert-butylphenyl)-2-chloro-1,3,5-triazine was synthesized according to a method described in JP-A No. 2008-179617.

Into a reaction vessel, 5-bromo-2-phenylpyridine (103.0 g, 440 mmol) and 1320 mL of dehydrated diethyl ether were measured and charged under nitrogen flow, and the mixture was cooled down to -67°C. Into this was dropped a n-butyllithium/hexane solution (1.59 M, 318.2 mL, 506 mmol) over a period of 20 minutes. After completion of dropping, the resultant solution was stirred at -67°C for 1.5 hours, then, triisopropyl borate (95.2 g, 506 mmol) was added, and the mixture was stirred at -67°C for 4 hours before gradually heating up to room temperature, and the mixture was stirred overnight. To the reaction solution was added 440 mL of a 1N sodium hydroxide aqueous solution and 500 mL of distilled water and the mixture was stirred at room temperature for 30 minutes. The aqueous layer was collected from the reaction solution by a liquid separation operation, and to this was added about 400 mL of 3N hydrochloric acid to regulate pH to 5, generating a sticky precipitate. From the reaction solution, the supernatant was removed by decantation, and this precipitate was washed with 200 mL of distilled water twice, then, dissolved in 800 mL of methanol to obtain a methanol solution. The supernatant was extracted with 1000 mL of ethyl acetate twice, dried over anhydrous magnesium sulfate, then, combined with this methanol solution and concentrated under reduced pressure. To the resultant residue was added ethyl acetate, and water was azeotropically removed, to obtain a compound L-1 (82.9 g) as a pale gray powder.

Into a reaction vessel, 4,6-bis(4-tert-butylphenyl)-2-chloro-1,3,5-triazine (137.1 g, 361 mmol), the compound L-1 (82.6 g, 415 mmol), toluene (2890 mL) and tetrakis(triphenylphosphine)palladium(0) (8.34 g, 7.22 mmol) were measured and charged, and the solid components were dissolved while stirring at 50°C under nitrogen flow. To the resultant solution was added a 2M sodium carbonate aqueous solution (722 mL), and the mixture was refluxed for 17 hours. The organic layer was collected from the reaction solution, and washed with 1000 mL of a 5 wt% sodium hydrogen carbonate aqueous solution and 100 mL of 10 wt% brine. The washed organic layer was dried over sodium sulfate, and concentrated to about 400 mL. The resultant concentrated liquid was purified by silica gel column chromatography (toluene), and the solvent was distilled off. The resultant residue was dissolved in 350 mL of chloroform, and ethanol (1400 mL) was added to cause crystallization. The crystal was collected by filtration, then, the crystal was washed with 500 mL of ethanol, and dried, to obtain a compound L-2 (169.2 g).

### (Compound L-2)

### LC-MS (APPI, positive) m/z: 499 ([M+H]⁺)

In measurement of LC-MS for the present compound, ion-exchanged water containing about 0.1 wt% of acetic acid added and acetonitrile containing about 0.1 wt% of acetic acid added were used while changing the ratio thereof as the mobile phase of LC-MS, and flowed at a flow rate of 0.2 mL/min.
¹H NMR(300MHz, CDCl₃)
δ 1.42 (s, 18 H), 7.52 (m, 3 H), 7.62 (d, J = 6.8 Hz, 4 H), 7.95 (d, J = 8.4 Hz, 1 H), 8.16 (d, J = 7.3 Hz, 2 H), 8.69 (d, J = 6.8 Hz, 4 H), 9.04 (d, J = 8.4 Hz, 1 H), 10. 02 (s, 1 H).

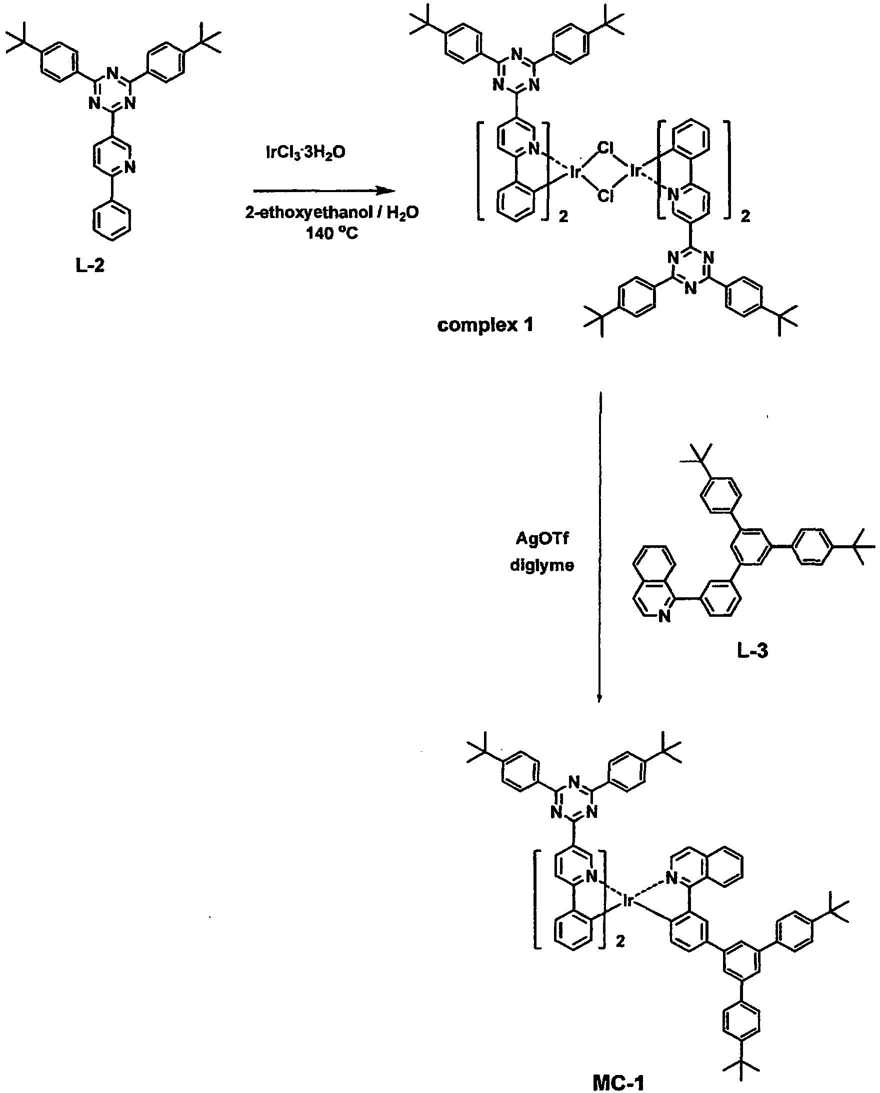

Into a reaction vessel, the compound L-2 (22.17 g, 44 mmol), iridium chloride trihydrate (6.95 g, 20 mmol), 2-ethoxyethanol (96 mL) and water (32 mL) were measured and charged, and heated at 140°C for 15 hours under argon flow. After air cooling, the resultant mixture was filtrated, and the residue was washed with methanol (150 mL), water (100 mL) and methanol (150 mL) in this order, to obtain a red solid. This red solid was dissolved in chloroform (200 mL), and ethanol (300 mL) was added and the mixture was refluxed for 2 hours. After air cooling, the deposited solid was collected by filtration, and washed with ethanol. This operation was repeated three times, then, the resultant solids were collected, and dried under reduced pressure, to obtain a metal complex, complex 1, (20.03 g).

Into a reaction vessel, the metal complex, complex 1, (759 mg, 0.30 mmol), a compound L-3 (330 mg, 0.61 mmol) synthesized according to a method described in WO 2006/062226 pamphlet and diglyme (9 mL) were measured and charged, and silver trifluoromethanesulfoante (157 mg, 0.61 mmol) was added, and the mixture was stirred at 100°C for 10 hours under argon flow. After air cooling, to the reaction mixture was added pure water (50 mL), and the generated precipitate was filtrated. To this precipitate was added a toluene/hexane (1/2 (by volume)) mixed solvent (40 mL), and the mixture was filtrated. The filtrate was dried over sodium sulfate. This solution was filtrated, purified by silica gel column chromatography (hexane/toluene = 1/1.5 (by volume)), and the solvent was distilled off. The resultant residue was washed with methanol (50 mL), and dried under reduced pressure, to obtain a metal complex MC-1 (252 mg, 0.15 mmol).

### (Metal complex MC-1)

LC-MS (APCI, positive) m/z: 1733 ([M+H]⁺)
• ¹H NMR (600MHz, THF-d8)
δ 1.22 (s, 18 H), 1.35 (s, 18 H), 1.38 (s, 18 H), 6.81 (m, 1 H), 6.82 (m, 1 H), 6.86 (m, 1 H), 6.90 (m, 1 H), 6.96 (d, J = 7.1 Hz, 1 H), 7.41 (d, J = 7.1 Hz, 1 H), 7.22 (d, J = 8.2 Hz, 1 H), 7.24 (d, J = 8.2 Hz, 1 H), 7.47 (d, J = 8.2 Hz, 4 H), 7.48 (d, J = 8.5 Hz, 4 H), 7.50 (d, J = 8.2 Hz, 4 H), 7.66 (m, 1 H), 7.66 (d, J = 8.2 Hz, 4 H), 7.71 (m, 2 H), 7.74 (s, 1 H), 7.84 (s, 2 H), 7.89 (d, J = 7.9 Hz, 1 H), 7.93 (d, J = 7.9 Hz, 1 H), 8.03 (d, J = 6.4 Hz, 1 H), 8.06 (m, 1 H), 8.29 (d, J = 8.8 Hz, 1 H), 8.38 (d, J = 8.5 Hz, 4 H), 8.41 (d, J = 8.8 Hz, 1 H), 8.43 (d, J = 8.2 Hz, 4 H), 8.67 (s, 1H), 8.99 (d, J = 8.8 Hz, 1 H), 9.21 (m, 1 H), 9.23 (d, J = 8.8 Hz, 1 H), 9.28 (s, 1 H), 9.44 (s,1 H).

### • EL light emission properties of metal complex MC-1

A 1.5 wt% xylene solution (hereinafter, referred to as "composition A") of a mixture of the polymer compound P-1 (92.5 parts by weight) and the metal complex MC-1 (7.5 parts by weight), and a 0.5 wt% xylene solution (hereinafter, referred to as "composition B") of a polymer compound P-5 composed of a repeating unit represented by the following formula: 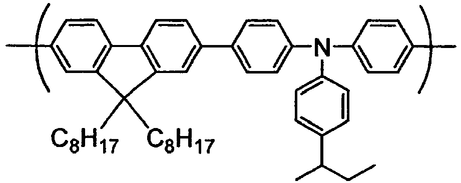 and having a polystyrene-equivalent weight-average molecular weight of 2.7×10⁵ and a polystyrene-equivalent number-average molecular weight of 7.9×10⁴ were prepared.

On a glass substrate carrying thereon an ITO film having a thickness of 150 nm formed by a sputtering method, a solution of poly(ethylenedioxythiophene)/polystyrenesulfonic acid (manufactured by Beyer, trade name: Baytron P) was spin-coated to form a film having a thickness of 65 nm, and dried on a hot plate at 200°C for 10 minutes.

Next, the composition B was spin-coated at a rotation speed of 2000 rpm to form a film which was then dried under a nitrogen gas atmosphere at 180°C for 60 minutes. The temperature of this substrate was returned to room temperature, then, the composition A was spin-coated at a rotation speed of 2050 rpm to form a film. The resultant film had an average thickness of about 80 nm. This was dried at 130°C for 10 minutes under a nitrogen gas atmosphere, then, as a cathode, barium was vapor deposited with a thickness of about 5 nm, then, aluminum was vapor deposited with a thickness of about 80 nm, fabricating an organic EL device. After the degree of vacuum reached 1×10⁻⁴ Pa or less, vapor deposition of a metal was initiated.

By applying voltage on the resultant organic EL device, red EL light emission showing a peak at 615 nm was obtained. This organic EL device showed light emission of 1000 cd/m² under a voltage of about 11.7 V and had maximum light emission efficiency of 7.38 cd/A.

### • PL light emission properties of metal complex MC-1

A 1.0 wt% toluene solution (hereinafter, referred to as "composition C") of a mixture of the polymer compound P-1 (92.5 parts by weight) and the metal complex MC-1 (7.5 parts by weight) was prepared. This composition C was stored under a fluorescent lamp pasted with an ultraviolet prevention film (trade name: Seiden (registered trademark) Crystal, item number: 1-9112-05) in a glove box (manufactured by MBRAUN, item number: UNIlab-2000). Directly after, two days after and nine days after preparation of the composition C, the composition was spin-coated at a rotation speed of 2000 rpm to form a film on a quartz glass, obtaining a film of the composition C.

The photoluminescence spectrum of the resultant film was measured using a spectrofluorometer (manufactured by JASCO Corporation, trade name: JASCO FP-6500 spectrofluorometer), to find red light emission showing a peak at 610 nm. The peak intensity of light emission of the film fabricated using, the composition C directly after preparation was standardized to 1, and the relative values of peak intensities of light emission of the films fabricated using the composition C after two days and after nine days were calculated, to obtain a value of 0.96 and a value of 0.95, respectively.

### <Example 2> (Synthesis and evaluation of metal complex MC-2)

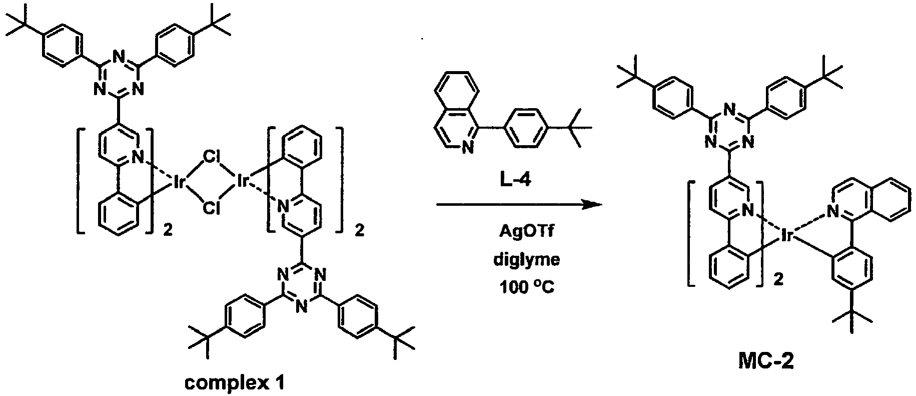

Into a reaction vessel, the metal complex, complex 1, (760 mg, 0.30 mmol), a compound L-4 (330 mg, 0.61 mmol) synthesized according to a method described in WO 2002/044189 pamphlet and diglyme (9 mL) were measured and charged, and silver trifluoromethanesulfonate (154 mg, 0.60 mmol) was added, and the mixture was stirred at 100°C for 20 hours under argon flow. After air cooling, to the reaction mixture was added pure water (50 mL), and the generated precipitate was filtrated. This precipitate was dissolved in a chloroform/hexane (1/3 (by volume)) mixed solvent (50 mL), and dried over sodium sulfate. The resultant solution was filtrated, purified by silica gel column chromatography (hexane/chloroform = 1/1.5 (by volume)), and the solvent was distilled off. The resultant residue was washed with methanol (50 mL), and dried under reduced pressure, to obtain a metal complex MC-2 (234 mg, 0.16 mmol).

### (Metal complex MC-2)

LC-MS (APCI, positive) m/z: 1448 ([M+H]⁺)
¹H NMR (300 MHz, CDCl₃)
δ 1.13 (s, 9 H), 1.22 (s, 18 H), 1.38 (s, 18 H), 6.85-7.04 (m, 7 H), 7.10 (s, 1 H), 7.40 (m, 9 H), 7.60 (m, 2 H), 7.83 (m, 4 H), 8.09 (d, 1 H), 8.18 (t, 2 H), 8.25 (d, 4 H), 8.35 (d, 4 H), 8.90 (d, 1 H), 9.01 (d, 1 H), 9.09 (d, 1 H), 9.15 (s, 1 H), 9.23 (s, 1 H).

### • PL light emission properties of metal complex MC-2

A 1.0 wt% toluene solution (hereinafter, referred to as "composition D") of a mixture of the polymer compound P-1 (92.5 parts by weight) and the metal complex MC-2 (7.5 parts by weight) was prepared. A film was fabricated in the same manner as in Example 1 excepting that the composition D was used instead of the composition C in the section of "PL light emission properties of metal complex MC-1" of Example 1. The photoluminescence spectrum of the resultant film was measured, to find red light emission showing a peak at 607 nm. The peak intensity of light emission of the film fabricated using the composition D directly after preparation was standardized to 1, and the relative values of peak intensities of light emission of the films fabricated using the composition D after two days and after nine days were calculated, to obtain a value of 0.93 and a value of 0.95, respectively.

### <Comparative Example 1> (Synthesis and evaluation of metal complex M-1)

### • PL light emission properties of metal complex M-1

A 1.0 wt% toluene solution (hereinafter, referred to as "composition E") of a mixture of the polymer compound P-1 (92.5 parts by weight) and a metal complex M-1 (7.5 parts by weight) represented by the following formula: 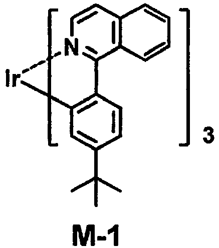 synthesized according to a method described in WO 2002/44189 pamphlet was prepared. A film was fabricated in the same manner as in Example 1 excepting that the composition E was used instead of the composition C in the section of "PL light emission properties of metal complex MC-1" of Example 1. The photoluminescence spectrum of the resultant film was measured, to find red light emission showing a peak at 619 nm. The peak intensity of light emission of the film fabricated using the composition E directly after preparation was standardized to 1, and the relative values of peak intensities of light emission of the films fabricated using the composition E after two days and after nine days were calculated, to obtain a value of 0.91 and a value of 0.47, respectively.

### <Example 3> (Another synthesis of metal complex MC-1)

### • Synthesis of metal complex MC-3

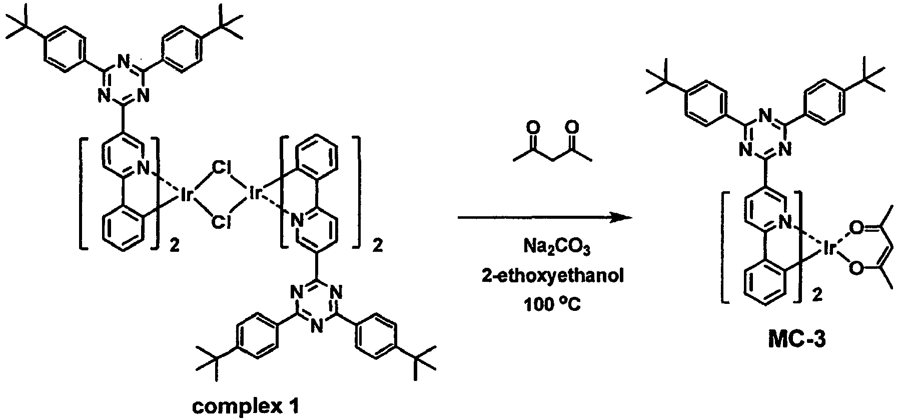

Into a reaction vessel, the metal complex, complex 1, (7.34 g, 3.0 mmol), acetylacetone (1.52 g, 15 mmol), sodium carbonate (3.18 g, 30 mmol) and 2-ethoxyethanol (73 mL) were measured and charged, and the mixture was stirred at 100°C for 6 hours under argon flow. After air cooling, the reaction mixture was filtrated, and the resultant solid was washed with methanol. The washed solid was dissolved in a chloroform/hexane (1/1 (by volume)) mixed solvent(350 mL), purified by silica gel column chromatography (hexane/chloroform = 1/1 (by volume)), and the solvent was distilled off. The resultant residue was dissolved in a chloroform/toluene (1/1 (by volume)) mixed solvent (600 mL), purified by silica gel column chromatography (chloroform/toluene = 1/1 (by volume)) again, and the solution was concentrated to about 50 mL. The generated precipitate was filtrated, the resultant solid was washed with methanol, and dried under reduced pressure, to obtain a metal complex MC-3. The filtrate was concentrated to about 30 mL, the generated precipitate was filtrated, the resultant solid was washed with methanol and dried under reduced pressure, to obtain a metal complex MC-3. The total amount of the resultant metal complex MC-3 was 2.73 g (2.1 mmol).

### (Metal complex MC-3)

MALDI-TOFMS (positive, [Measurement method 2]) m/z: 1287 ([M])
¹H NMR (300 MHz, CDCl₃)
δ 1.39 (s, 36 H), 2.02 (s, 6 H), 5.38 (s, 1 H), 6.45 (d, J = 7.8 Hz, 2 H), 6.74 (m, 2 H), 6.87 (m, 2 H), 7.57 (d, J = 7.5 Hz, 8 H), 7.70 (d, J = 7.7 Hz, 2 H), 8.06 (d, J = 8.4 Hz, 1 H), 8.68 (d, J = 7.5 Hz, 8 H ), 9.05 (d, J = 8.4 Hz, 2 H), 9.93 (s, 2 H).

### • Synthesis of metal complex MC-1

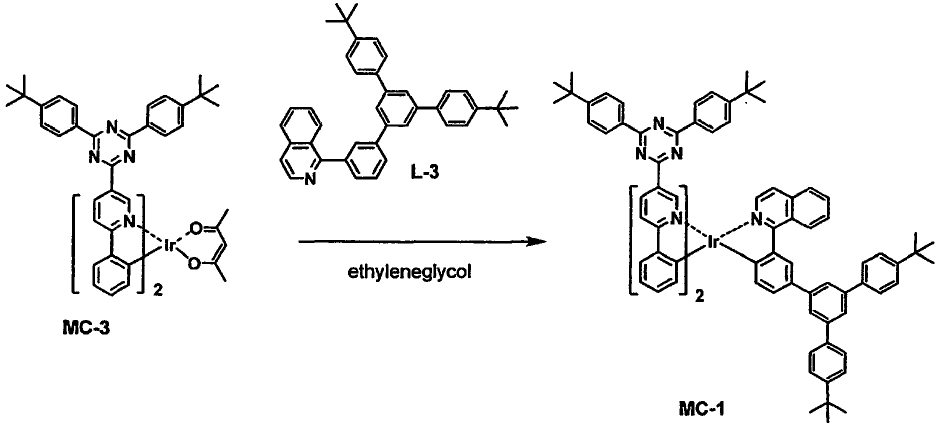

Into a reaction vessel, the metal complex MC-3 (387 mg, 0.30 mmol), the compound L-3 (165 mg, 0.30 mmol) and ethylene glycol (15 mL) were measured and charged under argon flow, and the mixture was stirred with heating at 180°C for 25 hours. After air cooling, the resultant reaction solution was filtrated, and the residue was washed with water (10 mL) and methanol (50 mL) in this order. To the washed residue was added a toluene/hexane mixed solvent (30 mL) to cause dissolution thereof. The resultant solution was purified by silica gel chromatography (developing solvent: toluene/hexane mixed solvent), and the solvent was distilled off. The resultant residue was washed with methanol, to obtain a metal complex MC-1 (91 mg, 0.05 mmol) represented by the above-described formula.

### <Example 4> (Synthesis of metal complex MC-5)

### • Synthesis of metal complex MC-4

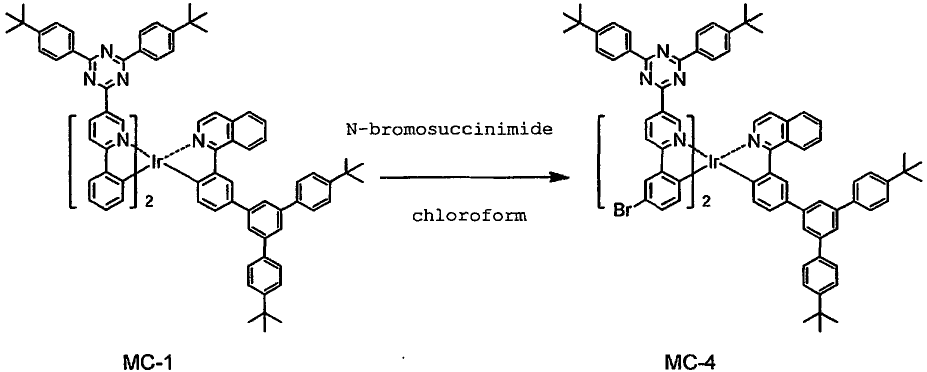

Into a reaction vessel, the metal complex MC-1 (4.25 g, 2.5 mmol) and chloroform (400 mL) were measured and charged under argon flow, and the metal complex was allowed to dissolve. To this was added N-bromosuccinimide (872 mg, 4.9 mmol), and the mixture was stirred at room temperature for 24 hours. The solvent was distilled off, and to the residue was added a chloroform/hexane mixed solvent (100 mL) to cause dissolution thereof. The resultant solution was purified by silica gel chromatography (developing solvent: chloroform/hexane mixed solvent). The eluted solution was collected, and the solvent was distilled off, then, the residue was washed with methanol, to obtain a metal complex (MC-4, 3.76 g, 2.0 mmol) represented by the above-described formula.

### (Metal complex MC-4)

MALDI-TOFMS (positive, [Measurement method 1]) m/z: 1890
([M])
¹H NMR (300 MHz, THF-d₈)
δ 1.27 (s, 18 H), 1.36 (s, 18 H), 1.41 (s, 18 H), 6.95 (m, 4 H), 7.24 (m, 2 H), 7.48 (m, 12 H), 7.69 (m, 5 H), 7.74 (m, 3 H), 7.83 (s, 2 H), 7.99 (d, J = 6.0 Hz, 1 H), 8.09 (m, 3 H ), 8.40 (m, 9 H ), 8.54 (d, J = 8.6 Hz, 1 H), 8.68 (s, 1 H ), 9.05 (m, 1 H ), 9.22 (m, 2 H ), 9.28 (d, J = 8.6 Hz, 1 H), 9.46 (s, 1 H).

### • Synthesis of metal complex MC-5

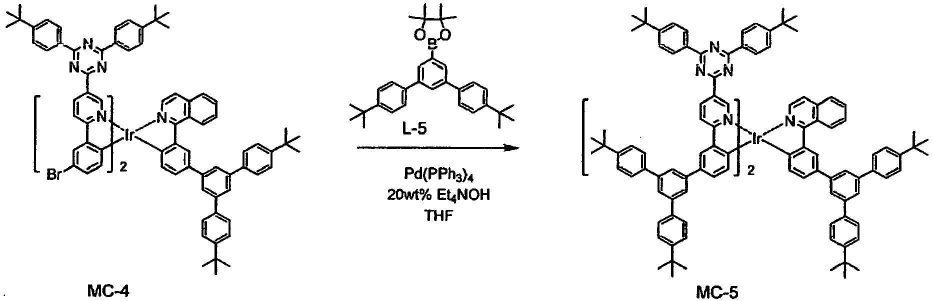

Into a reaction vessel, the metal complex MC-4 (2.84 g, 1.5 mmol), the compound L-5 (1.56 g, 3.3 mmol), a 20 wt% aqueous solution of tetraethylammonium hydroxide (5.42 g, 7.4 mmol), tetrakis(triphenylphosphine)palladium(0) (61 mg, 0.05 mmol) and tetrahydrofuran (90 mL) were measured and charged under argon flow, and the mixture was refluxed for 14 hours. To the reaction solution was added toluene (60 mL) and water (100 mL) and washing thereof was performed, and the organic layer was collected. This the organic layer was washed with saturated brine (60 mL), and dried over sodium sulfate. The resultant solution was filtrated, and concentrated to about 50 mL. The resultant concentrated liquid was purified by silica gel chromatography (developing solvent: toluene). The eluted solution was collected, the solvent was distilled off, and to the resultant residue was added a chloroform/hexane mixed solvent (50 mL) to cause dissolution thereof. The resultant solution was purified by silica gel chromatography (developing solvent: chloroform/hexane), and the solvent was distilled off. The residue was dissolved in toluene, and acetonitrile was added to the resultant solution to cause crystallization, thereby performing purification. The resultant solid was filtrated and collected, to obtain a metal complex MC-5 (2.85 g, 1.2 mmol) represented by the above-described formula.

The compound L-5 was synthesized according to a method described in WO 02/62226.

### (Metal complex MC-5)

MALDI-TOFMS (positive, [Measurement method 1]) m/z: 2413
([M])
¹H NMR (600 MHz, THF-d₈)
δ1.26 (s, 18 H), 1.37 (s, 18 H), 1.38 (s, 18 H), 1.40 (s, 18 H), 1.42 (s, 18 H), 7.26 (d, J = 8.1 Hz, 1 H), 7.31 (d, J = 8.1 Hz, 1 H), 7.33 (d, J = 8.1 Hz, 2 H), 7.35 (d, J = 8.1 Hz, 1 H), 7.39 (d, J = 8.1 Hz, 1 H), 7.48 (d, J = 8.4 Hz, 4 H), 7.49 (d, J = 8.3 Hz, 4 H), 7.50 (d, J = 8.1 Hz, 4 H), 7.51 (d, J = 8.1 Hz, 4 H), 7.52 (d, J = 8.5 Hz, 4 H), 7.68 (d, J = 8.4 Hz, 4 H), 7.69 (d, J = 8.5 Hz, 4 H), 7.72 (d, J = 8.3 Hz, 4 H), 7.74 (m, 4 H), 7.75 (s, 1 H), 7.78 (s, 1 H), 7.88 (s, 2 H), 7.91 (s, 2 H), 7.92 (s, 2 H), 8.10 (m, 1 H), 8.11 (d, J = 6.2 Hz, 1 H), 8.35 (s, 1 H), 8.39 (m, 5 H), 8.44 (d, J = 8.1 Hz, 4 H), 8.56 (d, J = 8.8 Hz, 1 H), 8.69 (d, J = 8.8 Hz, 1 H), 8.73 (s, 1 H), 9.03 (d, J = 8.8 Hz, 1 H), 9.26 (m, 1 H), 9.27 (d, J = 8.8 Hz, 1 H), 9.38 (s, 1 H), 9.51 (s, 1 H).

### <Example 5> (PL light emission properties of metal complex MC-1, 2-nd)

A 2.0 wt% xylene solution (hereinafter, referred to as "composition F") of a mixture of polystyrene available from Aldrich (standard for GPC, average Mw = 565500) (92.5 parts by weight) and the metal complex MC-1 (7.5 parts by weight) was prepared. A film was fabricated in the same manner as in Example 1 excepting that the composition F was used instead of the composition C in the section of "PL light emission properties of metal complex MC-1" of Example 1. The photoluminescence spectrum of the resultant film was measured, to find red light emission showing a peak at 610 nm. The peak intensity of light emission of the film fabricated using the composition F directly after preparation was standardized to 1, and the relative value of peak intensity of light emission of the film fabricated using the composition F after two days was calculated, to obtain a value of 1.10.

### <Example 6> (PL light emission properties of metal complex MC-2, 2-nd)

A 2.0 wt% xylene solution (hereinafter, referred to as "composition G") of a mixture of polystyrene available from Aldrich (standard for GPC, average Mw = 565500) (92.5 parts by weight) and the metal complex MC-2 (7.5 parts by weight) was prepared. A film was fabricated in the same manner as in Example 1 excepting that the composition G was used instead of the composition C in the section of "PL light emission properties of metal complex MC-1" of Example 1. The photoluminescence spectrum of the resultant film was measured, to find red light emission showing a peak at 609 nm. The peak intensity of light emission of the film fabricated using the composition G directly after preparation was standardized to 1, and the relative value of peak intensity of light emission of the film fabricated using the composition G after two days was calculated, to obtain a value of 1.05.

### <Example 7> (PL light emission properties of metal complex MC-5)

A 2.0 wt% xylene solution (hereinafter, referred to as "composition H") of a mixture of polystyrene available from Aldrich (standard for GPC, average Mw = 565500) (92.5 parts by weight) and the metal complex MC-5 (7.5 parts by weight) was prepared. A film was fabricated in the same manner as in Example 1 excepting that the composition H was used instead of the composition C in the section of "PL light emission properties of metal complex MC-1" of Example 1. The photoluminescence spectrum of the resultant film was measured, to find red light emission showing a peak at 610 nm. The peak intensity of light emission of the film fabricated using the composition H directly after preparation was standardized to 1, and the relative value of peak intensity of light emission of the film fabricated using the composition H after two days was calculated, to obtain a value of 0.99.

### <Comparative Example 2> (PL light emission properties of metal complex M-1, 2-nd)

A 2.0 wt% xylene solution (hereinafter, referred to as "composition I") of a mixture of polystyrene available from Aldrich (standard for GPC, average Mw = 565500) (92.5 parts by weight) and the metal complex M-1 (7.5 parts by weight) was prepared. A film was fabricated in the same manner as in Example 1 excepting that the composition I was used instead of the composition C in the section of "PL light emission properties of metal complex MC-1" of Example 1. The photoluminescence spectrum of the resultant film was measured, to find red light emission showing a peak at 620 nm. The peak intensity of light emission of the film fabricated using the composition I directly after preparation was standardized to 1, and the relative value of peak intensity of light emission of the film fabricated using the composition I after two days was calculated, to obtain a value of 0.76.

### <Example 8> (Synthesis of metal complex MC-7)

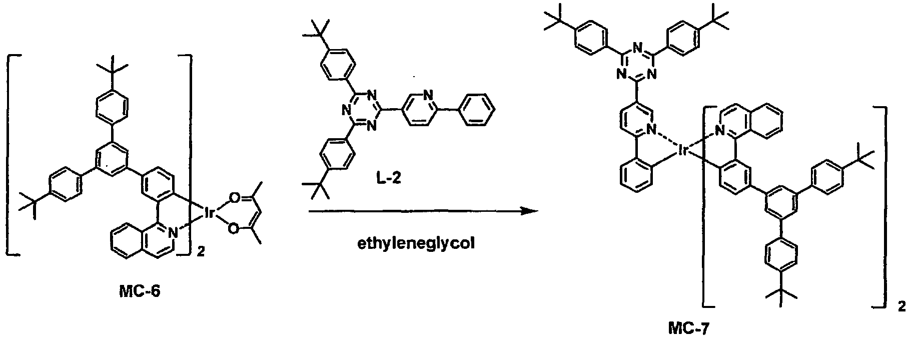

Into a reaction vessel, the metal complex MC-6 (2.61 g, 1.9 mmol), the compound L-2 (998 mg, 2.0 mmol) and ethylene glycol (100 mL) were measured and charged under argon flow, and the mixture was refluxed for 11 hours. After air cooling, the reaction solution was filtrated, and the residue was washed with water (200 mL) and methanol (50 mL) in this order. To the resultant residue was added a chloroform/hexane mixed solvent to cause dissolution thereof. The resultant solution was purified by silica gel chromatography (developing solvent: chloroform/hexane), and the solvent was distilled off. Silica gel column chromatography was further repeated three times, and the solvent was distilled off. The resultant residue was washed with methanol, to obtain a metal complex MC-7 (65 mg, 0.036 mmol) represented by the above-described formula.

The metal complex MC-6 was synthesized according to a method described in JP-A No. 2008-174499.

### (Metal complex MC-7)

MALDI-TOFMS (positive, [Measurement method 2]) m/z: 1779
([M])
¹H NMR (300 MHz, THF-d₈) δ 1.33 (s, 18 H), 1.35 (s, 18 H), 1.39 (s, 18 H), 6.79 (t, J = 7.5 Hz, 1 H), 6.88 (t, J = 7.5 Hz, 1 H), 7.01 (d, J = 7.5 Hz, 1 H), 7.16-7.33 (m, 4 H), 7.39-7.55 (m, 14 H), 7.62-7.76 (m, 16 H), 7.82 (s, 4 H), 7.86-8.01 (m, 3 H), 8.32 (m, 5 H), 8.58 (s, 1 H), 8.65 (s, 1 H), 8.97 (d, J = 8.6 Hz, 1 H), 9.11 (d, J = 8.1 Hz, 1 H), 9.21 (m, 1 H), 9.26 (s, 1 H).

### <Example 9> (Synthesis of polymer compound PC-1)

Into a 100 mL four-necked flask were added 1.33 g (2.1 mmol) of 2,7-bis(4,4,5,5-tetramethyl-1,3,2-dioxaborolan-2-yl)-9,9-dioctylfluorene, 1.08 g (2.0 mmol) of 9,9-dioctyl-2,7-dibromofluorene, 0.14 g (0.26 mmol) of 2,4-bis(4-bromophenyl)-6-(4-n-hexylphenyl)-1,3,5-triazine, 0.19 g (0.26 mmol) of N,N'-bis(4-bromophenyl)-N,N'-bis(2,6-dimethyl-4-tert-butylphenyl)-1,4-phenylenediamine, 0.20 g (0.10 mmol) of the metal complex MC-4 and 45 mL of toluene. Under a nitrogen atmosphere, the mixture was heated at 100°C. 8.2 mg of bistriphenylphosphinepalladium dichloride was added. Into the resultant solution, 9.15 g of a 20 wt% tetraethylammonium hydroxide aqueous solution was dropped, then, the mixture was stirred for 14 hours. Next, 0.18 g (0.28 mmol) of 2,7-bis(4,4,5,5-tetramethyl-1,3,2-dioxaborolan-2-yl)-9,9-dioctylfluorene, 10 mL of toluene and 9 mL of ion-exchanged water were added, and the mixture was stirred at 105°C for 3 hours. Next, to this were added 0.13 g of phenylboric acid, 1.8 mg of bistriphenylphosphinepalladium dichloride, 7 mL of toluene and 8 mL of a 20 wt% tetraethylammonium hydroxide aqueous solution, and the mixture was stirred at 105°C for 4 hours.

The aqueous layer was removed from the reaction solution, then, to this were added 0.73 g of sodium N,N-diethyldithiocarbamate trihydrate and 15 mL of ion-exchanged water, and the mixture was stirred at 80°C for 2 hours. The organic layer of the reaction solution was separated from the aqueous layer, then, the organic layer was washed with 40 mL of 3.6 wt% hydrochloric acid (twice), 40 mL of a 2.5 wt% ammonia aqueous solution (twice) and 40 mL of ion-exchanged water (five times) in this order.

The washed organic layer was dropped into 500 mL of methanol to find generation of a precipitate, and the precipitate was filtrated, then, dried, to obtain a solid. This solid was dissolved in 60 mL of toluene, and the solution was passed through a silica gel/alumina column through which toluene had been passed previously. The passed elution solution was dropped into 760 mL of methanol to find generation of a precipitate, the precipitate was filtrated, then, dried, to obtain 1.55 g of a polymer compound (hereinafter, referred to as "polymer compound PC-1") having repeating units represented by the following formulae at the following molar ratio. The polymer compound PC-1 had polystyrene-equivalent number-average molecular weight (Mn) and weight-average molecular weight (Mw), measured under [Measurement condition 2], of Mn = 1.1×10⁴ and Mw = 3.4×10⁴, respectively.

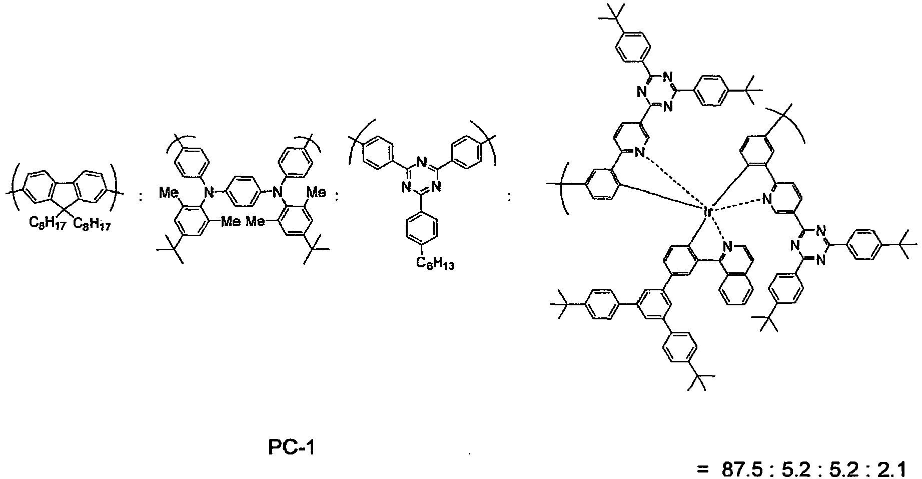

### <Example 10> (Fabrication of organic EL device X)

A 1.6 wt% xylene solution (hereinafter, referred to as "composition J") of a mixture of the polymer compound P-1 (80 parts by weight) and the metal complex MC-1 (20 parts by weight), and, a 0.6 wt% xylene solution (hereinafter, referred to as "composition K") of the polymer compound P-5 were prepared.

On a glass substrate carrying thereon an ITO film having a thickness of 150 nm formed by a sputtering method, a solution of
poly(ethylenedioxythiophene)/polystyrenesulfonic acid (manufactured by H C Starck, trade name: CLEVIOS P AI 4083) was spin-coated to form a film having a thickness of 65 nm, and dried on a hot plate at 200°C for 10 minutes.

Next, the composition K was spin-coated at a rotation speed of 2000 rpm to form a film having a thickness of about 20 nm which was then dried under a nitrogen gas atmosphere at 180°C for 60 minutes. The temperature of thus obtained substrate was returned to room temperature, then, the composition J was spin-coated at a rotation speed of 2180 rpm to form a film having a thickness of about 80 nm. This was dried at 130°C for 10 minutes under a nitrogen gas atmosphere, then, as a cathode, barium was vapor deposited with a thickness of about 5 nm, then, aluminum was vapor deposited with a thickness of about 80 nm, fabricating an organic EL device X. After the degree of vacuum reached 1×10⁻⁴ Pa or less, vapor deposition of a metal was initiated.

By applying voltage on the organic EL device X, red EL light emission showing a peak at 615 nm was obtained. The organic EL device X showed light emission of 1000 cd/m² under a voltage of about 9.3 V and had maximum light emission efficiency of 11.1 cd/A.

### <Comparative Example 3> (Fabrication of organic EL device CX)

A 1.6 wt% xylene solution (hereinafter, referred to as "composition L") of a mixture of the polymer compound P-1 (80 parts by weight) and the metal complex M-1 (20 parts by weight) was prepared. An organic EL device CX was fabricated in the same manner as in Example 10 excepting that the composition L was used instead of the composition J in Example 10. By applying voltage on the organic EL device CX, red EL light emission showing a peak at 620 nm was obtained. The organic EL device CX showed light emission of 1000 cd/m² under a voltage of about 7.7 V and had maximum light emission efficiency of 5.8 cd/A.

### <Synthesis Example 2> (Synthesis of polymer compound P-3)

Into a reaction vessel were added 91.83 g (124 mmol) of 9,9-bis(3-hexylphenyl)-2,7-bis(4,4,5,5-tetramethyl-1,3,2-dioxaborolan-2-yl)fluorene, 17.30 g (31.6 mmol) of 9,9-dioctyl-2,7-dibromofluorene, 51.69 g (75.7 mmol) of N,N-bis(4-bromophenyl)-N',N'-bis(4-butylphenyl)-1,4-phenylenediamine, 10.00 g (18.9 mmol) of 9,9-bis(bicyclo[4.2.0]octa-1,3,5-trien-3-yl)-2,7-dibromofluorene and 2112 g of toluene. Under a nitrogen gas atmosphere, the solution was heated at 90°C, and 88.6 mg of bistriphenylphosphinepalladium dichloride was added. Next, 436.7 g of a 20 wt% tetraethylammonium hydroxide aqueous solution was dropped into this over a period of 60 minutes. The mixture was stirred at 90°C for 5 hours from initiation of dropping of the 20 wt% tetraethylammonium hydroxide aqueous solution, then, 1.54 g of phenylboric acid and 60 g of toluene were additionally added, further, the mixture was stirred for 14 hours. The aqueous layer was removed from the reaction solution, then, to this were added 140.3 g of sodium N,N-diethyldithiocarbamate trihydrate, 1.20 kg of ion-exchanged water and 1.80 kg of toluene, and the mixture was stirred at 40°C for 3 hours. The organic layer of the reaction solution was separated from the aqueous layer, then, the organic layer was washed with 10 wt% hydrochloric acid (twice), a 3 wt% ammonia aqueous solution (twice) and ion-exchanged water (twice) in this order. The washed organic layer was passed through a column filled with silica gel and alumina though which toluene had been passed previously, and the passed solution was dropped into methanol to find deposition of a precipitate. The precipitate was filtrated and dried, to obtain 204 g of a polymer compound (hereinafter, referred to as "polymer compound P-3") having repeating units represented by the following formulae at the following molar ratio. The polymer compound P-3 had polystyrene-equivalent number-average molecular weight and weight-average molecular weight, measured under [Measurement condition 2], of Mn = 6.4×10⁴ and Mw = 2.1×10⁵, respectively.

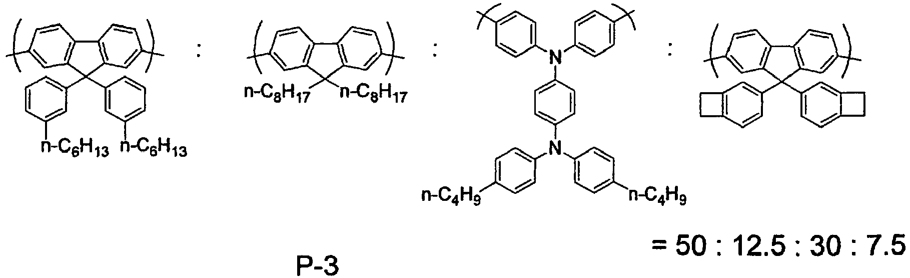

9,9-bis(3-hexylphenyl)-2,7-bis(4,4,5,5-tetramethyl-1,3,2-dioxaborolan-2-yl)fluorene was synthesized according to a method described in WO 2010/013723 pamphlet. N,N-bis(4-bromophenyl)-N',N'-bis(4-butylphenyl)-1,4-phenylenediamine was synthesized according to a method described in JP-A No. 2003-226744. 9,9-bis(bicyclo[4.2.0]octa-1,3,5-trien-3-yl)-2,7-dibromofluorene was synthesized according to a method described in JP-A No. 2008-106241.

### <Synthesis Example 3> (Synthesis of polymer compound P-4)

Into a reaction vessel were added 15.27 g (20.7 mmol) of 9,9-bis(3-hexylphenyl)-2,7-bis(4,4,5,5-tetramethyl-1,3,2-dioxaborolan-2-yl)fluorene, 15.97 g (17.5 mmol) of 2,7-bis(N-(4-bromophenyl)-N-(4-methylphenyl)amino)-9,9-dioctylfluorene, 1.33 g (3.1 mmol) of 9,9-bis(bicyclo[4.2.0]octa-1,3,5-trien-3-yl)-2,7-dibromofluorene and 436 g of toluene. Under a nitrogen gas atmosphere, the solution was heated at 90°C, and 14.4 mg of bistriphenylphosphinepalladium dichloride was added. Next, 71.50 g of a 20 wt% tetraethylammonium hydroxide aqueous solution was dropped into this over a period of 60 minutes.

The mixture was stirred at 90°C for 7 hours from initiation of dropping of the 20 wt% tetraethylammonium hydroxide aqueous solution, then, 0.25 g of phenylboric acid and 12 g of toluene were additionally added, further, the mixture was stirred for 14 hours. Next, 11.46 g of sodium N,N-diethyldithiocarbamate trihydrate, 120 g of ion-exchanged water and 183 g of toluene were added, and the mixutre was stirred at 40°C for 3 hours. The aqueous layer was removed from the reaction solution, then, to this was added 120 g of ion-exchanged water, and the mixture was stirred at 40°C for 0.5 hours. The organic layer of the reaction solution was separated from the aqueous layer, then, the organic layer was washed with 10 wt% hydrochloric acid (twice), a 3 wt% ammonia aqueous solution (twice) and ion-exchanged water (twice) in this order. The washed organic layer was passed through a column filled with silica gel and alumina though which toluene had been passed previously, and the passed solution was dropped into methanol to find deposition of a precipitate. The precipitate was filtrated and dried, to obtain 21.14 g of a polymer compound (hereinafter, referred to as "polymer compound P-4") having repeating units represented by the following formulae at the following molar ratio. The polymer compound P-4 had polystyrene-equivalent number-average molecular weight and weight-average molecular weight, measured under [Measurement condition 2], of Mn = 4.5×10⁴ and Mw = 2.0×10⁵, respectively.

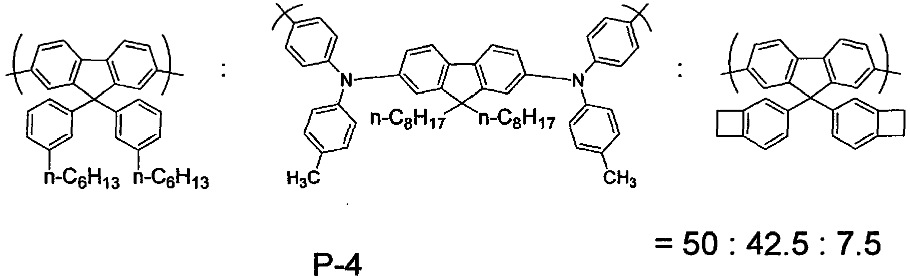

2,7- bis(N-(4-bromophenyl)-N-(4-methylphenyl)amino)-9,9-dioctylfluorene was synthesized according to a method described in Japanese Patent Application National Publication (Laid-Open) No. 2007-512249.

### <Example 11> (Fabrication of organic EL device Y)

A 1.6 wt% xylene solution (hereinafter, referred to as "composition M") of a mixture of a polymer compound P-2 (92.5 parts by weight) having repeating units represented by the following formulae at the following molar ratio and the metal complex MC-1 (7.5 parts by weight), and a 0.6 wt% xylene solution (hereinafter, referred to as "composition N") of the polymer compound P-3 were prepared.

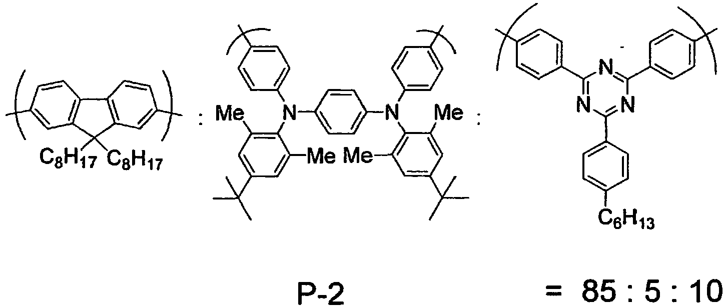

The polymer compound P-2 was synthesized according to a method described in WO 2009/157430 pamphlet. The polymer compound P-2 had polystyrene-equivalent number-average molecular weight (Mn) and weight-average molecular weight (Mw), measured under [Measurement condition 2], of Mn = 1.4×10⁵ and Mw = 3.7×10⁵, respectively.

On a glass substrate carrying thereon an ITO film having a thickness of 150 nm formed by a sputtering method, a solution of
poly(ethylenedioxythiophene)/polystyrenesulfonic acid (manufactured by H C Starck, trade name: CLEVIOS P AI 4083) was spin-coated to form a film having a thickness of 65 nm, and dried on a hot plate at 200°C for 10 minutes.

Next, the composition N was spin-coated at a rotation speed of 1200 rpm to form a film having a thickness of about 20 nm which was then dried under a nitrogen gas atmosphere at 180°C for 60 minutes. The temperature of this substrate was returned to room temperature, then, the composition M was spin-coated at a rotation speed of 3280 rpm to form a film having a thickness of about 80 nm. This was dried at 130°C for 10 minutes under a nitrogen gas atmosphere, then, as a cathode, barium was vapor deposited with a thickness of about 5 nm, then, aluminum was vapor deposited with a thickness of about 80 nm, fabricating an organic EL device Y. After the degree of vacuum reached 1×10⁻⁴ Pa or less, vapor deposition of a metal was initiated.

By applying voltage on the organic EL device Y, red EL light emission showing a peak at 615 nm was obtained. The organic EL device Y showed light emission of 1000 cd/m² under a voltage of about 5.2 V and had maximum light emission efficiency of 15.8 cd/A.

### <Comparative Example 4> (Fabrication of organic EL device CY)

A 1.6 wt% xylene solution (hereinafter, referred to as "composition O") of a mixture of the polymer compound P-2 (92.5 parts by weight) and the metal complex M-1 (7.5 parts by weight) was prepared. An organic EL device CY was fabricated in the same manner as in Example 11 excepting that the composition O was used instead of the composition M in Example 11. By applying voltage on the organic EL device CY, red EL light emission showing a peak at 620 nm was obtained. The organic EL device CY showed light emission of 1000 cd/m² under a voltage of about 5.2 V and had maximum light emission efficiency of 11.6 cd/A.

### <Example 12> (Fabrication of organic EL device Z)

A 0.6 wt% xylene solution (hereinafter, referred to as "composition P") of the polymer compound P-4 was prepared.

On a glass substrate carrying thereon an ITO film having a thickness of 150 nm formed by a sputtering method, a solution of
poly(ethylenedioxythiophene)/polystyrenesulfonic acid (manufactured by H C Starck, trade name: CLEVIOS P AI 4083) was spin-coated to form a film having a thickness of 65 nm, and dried on a hot plate at 200°C for 10 minutes.

Next, the composition P was spin-coated at a rotation speed of 1050 rpm to form a film having a thickness of about 20 nm which was then dried under a nitrogen gas atmosphere at 180°C for 60 minutes. The temperature of this substrate was returned to room temperature, then, the composition M was spin-coated at a rotation speed of 3400 rpm to form a film having a thickness of about 80 nm. This was dried at 130°C for 10 minutes under a nitrogen gas atmosphere, then, as a cathode, barium was vapor deposited with a thickness of about 5 nm, then, aluminum was vapor deposited with a thickness of about 80 nm, fabricating an organic EL device Z. After the degree of vacuum reached 1×10⁻⁴ Pa or less, vapor deposition of a metal was initiated.

By applying voltage on the organic EL device Z, red EL light emission showing a peak at 615 nm was obtained. The organic EL device Z showed light emission of 1000 cd/m² under a voltage of about 5.9 V and had maximum light emission efficiency of 15.2 cd/A.

### <Example 13> (Fabrication of organic EL device XX)

A 1.6 wt% xylene solution (hereinafter, referred to as "composition Q") of a mixture of the polymer compound P-2 (92.5 parts by weight) and the metal complex MC-5 (7.5 parts by weight) was prepared. An organic EL device XX was fabricated in the same manner as in Example 12 excepting that the composition Q was used instead of the composition M in Example 12. By applying voltage on the organic EL device XX, red EL light emission showing a peak at 615 nm was obtained. The organic EL device XX showed light emission of 1000 cd/m² under a voltage of about 6.3 V and had maximum light emission efficiency of 17.5 cd/A.

### <Comparative Example 5> (Fabrication of organic EL device CXX)

An organic EL device CXX was fabricated in the same manner as in Example 12 excepting that the composition O was used instead of the composition M in Example 12. By applying voltage on the organic EL device CXX, red EL light emission showing a peak at 620 nm was obtained. The organic EL device CXX showed light emission of 1000 cd/m² under a voltage of about 5.9 V and had maximum light emission efficiency of 11.1 cd/A.

### <Example 14> (Fabrication of organic EL device YY)

A 2.5 wt% xylene solution (hereinafter, referred to as "composition R") of the polymer compound PC-1 was prepared. An organic EL device YY was fabricated in the same manner as in Example 12 excepting that the composition R was used instead of the composition M in Example 12. By applying voltage on the organic EL device YY, red EL light emission showing a peak at 615 nm was obtained. The organic EL device YY showed light emission of 1000 cd/m² under a voltage of about 5.9 V and had maximum light emission efficiency of 17.5 cd/A.

### Industrial Applicability

The metal complex, the metal-containing polymer compound and the composition of the present invention are red light emitting materials excellent in solution stability (that is, red light emitting materials having the peak wavelengths of EL light emission spectra of the metal complex, the metal-containing polymer compound and the composition of the present invention are 570 to 700 nm). An organic EL device using this red light emitting material is excellent in light emission efficiency.

## Claims

1. A metal complex represented by the following formula (1) : 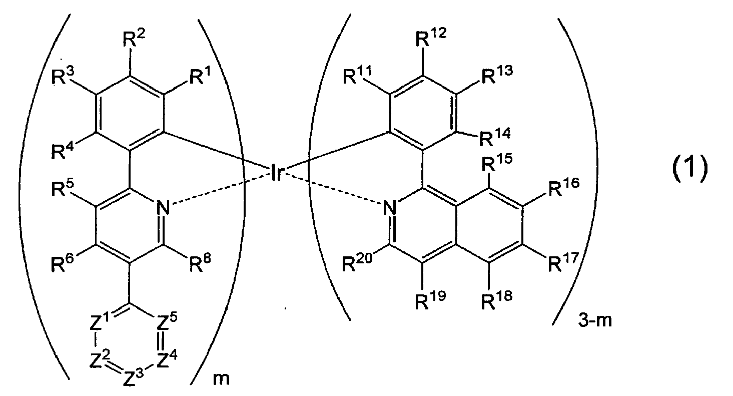
wherein R¹, R², R³, R⁴, R⁵, R⁶, R⁸, R¹¹, R¹², R¹³, R¹⁴, R¹⁵, R¹⁶, R¹⁷, R¹⁸, R¹⁹ and R²⁰ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent;
Z¹, Z², Z³, Z⁴ and Z⁵ each independently represent -C(R*)= or a nitrogen atom, wherein R* represents a hydrogen atom or a substituent;
when there are two or more moieties of each of R¹, R², R³, R⁴, R⁵, R⁶, R⁸, R¹¹, R¹², R¹³, R¹⁴, R¹⁵, R¹⁶,R¹⁷, R¹⁸, R¹⁹, R²⁰, Z¹, Z², Z³, Z⁴ and Z⁵, the two or more moieties may be the same or different, provided that at least two of Z¹, Z², Z³, Z⁴ and Z⁵ are nitrogen atoms;
m represents 1 or 2.

2. The metal complex according to claim 1, represented by the following formula (1c): 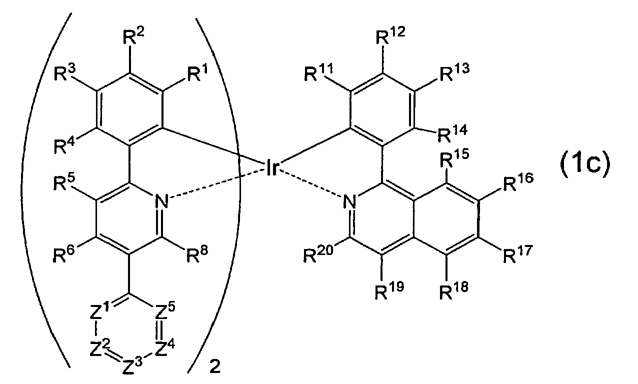 wherein R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above.

3. The metal complex according to claim 2, represented by the following formula (1a) or the following formula (1b) : 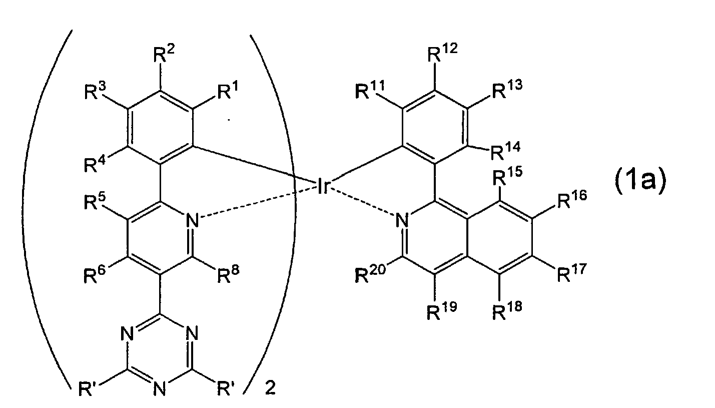 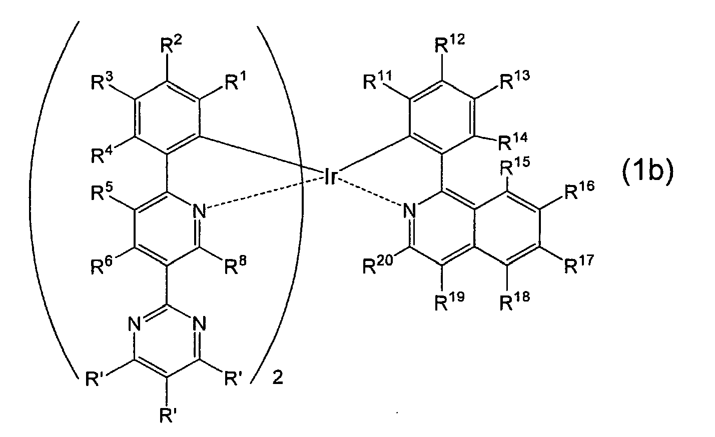
wherein R¹, R², R³, R⁴, R⁵, R⁶, R⁸, R¹¹, R¹², R¹³, R¹⁴, R¹⁵, R¹⁶, R¹⁷, R¹⁸, R¹⁹ and R²⁰ have the same meaning as described above;
R' represents a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent, wherein the plurality of R' moieties may be the same or different.

4. A metal-containing polymer compound having a residue of the metal complex according to any one of claims 1 to 3.

5. A composition comprising the metal complex according to any one of claims 1 to 3 or the metal-containing polymer compound according to claim 4.

6. The composition according to claim 5, comprising two or more metal complexes according to any one of claims 1 to 3 or comprising two or more metal-containing polymer compounds according to claim 4.

7. The composition according to claim 5 or 6, comprising the metal complex according to any one of claims 1 to 3, and a metal complex represented by the following formula (2), a metal complex represented by the following formula (3) or a combination thereof: 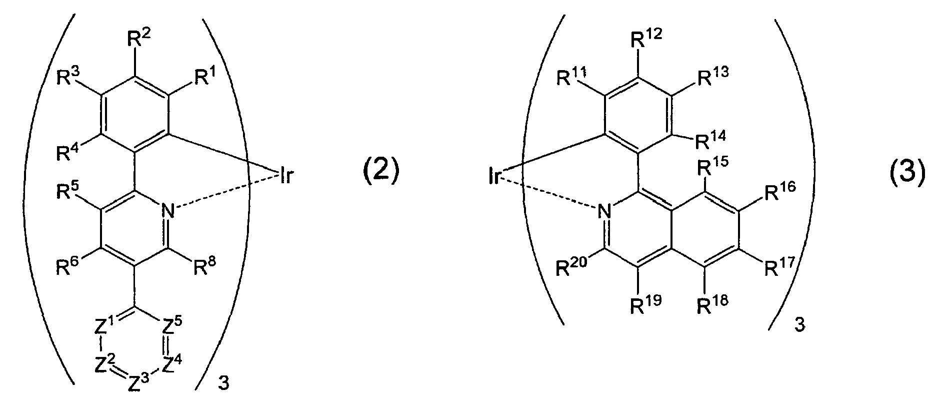 wherein R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described above.

8. The composition according to any one of claims 5 to 7, further comprising a non-metal-containing polymer compound
wherein said non-metal-containing polymer compound preferably has at least one member selected from the group consisting of a divalent group represented by the following formula (7-1), a divalent group represented by the following formula (7-2), a divalent group represented by the following formula (7-3), a divalent group represented by the following formula (7-4), a divalent group represented by the following formula (7-5) and a divalent group represented by the following formula (8), as a repeating unit: 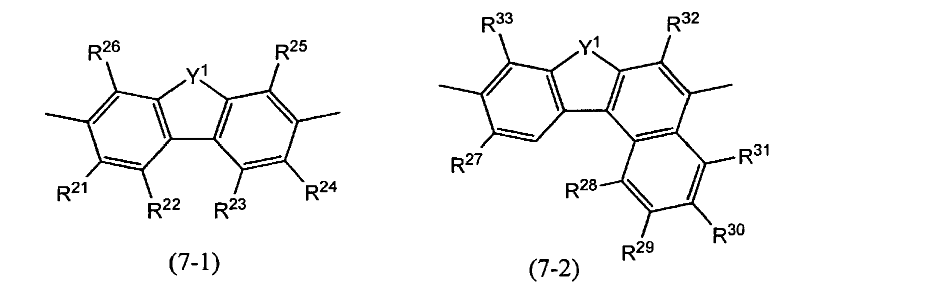 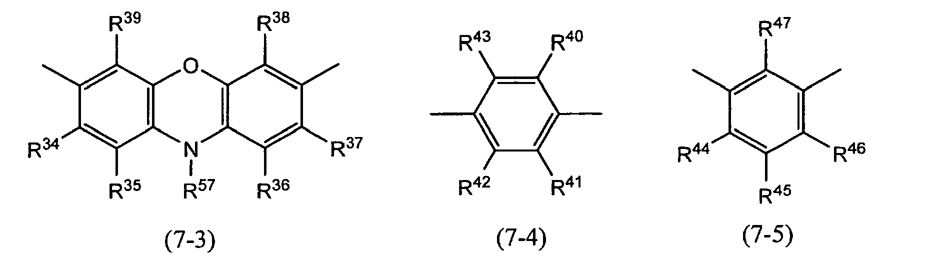
wherein Y¹ represents -C(R⁴⁸)(R⁴⁹)-, -O-C(R⁵⁰)(R⁵¹)-, -O-, -S-, -B(R⁵²)-, -Si(R⁵³)(R⁵⁴)-, -P(R⁵⁵)-, -P(R⁵⁶)( = O)- or N(R⁵⁷)-;
R⁴⁸, R⁴⁹, R⁵⁰, R⁵¹, R⁵², R⁵³, R⁵⁴, R⁵⁵, R⁵⁶ and R⁵⁷ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a silyloxy group, a substituted silyloxy group, a monovalent heterocyclic group or a halogen atom, each of which may have a substituent;
R²¹, R²², R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶ and R⁴⁷ each independently represent a hydrogen atom, an alkyl group, an alkoxy group, an alkylthio group, an aryl group, an aryloxy group, an arylthio group, an arylalkyl group, an arylalkoxy group, an arylalkylthio group, an arylalkenyl group, an arylalkynyl group, an amino group, a substituted amino group, a silyl group, a substituted silyl group, a halogen atom, an acyl group, an acyloxy group, an imine residue, an amide group, an acid imide group, a monovalent heterocyclic group, a carboxyl group, a substituted carboxyl group or a cyano group, each of which may have a substituent: 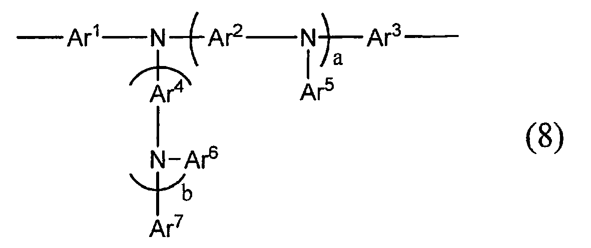
wherein Ar¹, Ar², Ar³ and Ar⁴ each independently represent an arylene group or a divalent heterocyclic group, and Ar⁵, Ar⁶ and Ar⁷ each independently represent an aryl group or a monovalent heterocyclic group, wherein Ar¹, Ar², Ar³, Ar⁴, Ar⁵, Ar⁶ and Ar⁷ may have a substituent; a and b each independently represent 0 or 1, and 0 ≤ a+b ≤ 1.

9. The composition according to claim 8, wherein said non-metal-containing polymer compound further has a repeating unit represented by the following formula (10): 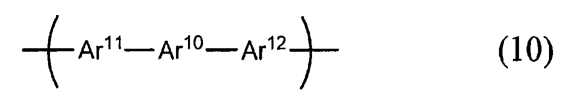
wherein Ar¹⁰ represents a divalent nitrogen-containing aromatic heterocyclic group, and Ar¹¹ and Ar¹² each independently represent an arylene group or a divalent heterocyclic group;
wherein the repeating unit represented by the formula (10) is preferably a repeating unit represented by the following formula (11) : 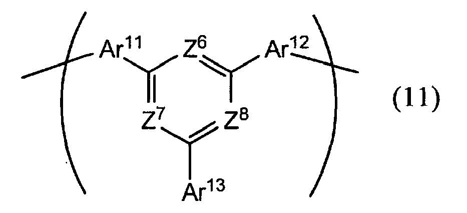
wherein Ar¹¹ and Ar¹² have the same meaning as described above and Ar¹³ represents an aryl group or a monovalent heterocyclic group, wherein Ar¹¹, Ar¹² and Ar¹³ may have a substituent;
Z⁶, Z⁷ and Z⁸ each independently represent -C(R^{k})= or a nitrogen atom and R^{k} represents a hydrogen atom or a substituent, provided that at least two of Z⁶, Z⁷ and Z⁸ are nitrogen atoms.

10. The composition according to any one of claims 5 to 9, further comprising a charge transporting material, a light emitting material or a combination thereof; and/or
further comprising a solvent or a dispersing medium.

11. A film comprising the metal complex according to any one of claims 1 to 3, the metal-containing polymer compound according to claim 4 or the composition according to any one of claims 5 to 10.

12. A device comprising the metal complex according to any one of claims 1 to 3, the metal-containing polymer compound according to Claim 4 or the composition according to any one of claims 5 to 10.

13. The device according to claim 12, wherein said device is a light emitting device.

14. A surface light source or illuminating system comprising the device according to claim 13.

15. A method for producing a metal complex represented by the following formula (1c) : 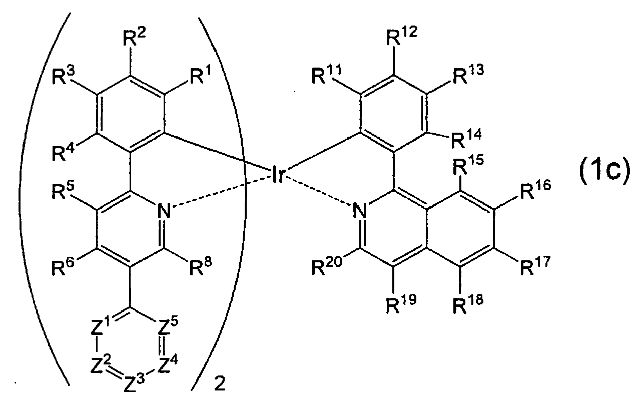
wherein R¹, R², R³, R⁴ R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ and Z⁵ have the same meaning as described below,
the method comprising (a) reacting a metal complex represented by the following formula (4): 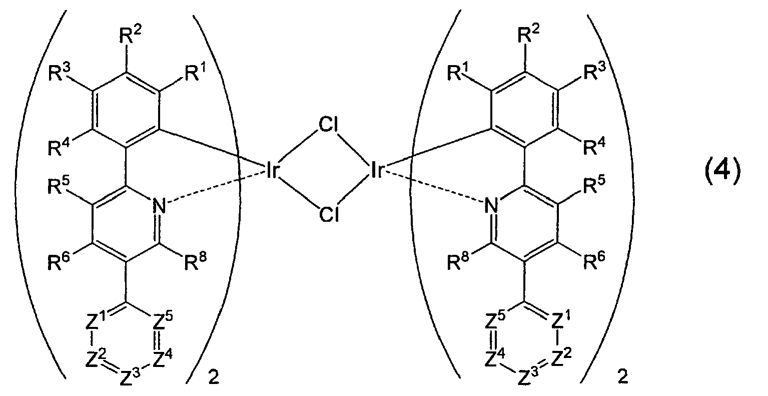
wherein R¹ , R², R³, R⁴, R⁵, R⁶ and R⁸ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent;
Z¹, Z², Z³, Z⁴ and Z⁵ each independently represent, -C(R*)= or a nitrogen atom, wherein R* represents a hydrogen atom or a substituent;
the plurality of moieties of each of R¹, R², R³, R⁴, R⁵, R⁶, R⁸, Z¹, Z², Z³, Z⁴ and Z⁵ may be the same or different, provided that at least two of Z¹, Z², Z³, Z⁴ and Z⁵ are nitrogen atoms,
with a compound represented by the following formula (5): 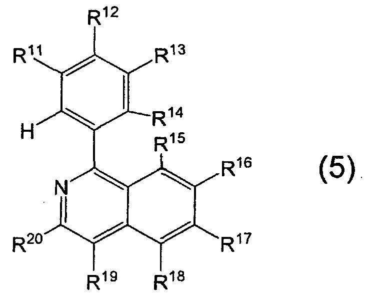
wherein R¹¹, R¹², R¹³, R¹⁴, R¹⁵, R¹⁶, R¹⁷, R¹⁸, R¹⁹ and R²⁰ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent; or (b) reacting a metal complex represented by the following formula (6): 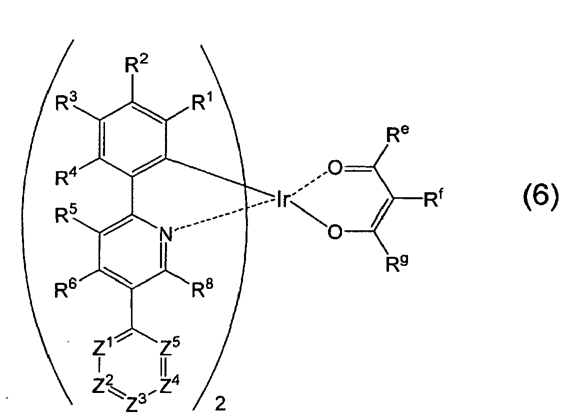
wherein R¹, R² , R³, R⁴, R⁵, R⁶ and R⁸ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent;
Z¹, Z², Z³, Z⁴ and Z⁵ each independently represent, -C(R*)= or a nitrogen atom, wherein R* represents a hydrogen atom or a substituent;
the two moieties of each of R¹, R², R³, R⁴, R⁵, R⁶, R⁸, Z¹, Z², Z³, Z⁴ and Z⁵ may be the same or different, provided that at least two of Z¹, Z², Z³, Z⁴ and Z⁵ are nitrogen atoms;
R^{e}, R^{f} and R^{g} each independently represent a hydrogen atom, an alkyl group, an alkoxy group, an aryl group or a monovalent heterocyclic group,
with a compound represented by the following formula (5): 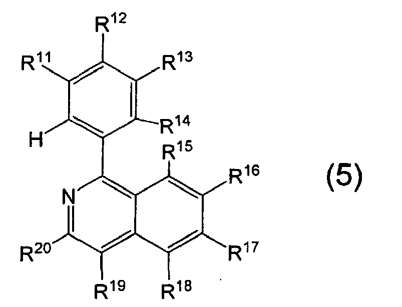
wherein R¹¹, R¹², R¹³, R¹⁴, R¹⁵, R¹⁶, R¹⁷, R¹⁸, R¹⁹ and R²⁰ each independently represent a hydrogen atom, a halogen atom, an alkyl group, an alkoxy group, an alkylthio group, an aryl group, an aryloxy group, an arylthio group, an arylalkyl group, an arylalkoxy group, an arylalkylthio group, an acyl group, an acyloxy group, an amide group, an acid imide group, an imine residue, a substituted amino group, a substituted silyl group, a substituted silyloxy group, a substituted silylthio group, a substituted silylamino group, a monovalent heterocyclic group, a heteroaryloxy group, a heteroarylthio group, an arylalkenyl group, an arylalkynyl group, a substituted carboxyl group or a cyano group, each of which may have a substituent.

## Patentansprüche

1. Ein Metallkomplex, dargestellt durch die folgende Formel (1): 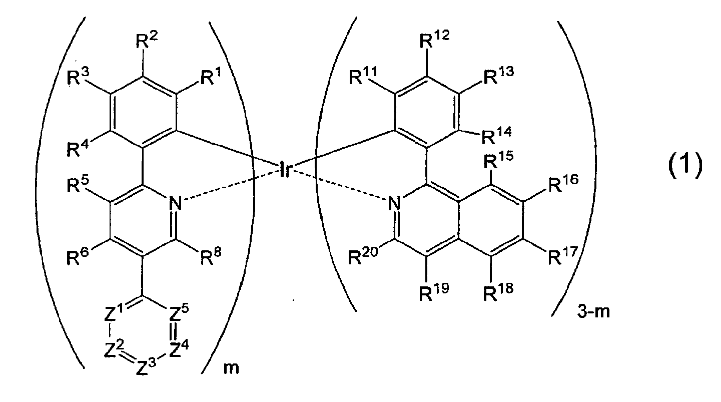
wobei R¹, R², R³, R⁴, R⁵, R⁶, R⁸, R¹¹, R¹², R¹³, R¹⁴, R¹⁵, R¹⁶, R¹⁷, R¹⁸, R¹⁹ und R²⁰ jeweils unabhängig ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Acylrest, einen Acyloxyrest, einen Amidrest, einen Säureimidrest, einen Iminrest, einen substituierten Aminorest, einen substituierten Silylrest, einen substituierten Silyloxyrest, einen substituierten Silylthiorest, einen substituierten Silylaminorest, einen einwertigen heterocyclischen Rest, einen Heteroaryloxyrest, einen Heteroarylthiorest, einen Arylalkenylrest, einen Arylalkinylrest, einen substituierten Carboxylrest oder einen Cyanorest darstellen, von denen jeder einen Substituenten aufweisen kann;
Z¹, Z², Z³, Z⁴ und Z⁵ jeweils unabhängig -C(R*)= oder ein Stickstoffatom darstellen, wobei R* ein Wasserstoffatom oder einen Substituenten darstellt;
wenn es zwei oder mehrere Einheiten von jedem aus R¹, R², R³, R⁴, R⁵, R⁶, R⁸, R¹¹, R¹², 13 14 15 16 17 18 19 R²⁰, Z¹, Z², Z³, Z⁴ und Z⁵ gibt, die zwei oder mehrere Einheiten die gleichen oder verschieden sein können, mit der Maßgabe, dass mindestens zwei aus Z¹, Z², Z³, Z⁴ und Z⁵ Stickstoffatome sind;
m gleich 1 oder 2 darstellt.

2. Der Metallkomplex nach Anspruch 1, dargestellt durch die folgende Formel (1c): 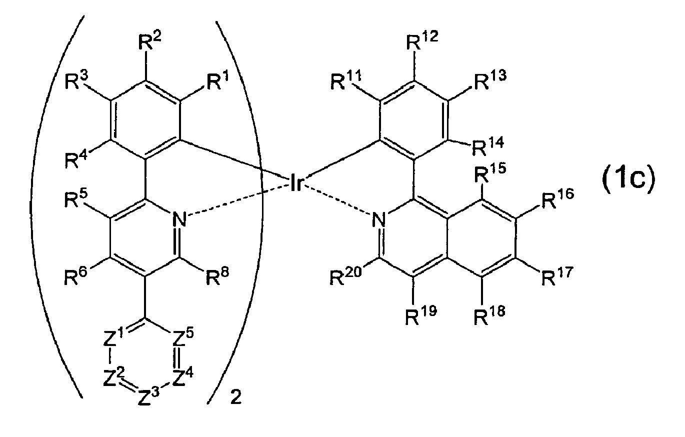 wobei R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ und Z⁵ die gleiche Bedeutung wie vorstehend beschrieben haben.

3. Der Metallkomplex nach Anspruch 2, dargestellt durch die folgende Formel (1a) oder die folgende Formel (1b): 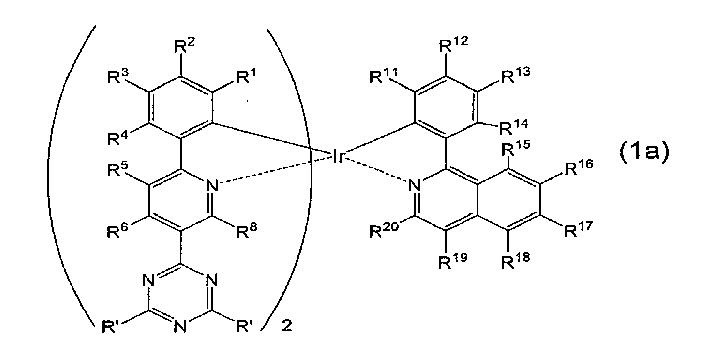 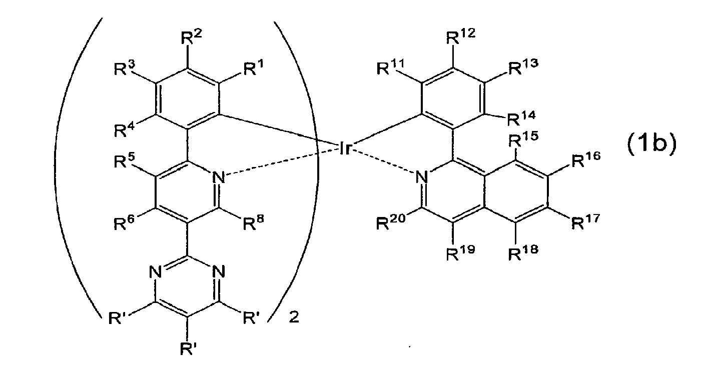
wobei R¹, R², R³, R⁴, R⁵, R⁶, R⁸, R¹¹, R¹², R¹³, R¹⁴, R¹⁵, R¹⁶, R¹⁷, R¹⁸, R¹⁹ und R²⁰ die gleiche Bedeutung wie vorstehend beschrieben haben;
R' ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Acylrest, einen Acyloxyrest, einen Amidrest, einen Säureimidrest, einen Iminrest, einen substituierten Aminorest, einen substituierten Silylrest, einen substituierten Silyloxyrest, einen substituierten Silylthiorest, einen substituierten Silylaminorest, einen einwertigen heterocyclischen Rest, einen Heteroaryloxyrest, einen Heteroarylthiorest, einen Arylalkenylrest, einen Arylalkinylrest, einen substituierten Carboxylrest oder einen Cyanorest darstellt, von denen jeder einen Substituenten aufweisen kann, wobei die Mehrzahl von R'-Einheiten die gleiche oder verschieden sein kann.

4. Eine metallhaltige Polymerverbindung, die einen Rest des Metallkomplexes nach einem der Ansprüche 1 bis 3 aufweist.

5. Eine Zusammensetzung, umfassend den Metallkomplex nach einem der Ansprüche 1 bis 3 oder die metallhaltige Polymerverbindung nach Anspruch 4.

6. Die Zusammensetzung nach Anspruch 5, umfassend zwei oder mehrere Metallkomplexe nach einem der Ansprüche 1 bis 3 oder umfassend zwei oder mehrere metallhaltige Polymerverbindungen nach Anspruch 4.

7. Die Zusammensetzung nach Anspruch 5 oder 6, umfassend den Metallkomplex nach einem der Ansprüche 1 bis 3, und einen Metallkomplex, dargestellt durch die folgende Formel (2), einen Metallkomplex, dargestellt durch die folgende Formel (3) oder eine Kombination davon: 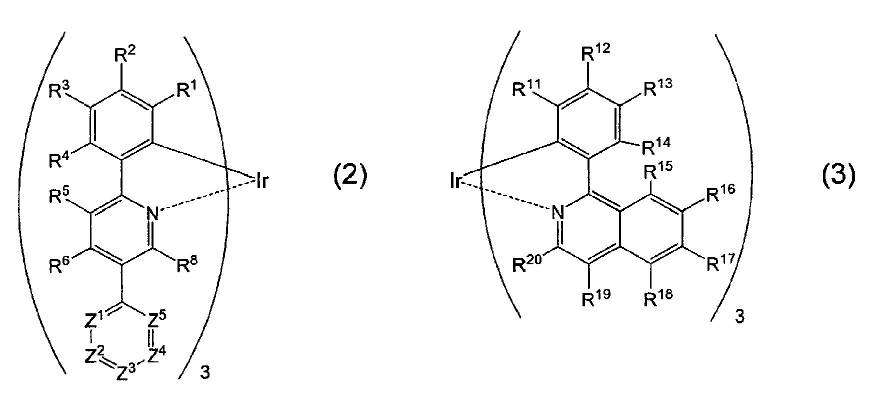 wobei R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ und Z⁵ die gleiche Bedeutung wie vorstehend beschrieben haben.

8. Die Zusammensetzung nach einem der Ansprüche 5 bis 7, weiter umfassend eine nicht metallhaltige Polymerverbindung,
wobei die nicht-metallhaltige Polymerverbindung vorzugsweise mindestens einen Bestandteil, ausgewählt aus der Gruppe bestehend aus einem zweiwertigen Rest, dargestellt durch die folgende Formel (7-1), einem zweiwertigen Rest, dargestellt durch die folgende Formel (7-2), einem zweiwertigen Rest, dargestellt durch die folgende Formel (7-3), einem zweiwertigen Rest, dargestellt durch die folgende Formel (7-4), einem zweiwertigen Rest, dargestellt durch die folgende Formel (7-5) und einem zweiwertigen Rest, dargestellt durch die folgende Formel (8), als eine sich wiederholende Einheit aufweist: 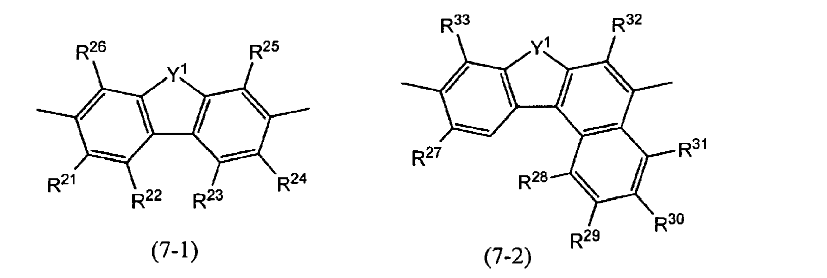 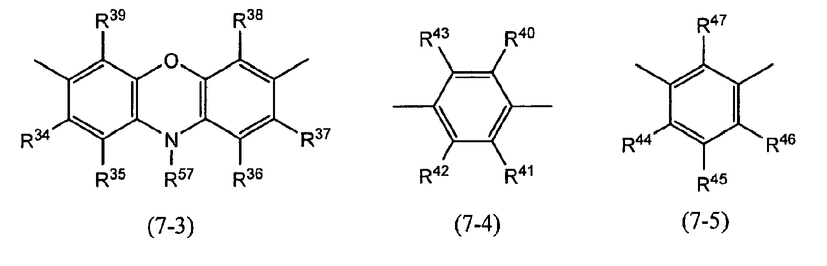
wobei Y¹ für -C(R⁴⁸)(R⁴⁹)-, -O-C(R⁵⁰)(R⁵¹)-, -O-, -S-, -B(R⁵²)-, -Si(R⁵³)(R⁵⁴)-, -P(R⁵⁵)-, -P(R⁵⁶)(=O)- oder N(R⁵⁷)- steht;
R⁴⁸, R⁴⁹, R⁵⁰, R⁵¹, R⁵², R⁵³, R⁵⁴, R⁵⁵, R⁵⁶ und R⁵7 jeweils unabhängig ein Wasserstoffatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Arylalkenylrest, einen Arylalkinylrest, einen Aminorest, einen substituierten Aminorest, einen Silylrest, einen substituierten Silylrest, einen Silyloxyrest, einen substituierten Silyloxyrest, einen einwertigen heterocyclischen Rest oder ein Halogenatom darstellen, von denen jeder einen Substituenten aufweisen kann; R²¹, R²², R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵ R⁴⁶ und R⁴⁷ jeweils unabhängig ein Wasserstoffatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Arylalkenylrest, einen Arylalkinylrest, einen Aminorest, einen substituierten Aminorest, einen Silylrest, einen substituierten Silylrest, ein Halogenatom, einen Acylrest, einen Acyloxyrest, einen Iminrest, einen Amidrest, einen Säureimidrest, einen einwertigen heterocyclischen Rest, einen Carboxylrest, einen substituierten Carboxylrest oder einen Cyanorest darstellen, von denen jeder einen Substituenten aufweisen kann: 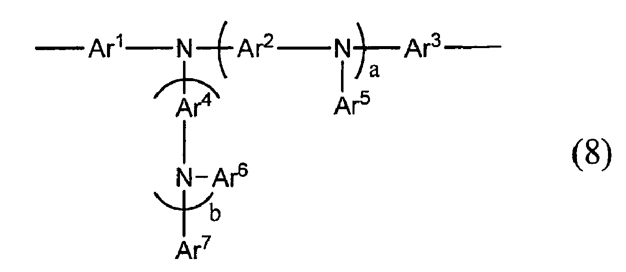
wobei Ar¹, Ar², Ar³ und Ar⁴ jeweils unabhängig einen Arylenrest oder einen zweiwertigen heterocyclischen Rest darstellen und Ar⁵, Ar⁶ und Ar⁷ jeweils unabhängig einen Arylrest oder einen einwertigen heterocyclischen Rest darstellen, wobei Ar¹, Ar², Ar³, Ar⁴, Ar⁵, Ar⁶ und Ar⁷ einen Substituenten aufweisen können; a und b jeweils unabhängig 0 oder 1 darstellen und 0 ≤ a+b ≤ 1.

9. Die Zusammensetzung nach Anspruch 8, wobei die nicht-metallhaltige Polymerverbindung ferner eine sich wiederholende Einheit aufweist, dargestellt durch die folgende Formel (10): 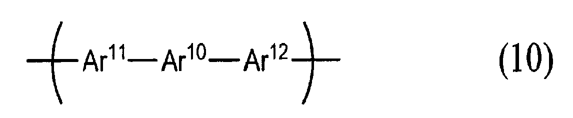
wobei Ar¹⁰ einen zweiwertigen, stickstoffhaltigen, aromatischen, heterocyclischen Rest darstellt und Ar¹¹ und Ar¹² jeweils unabhängig einen Arylenrest oder einen zweiwertigen heterocyclischen Rest darstellen;
wobei die sich wiederholende Einheit, dargestellt durch die Formel (10), vorzugsweise eine sich wiederholende Einheit, dargestellt durch die folgende Formel (11), ist: 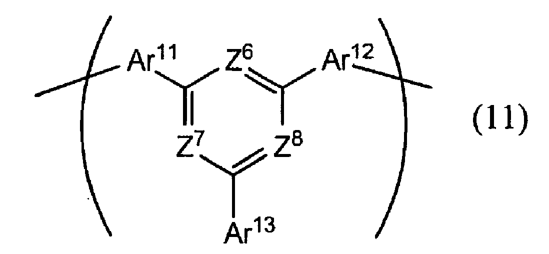
wobei Ar¹¹ und Ar¹² die gleiche Bedeutung wie vorstehend beschrieben haben und Ar¹³ einen Arylrest oder einen einwertigen heterocyclischen Rest darstellt, wobei Ar¹¹, Ar¹² und Ar¹³ einen Substituenten aufweisen können;
Z⁶, Z⁷ und Z⁸ jeweils unabhängig -C(R^{k})= oder ein Stickstoffatom darstellen und R^{k} ein Wasserstoffatom oder einen Substituenten darstellt, mit der Maßgabe, dass mindestens zwei aus Z⁶, Z⁷ und Z⁸ Stickstoffatome sind.

10. Die Zusammensetzung nach einem der Ansprüche 5 bis 9, ferner umfassend ein ladungstransportierendes Material, ein Licht emittierendes Material oder eine Kombination davon; und/oder ferner umfassend ein Lösungsmittel oder ein Dispersionsmedium.

11. Eine Folie, umfassend den Metallkomplex nach einem der Ansprüche 1 bis 3, die metallhaltige Polymerverbindung nach Anspruch 4 oder die Zusammensetzung nach einem der Ansprüche 5 bis 10.

12. Eine Vorrichtung, umfassend den Metallkomplex nach einem der Ansprüche 1 bis 3, die metallhaltige Polymerverbindung nach Anspruch 4 oder die Zusammensetzung nach einem der Ansprüche 5 bis 10.

13. Die Vorrichtung nach Anspruch 12, wobei die Vorrichtung eine Licht emittierende Vorrichtung ist.

14. Eine Flächenlichtquelle oder ein Belichtungssystem, umfassend die Vorrichtung nach Anspruch 13.

15. Ein Verfahren zur Herstellung eines Metallkomplexes, dargestellt durch die folgende Formel (1c): 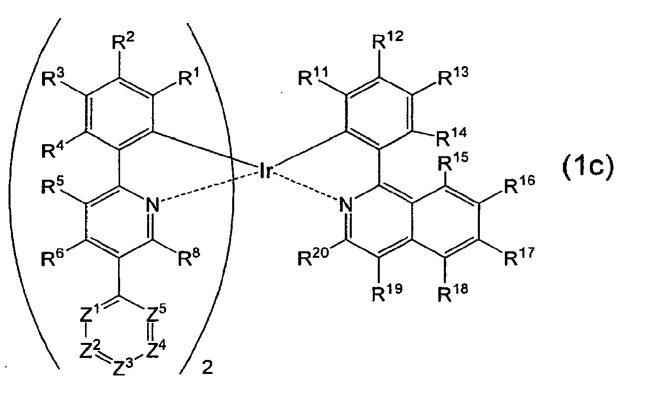
wobei R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ und Z⁵ die gleiche Bedeutung wie vorstehend beschrieben haben,
wobei das Verfahren umfasst
(a) Umsetzen eines Metallkomplexes, dargestellt durch die folgende Formel (4): 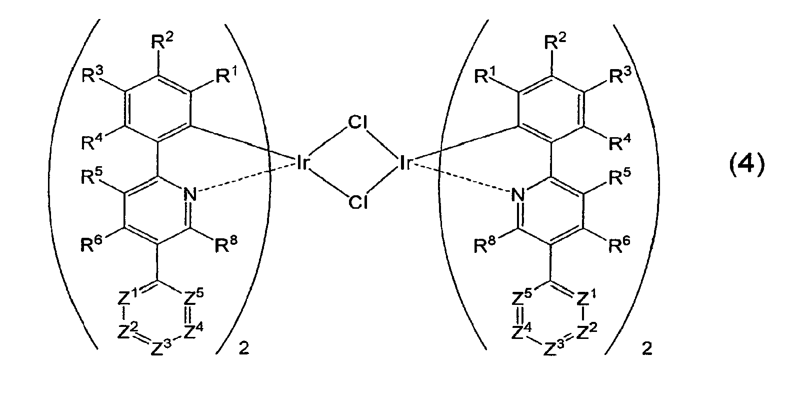
wobei R¹, R², R³, R⁴, R⁵, R⁶ und R⁸ jeweils unabhängig ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Acylrest, einen Acyloxyrest, einen Amidrest, einen Säureimidrest, einen Iminrest, einen substituierten Aminorest, einen substituierten Silylrest, einen substituierten Silyloxyrest, einen substituierten Silylthiorest, einen substituierten Silylaminorest, einen einwertigen heterocyclischen Rest, einen Heteroaryloxyrest, einen Heteroarylthiorest, einen Arylalkenylrest, einen Arylalkinylrest, einen substituierten Carboxylrest oder einen Cyanorest darstellen, von denen jeder einen Substituenten aufweisen kann;
Z¹, Z², Z³, Z⁴ und Z⁵ jeweils unabhängig -C(R^{*})= oder ein Stickstoffatom darstellen, wobei R^{*} ein Wasserstoffatom oder einen Substituenten darstellt;
die Mehrzahl von Einheiten von jedem aus R¹, R², R³, R⁴, R⁵, R⁶, R⁸, Z¹, Z², Z³, Z⁴ und Z⁵ die gleiche oder verschieden sein kann, mit der Maßgabe, dass mindestens zwei aus Z¹, Z², Z³, Z⁴ und Z⁵ Stickstoffatome sind,
mit einer Verbindung, dargestellt durch die folgende Formel (5): 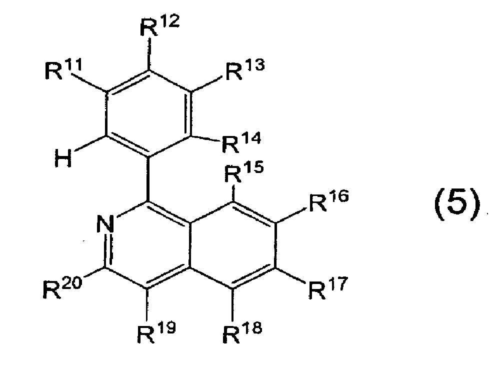
wobei R¹¹, R¹², R¹³, R¹⁴, R¹⁵, R¹⁶, R¹⁷, R¹⁸, R¹⁹ und R²⁰ jeweils unabhängig ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Acylrest, einen Acyloxyrest, einen Amidrest, einen Säureimidrest, einen Iminrest, einen substituierten Aminorest, einen substituierten Silylrest, einen substituierten Silyloxyrest, einen substituierten Silylthiorest, einen substituierten Silylaminorest, einen einwertigen heterocyclischen Rest, einen Heteroaryloxyrest, einen Heteroarylthiorest, einen Arylalkenylrest, einen Arylalkinylrest, einen substituierten Carboxylrest oder einen Cyanorest darstellen, von denen jeder einen Substituenten aufweisen kann; oder
(b) Umsetzen eines Metallkomplexes, dargestellt durch die folgende Formel (6): 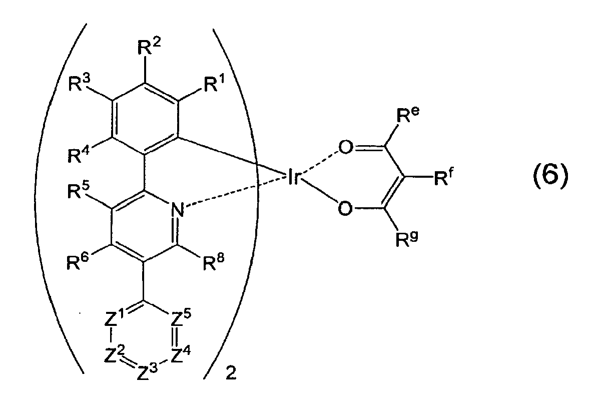
wobei R¹, R², R³, R⁴, R⁵, R⁶ und R⁸ jeweils unabhängig ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Acylrest, einen Acyloxyrest, einen Amidrest, einen Säureimidrest, einen Iminrest, einen substituierten Aminorest, einen substituierten Silylrest, einen substituierten Silyloxyrest, einen substituierten Silylthiorest, einen substituierten Silylaminorest, einen einwertigen heterocyclischen Rest, einen Heteroaryloxyrest, einen Heteroarylthiorest, einen Arylalkenylrest, einen Arylalkinylrest, einen substituierten Carboxylrest oder einen Cyanorest darstellen, von denen jeder einen Substituenten aufweisen kann;
Z¹, Z², Z³, Z⁴ und Z⁵ jeweils unabhängig -C(R^{*})= oder ein Stickstoffatom darstellen, wobei R^{*} ein Wasserstoffatom oder einen Substituenten darstellt;
die zwei Einheiten von jedem aus R¹, R², R³, R⁴, R⁵, R⁶, R⁸, Z¹, Z², Z³, Z⁴ und Z⁵ die gleichen oder verschieden sein können, mit der Maßgabe, dass mindestens zwei aus Z¹, Z², Z³, Z⁴ und Z⁵ Stickstoffatome sind;
R^{e}, R^{f} und R^{g} jeweils unabhängig ein Wasserstoffatom, einen Alkylrest, einen Alkoxyrest, einen Arylrest oder einen einwertigen heterocyclischen Rest darstellen, mit einer Verbindung, dargestellt durch die folgende Formel (5): 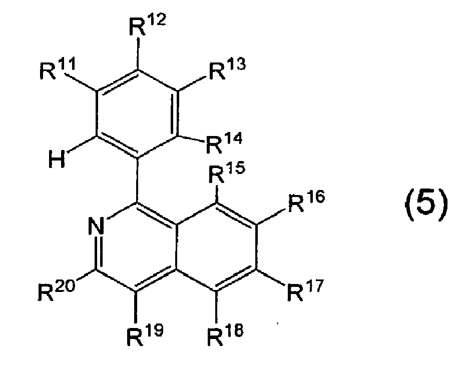
wobei R¹¹, R¹², R¹³, R¹⁴, R¹⁵, R¹⁶, R¹⁷, R¹⁸, R¹⁹ und R²⁰ jeweils unabhängig ein Wasserstoffatom, ein Halogenatom, einen Alkylrest, einen Alkoxyrest, einen Alkylthiorest, einen Arylrest, einen Aryloxyrest, einen Arylthiorest, einen Arylalkylrest, einen Arylalkoxyrest, einen Arylalkylthiorest, einen Acylrest, einen Acyloxyrest, einen Amidrest, einen Säureimidrest, einen Iminrest, einen substituierten Aminorest, einen substituierten Silylrest, einen substituierten Silyloxyrest, einen substituierten Silylthiorest, einen substituierten Silylaminorest, einen einwertigen heterocyclischen Rest, einen Heteroaryloxyrest, einen Heteroarylthiorest, einen Arylalkenylrest, einen Arylalkinylrest, einen substituierten Carboxylrest oder einen Cyanorest darstellen, von denen jeder einen Substituenten aufweisen kann.

## Revendications

1. Complexe métallique représenté par la formule (1) suivante : 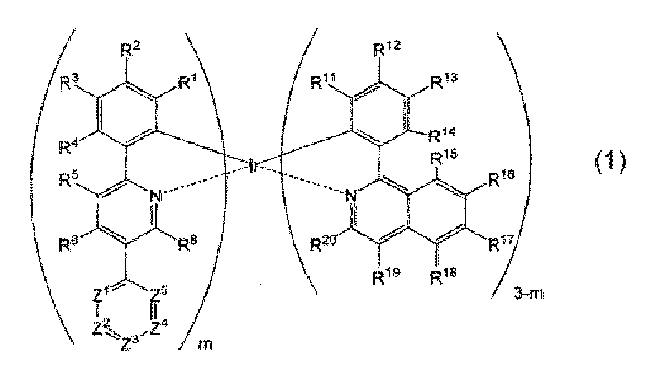
où R¹, R², R³, R⁴, R⁵, R⁶, R⁸, R¹¹, R¹², R¹³, R¹⁴, R¹⁵, R¹⁶, R¹⁷, R¹⁸, R¹⁹ et R²⁰ représentent chacun indépendamment un atome d'hydrogène, un atome d'halogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe acyle, un groupe acyloxy, un groupe amide, un groupe imide d'acide, un résidu imine, un groupe amino substitué, un groupe silyle substitué, un groupe silyloxy substitué, un groupe silylthio substitué, un groupe silylamino substitué, un groupe hétérocyclique monovalent, un groupe hétéroaryloxy, un groupe hétéroarylthio, un groupe arylalcényle, un groupe arylalcynyle, un groupe carboxyle substitué ou un groupe cyano, chacun desquels peut avoir un substituant ;
Z¹, Z², Z³, Z⁴ et Z⁵ représentent chacun indépendamment -C(R*)= ou un atome d'azote, où R* représente un atome d'hydrogène ou un substituant ;
quand il y a deux ou plusieurs groupements de chacun de R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ et Z⁵, les deux ou plusieurs groupements peuvent être identiques ou différents, à condition qu'au moins deux de Z¹, Z², Z³, Z⁴ et Z⁵ soient des atomes d'azote ;
m représente 1 ou 2.

2. Complexe métallique selon la revendication 1, représenté par la formule (1c) suivante : 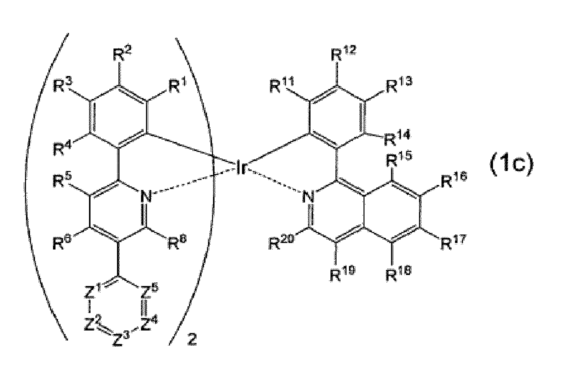 OÙ R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ et Z⁵ ont la même signification que décrit ci-dessus.

3. Complexe métallique selon la revendication 2, représenté par la formule (la) suivante ou la formule (1b) suivante : 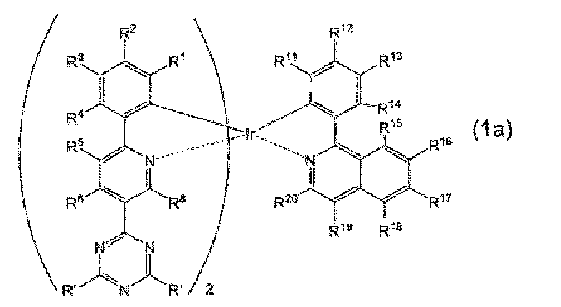 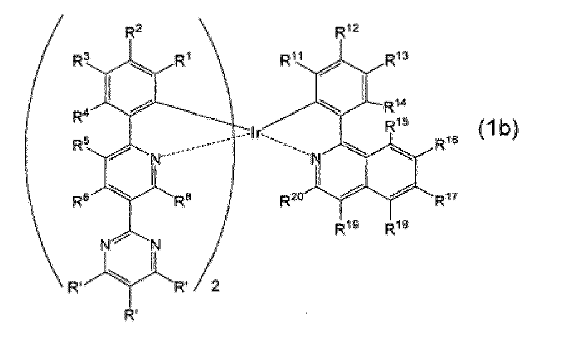
où R¹, R², R³, R⁴, R⁵, R⁶, R⁸, R¹¹, R¹², R¹³, R¹⁴, R¹⁵, R¹⁶, R¹⁷, R¹⁸, R¹⁹ et R²⁰ ont la même signification que décrit ci-dessus ;
R' représente un atome d'hydrogène, un atome d'halogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe acyle, un groupe acyloxy, un groupe amide, un groupe imide d'acide, un résidu imine, un groupe amino substitué, un groupe silyle substitué, un groupe silyloxy substitué, un groupe silylthio substitué, un groupe silylamino substitué, un groupe hétérocyclique monovalent, un groupe hétéroaryloxy, un groupe hétéroarylthio, un groupe arylalcényle, un groupe arylalcynyle, un groupe carboxyle substitué ou un groupe cyano, chacun desquels peut avoir un substituant, où la pluralité de groupements R' peuvent être identiques ou différents.

4. Composé polymère contenant un métal ayant un résidu du complexe métallique selon l'une quelconque des revendications 1 à 3.

5. Composition comprenant le complexe métallique selon l'une quelconque des revendications 1 à 3 ou le composé polymère contenant un métal selon la revendication 4.

6. Composition selon la revendication 5, comprenant deux ou plusieurs complexes métalliques selon l'une quelconque des revendications 1 à 3 ou comprenant deux ou plusieurs composés métalliques contenant un métal selon la revendication 4.

7. Composition selon la revendication 5 ou 6, comprenant le complexe métallique selon l'une quelconque des revendications 1 à 3 et un complexe métallique représenté par la formule (2) suivante, un complexe métallique représenté par la formule (3) suivante ou une combinaison de ceux-ci : 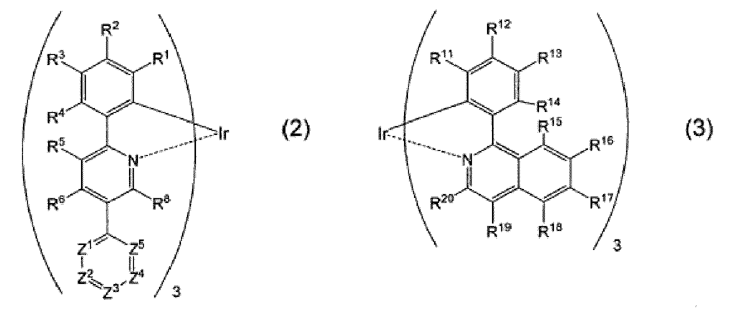 où R¹, R², R³, R⁴, R⁵, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ et Z⁵ ont la même signification que décrit ci-dessus.

8. Composition selon l'une quelconque des revendications 5 à 7, comprenant en outre un composé polymère ne contenant pas de métal
où ledit composé polymère ne contenant pas de métal a de préférence au moins un membre choisi dans le groupe consistant en un groupe divalent représenté par la formule (7-1) suivante, un groupe divalent représenté par la formule (7-2) suivante, un groupe divalent représenté par la formule (7-3) suivante, un groupe divalent représenté par la formule (7-4) suivante, un groupe divalent représenté par la formule (7-5) suivante et un groupe divalent représenté par la formule (8) suivante, comme unité répétée : 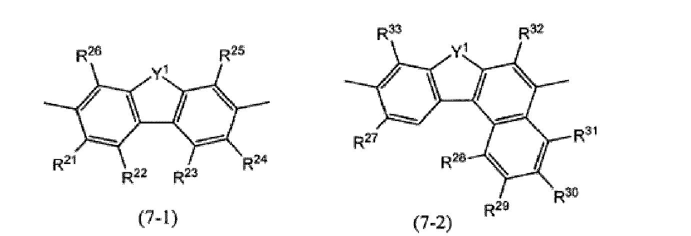 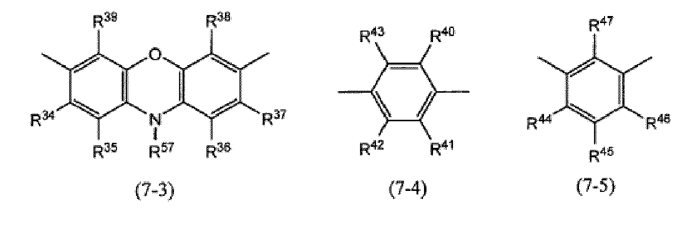
où Y¹ représente -(CR⁴⁸) (R⁴⁹)-, -O-C(R⁵⁰)(R⁵¹)-, -O-, -S-, -B(R⁵²)-, -Si(R⁵³)(R⁵⁴)-, -P(R⁵⁵)-, -P(R⁵⁶)(=O)- ou N(R⁵⁷)-;
R⁴⁸, R⁴⁹, R⁵⁰, R⁵¹, R⁵², R⁵³, R⁵⁴, R⁵⁵, R⁵⁶ et R⁵⁷ représentent chacun indépendamment un atome d'hydrogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe arylalcényle, un groupe arylalcynyle, un groupe amino, un groupe amino substitué, un groupe silyle, un groupe silyle substitué, un groupe silyloxy, un groupe silyloxy substitué, un groupe hétérocyclique monovalent ou un atome d'halogène, chacun desquels peut avoir un substituant ;
R²¹, R²², R²³, R²⁴, R²⁵, R²⁶, R²⁷, R²⁸, R²⁹, R³⁰, R³¹, R³², R³³, R³⁴, R³⁵, R³⁶, R³⁷, R³⁸, R³⁹, R⁴⁰, R⁴¹, R⁴², R⁴³, R⁴⁴, R⁴⁵, R⁴⁶, et R⁴⁷ représentent chacun indépendamment un atome d'hydrogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe arylalcényle, un groupe arylalcynyle, un groupe amino, un groupe amino substitué, un groupe silyle, un groupe silyle substitué, un atome d'halogène, un groupe acyle, un groupe acyloxy, un résidu imine, un groupe amide, un groupe imide d'acide, un groupe hétérocyclique monovalent, un groupe carboxyle, un groupe carboxyle substitué ou un groupe cyano, chacun desquels peut avoir un substituant : 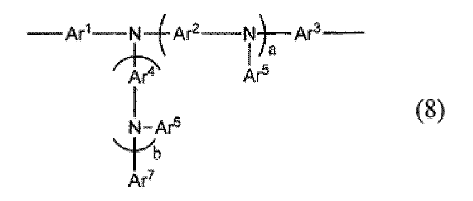
où Ar¹, Ar², Ar³ et Ar⁴ représentent chacun indépendamment un groupe arylène ou un groupe hétérocyclique divalent, et Ar⁵, Ar⁶ et Ar⁷ représentent chacun indépendamment un groupe aryle ou un groupe hétérocyclique monovalent, où Ar¹, Ar², Ar³, Ar⁴, Ar⁵_{.} Ar⁶ et Ar⁷ peuvent avoir un substituant ; a et b représentent chacun indépendamment 0 ou 1, et 0 ≤ a+b ≤ 1.

9. Composition selon la revendication 8, où ledit composé polymère ne contenant pas de métal a en outre une unité répétée représentée par la formule (10) suivante : 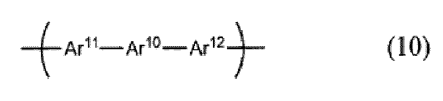
où Ar¹⁰ représente un groupe hétérocyclique aromatique contenant de l'azote divalent, et Ar¹¹ et Ar¹² représentent chacun indépendamment un groupe arylène ou un groupe hétérocyclique divalent ;
où l'unité répétée représentée par la formule (10) est de préférence une unité répétée représentée par la formule (11) suivante : 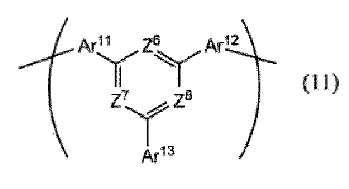
où Ar¹¹ et Ar¹² ont la même signification que décrit ci-dessus et Ar¹³ représente un groupe aryle ou un groupe hétérocyclique monovalent, où Ar¹¹, Ar¹² et Ar¹³ peuvent avoir un substituant ;
Z⁶, Z⁷ et Z⁸ représentent chacun indépendamment -C(R^{k}) = ou un atome d'azote et R^{k} représente un atome d'hydrogène ou un substituant, à condition qu'au moins deux de Z⁶, Z⁷ et Z⁸ soient des atomes d'azote.

10. Composition selon l'une quelconque des revendications 5 à 9, comprenant en outre un matériau transportant des charges, un matériau émettant de la lumière ou une combinaison de ceux-ci ; et/ou comprenant en outre un solvant ou un milieu dispersant.

11. Film comprenant le complexe métallique selon l'une quelconque des revendications 1 à 3, le composé polymère contenant un métal selon la revendication 4 ou la composition selon l'une quelconque des revendications 5 à 10.

12. Dispositif comprenant le complexe métallique selon l'une quelconque des revendications 1 à 3, le composé polymère contenant un métal selon la revendication 4 ou la composition selon l'une quelconque des revendications 5 à 10.

13. Dispositif selon la revendication 12, où ledit dispositif est un dispositif émettant de la lumière.

14. Source lumineuse de surface ou système d'illumination comprenant le dispositif selon la revendication 13.

15. Procédé pour produire un complexe métallique représenté par la formule (1c) suivante : 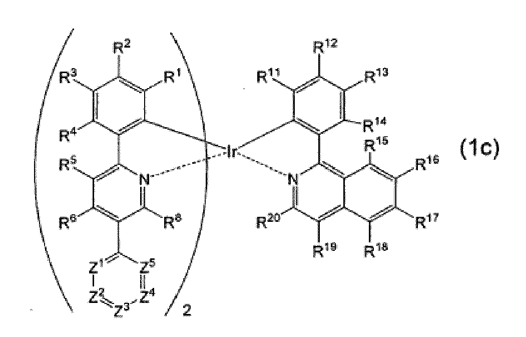
OÙ R¹, R²¹, R³¹, R⁴¹, R⁵¹, R⁶, R⁸, R¹¹, R¹², R¹³, R¹⁴, R¹⁵, R¹⁶, R¹⁷, R¹⁸, R¹⁹, R²⁰, Z¹, Z², Z³, Z⁴ et Z⁵ ont la même signification que décrit ci-dessus,
le procédé comprenant (a) la réaction d'un complexe métallique représenté par la formule (4) suivante : 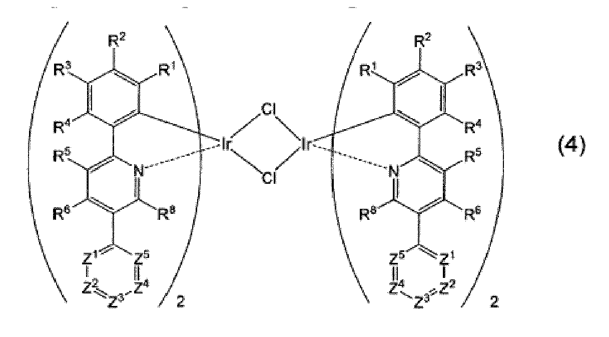
où R¹, R², R³, R⁴, R⁵, R⁶ et R⁸ représentent chacun indépendamment un atome d'hydrogène, un atome d'halogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe acyle, un groupe acyloxy, un groupe amide, un groupe imide d'acide, un résidu imine, un groupe amino substitué, un groupe silyle substitué, un groupe silyloxy substitué, un groupe silylthio substitué, un groupe silylamino substitué, un groupe hétérocyclique monovalent, un groupe hétéroaryloxy, un groupe hétéroarylthio, un groupe arylalcényle, un groupe arylalcynyle, un groupe carboxyle substitué ou un groupe cyano, chacun desquels peut avoir un substituant ;
Z¹, Z², Z³, Z⁴ et Z⁵ représentent chacun indépendamment -C(R*)= ou un atome d'azote, où R* représente un atome d'hydrogène ou un substituant ;
la pluralité de groupements de chacun de R¹, R², R³, R⁴, R⁵, R⁶, R⁸, Z¹, Z², Z³, Z⁴ et Z⁵ peuvent être identiques ou différents, à condition qu'au moins deux de Z¹, Z², Z³, Z⁴ et Z⁵ soient des atomes d'azote,
avec un composé représenté par la formule (5) suivante: 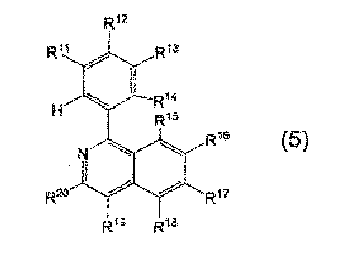
où R¹¹, R¹², R¹³, R¹⁴, R¹⁵, R¹⁶, R¹⁷, R¹⁸, R¹⁹ et R²⁰ représentent chacun indépendamment un atome d'hydrogène, un atome d'halogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe acyle, un groupe acyloxy, un groupe amide, un groupe imide d'acide, un résidu imine, un groupe amino substitué, un groupe silyle substitué, un groupe silyloxy substitué, un groupe silylthio substitué, un groupe silylamino substitué, un groupe hétérocyclique monovalent, un groupe hétéroaryloxy, un groupe hétéroarylthio, un groupe arylalcényle, un groupe arylalcynyle, un groupe carboxyle substitué ou un groupe cyano, chacun desquels peut avoir un substituant ; ou (b) la réaction d'un complexe métallique représenté par la formule (6) suivante : 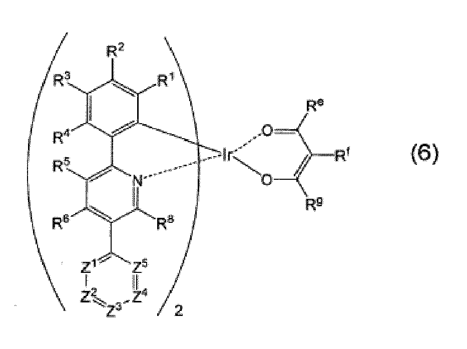
où R¹, R², R³, R⁴, R⁵, R⁶ et R⁸ représentent chacun indépendamment un atome d'hydrogène, un atome d'halogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe acyle, un groupe acyloxy, un groupe amide, un groupe imide d'acide, un résidu imine, un groupe amino substitué, un groupe silyle substitué, un groupe silyloxy substitué, un groupe silylthio substitué, un groupe silylamino substitué, un groupe hétérocyclique monovalent, un groupe hétéroaryloxy, un groupe hétéroarylthio, un groupe arylalcényle, un groupe arylalcynyle, un groupe carboxyle substitué ou un groupe cyano, chacun desquels peut avoir un substituant ;
Z¹, Z², Z³, Z⁴ et Z⁵ représentent chacun indépendamment -C(R*)= ou un atome d'azote, où R* représente un atome d'hydrogène ou un substituant ;
les deux groupements de chacun de R¹, R², R³, R⁴, R⁵, R⁶, R⁸, Z¹, Z², Z³, Z⁴ et Z⁵ peuvent être identiques ou différents, à condition qu'au moins deux de Z¹, Z², Z³, Z⁴ et Z⁵ soient des atomes d'azote ;
R^{e}, R^{f} et R^{g} représentent chacun indépendamment un atome d'hydrogène, un groupe alkyle, un groupe alcoxy, un groupe aryle ou un groupe hétérocyclique monovalent,
avec un composé représenté par la formule (5) suivante : 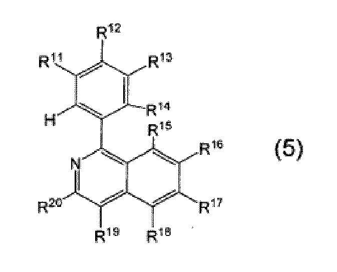
où R¹¹, R¹², R¹³, R¹⁴, R¹⁵, R¹⁶, R¹⁷, R¹⁸, R¹⁹ et R²⁰ représentent chacun indépendamment un atome d'hydrogène, un atome d'halogène, un groupe alkyle, un groupe alcoxy, un groupe alkylthio, un groupe aryle, un groupe aryloxy, un groupe arylthio, un groupe arylalkyle, un groupe arylalcoxy, un groupe arylalkylthio, un groupe acyle, un groupe acyloxy, un groupe amide, un groupe imide d'acide, un résidu imine, un groupe amino substitué, un groupe silyle substitué, un groupe silyloxy substitué, un groupe silylthio substitué, un groupe silylamino substitué, un groupe hétérocyclique monovalent, un groupe hétéroaryloxy, un groupe hétéroarylthio, un groupe arylalcényle, un groupe arylalcynyle, un groupe carboxyle substitué ou un groupe cyano, chacun desquels peut avoir un substituant.
